(19) Europäisches Patentamt
European Patent Office
Office européen des brevets

(11) Publication number: **0 161 304 B1**

(12) **EUROPEAN PATENT SPECIFICATION**

(45) Date of publication of patent specification: **10.01.90**

(51) Int. Cl.[5]: **C 07 D 257/04,** A 01 N 43/713

(21) Application number: **84904262.7**

(22) Date of filing: **02.11.84**

(86) International application number: **PCT/US84/01799**

(87) International publication number: **WO 85/01939 09.05.85 Gazette 85/11**

(54) **HERBICIDAL 1-ARYL-4-SUBSTITUTED-1,4-DIHYDRO-5H-TETRAZOL-5-ONES AND SULFUR ANALOGS THEREOF.**

(30) Priority: **04.11.83 US 549334**

(43) Date of publication of application: **21.11.85 Bulletin 85/47**

(45) Publication of the grant of the patent: **10.01.90 Bulletin 90/02**

(84) Designated Contracting States: **CH DE FR GB LI NL**

(56) References cited:
**EP-A-0 003 619**
**DD-A- 160 447**
**FR-A-1 451 028**
**JP-A-56 053 663**
**US-A-3 865 570**
**US-A-4 318 731**
**US-A-4 399 285**
**US-A-4 404 019**

(73) Proprietor: **FMC Corporation**
**2000 Market Street**
**Philadelphia Pennsylvania 19103 (US)**

(72) Inventor: **THEODORIDIS, George**
**85 Erdman Avenue**
**Princeton, NJ 08540 (US)**

(74) Representative: **Crampton, Keith John Allen et al**
**D YOUNG & CO 10 Staple Inn**
**London WC1V 7RD (GB)**

**EP 0 161 304 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention described in this application relates to the control of undesirable plants. It is more particularly but not exclusively concerned with weed control in agriculture, horticulture, and other applications where unwanted plant growth needs to be controlled. The present specification describes a series of novel herbicidal 1-aryl-5-tetrazolinones and thiones, herbicidal compositions containing them, methods of preparing them, and methods for preventing or destroying undesired plant growth by preemergence or postemergence application of the herbicidal compositions to the locus where control is desired. Also described are intermediate compounds useful in the preparation of the herbicidal compounds. As will appear below, the herbicidal compounds of the invention may be used to effectively control a variety of both grassy and broadleaf plant species. The present invention is particularly useful in agriculture, as a number of the novel aryltetrazolinones described herein show a selectivity favorable to soybean, cotton, wheat, lima bean, corn, sorghum, and other crops at application levels that inhibit growth of or destroy a variety of weeds.

1-Phenyl-5(4H)-tetrazolinone, formula A below wherein Y and R are hydrogen, is believed to be the first 1-aryl-5-tetrazolinone reported in the literature. The compound was made by M. Freund et al. and described in *Ber., 28,* 78 (1895).

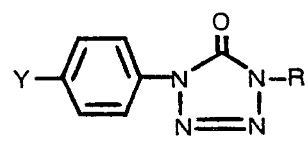

A

In the almost ninety years since disclosure of the parent compound, only a handful of substituted derivatives of it have been described in the literature.

1-(Monosubstituted-phenyl)-5-tetrazolinones of formula A are disclosed in a paper by J. P. Horwitz et al. in *J. Am. Chem. Soc., 81,* 3076 (1959). In the disclosed compounds, Y is methyl, methoxy, chloro, bromo, nitro, or amino and R is hydrogen.

O. Tsuge et al., *J. Org. Chem., 45,* 5130 (1980), disclose a number of the compounds disclosed by Horwitz et al., above, and additional compounds of formula A wherein the phenyl group is unsubstituted (Y is hydrogen) and R is phenylaminocarbonyl, acetyl, or 2-methyl-propanoyl.

J-M. Vandensavel et al., *J. Org. Chem., 38,* 675 (1973), report the preparation of compounds of formula A above wherein Y is hydrogen and R is n-butyl or 4-chlorophenylsulfonyl, Y is nitro and R is n-butyl or cyclohexyl, and Y is chloro or methoxy and R is 4-chlorophenylsulfonyl.

J. K. Elwood et al., *J. Org. Chem., 32,* 2956 (1967), describe compounds of formula A wherein Y is hydrogen and R is 2-propenyl, 1-methyl-2-propenyl, or 2-butenyl.

Additional compounds of formula A wherein R is methyl and Y is hydrogen, chloro, or nitro, or R is benzyl and Y is chloro are disclosed by A. Vollmar et al., *J. Heterocycl. Chem., 11,* 491 (1974).

None of the above documents discloses that the compounds mentioned have herbicidal properties.

DDR Patent Specification DD—A—160447 discloses herbicidal compositions containing a triazolin-5-one derivative of Formula (I)

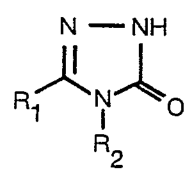

(I)

where each of $R_1$ and $R_2$ is hydrogen or optionally substituted alkyl, aryl, aralkyl or cycloalkyl, where typical substituents are one or more of halo, hydroxy, amino (optionally substituted by alkyl and/or aryl), alkoxy, aryloxy, alkylthio or arylthio.

US Patent Specification US—A—4318731 discloses herbicidal $\Delta^2$-1,2,4-triazolin-5-one derivatives represented by the formula

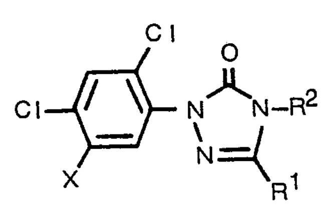

where $R^1$ is a alkyl; $R^2$ is hydrogen, alkyl or alkenyl; and X is hydroxy, alkyl, alkoxy, alkoxyalkoxy, alkenyloxy, or alkoxycarbonylalkoxy.

US—A—4409019 discloses herbicidal compounds of the formula:

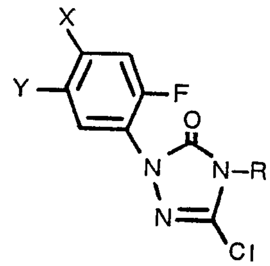

where R is alkyl, alkenyl or cycloalkyl; X is chlorine or bromine and Y is hydrogen or alkoxy.

Japanese Patent Specification JP—A—56—53663 discloses herbicidal triazolinones of formula (I):

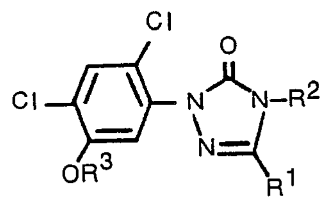

(I)

in which $R^1$ is alkyl; $R^2$ is H, alkyl, alkoxyalkyl, alkoxycarbonylalkyl, aralkyl, alkylcarbonyl or alkenyl; $R^3$ is alkyl, aralkyl, alkoxyalkyl, alkylthioalkyl, alkylcarbonyl, alkoxycarbonylalkyl, dialkylcarbamoyl or allyl.

European Patent Specification EP—A—003619 discloses herbicidal compounds of the general formula

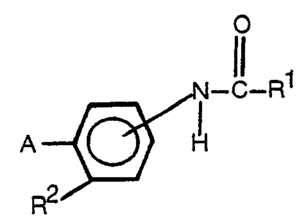

(I)

where $R^1$ represents hydrogen, cycloalkyl, alkyl, alkenyl or halogenated derivatives thereof; $R^2$ is halogen, trifluoromethyl, nitro, cyano, alkyl, alkoxy or alkylthio; and A represents a heterocyclic group.

US—A—3865670 discloses that compounds of formula

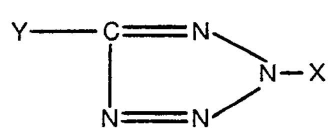

where Y is an aryl or heterocyclic group and X represents hydrogen, hydroxyalkyl, carboxylate ester or carboxyalkyl, and salts, esters, amides and nitriles thereof, have herbicidal properties.

The present invention provides, in accordance with a first embodiment, a series of herbicidal compounds of the formula

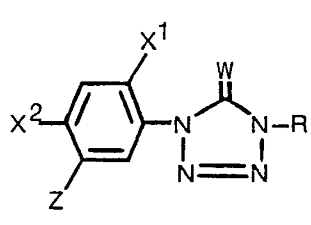

I

in which

W is oxygen or sulfur;

R is alkyl (preferably of 1 to 6 carbon atoms), haloalkyl (preferably of 1 to 5 carbon atoms), alkoxyalkyl (preferably of 2 to 6 carbon atoms), alkylthioalkyl (preferably of 2 to 6 carbon atoms), cyanoalkyl (preferably of 1 to 5 alkyl carbon atoms), haloalkoxyalkyl (preferably of 2 to 6 carbon atoms), trifluoromethylthio, alkenyl (preferably of 2 to 5 carbon atoms), or haloalkenyl (preferably of 2 to 5 carbon atoms); and

one of $X^1$ and $X^2$ is fluorine, chlorine, or bromine and the other is fluorine, chlorine, bromine, alkyl (preferably of 1 to 6 carbon atoms) such as methyl, or haloalkyl (preferably of 1 to 5 carbon atoms) such as bromomethyl, fluoromethyl, or trifluoromethyl. When $X^1$ is fluorine, chlorine, or bromine, $X^2$ may alternatively be nitro.

Z is

(a) hydrogen, fluorine, chlorine, bromine, cyano, nitro, alkyl, alkynyl, or alkyl substituted with fluorine, chlorine, bromine or $C_{1-4}$ alkoxy;

(b) a group $-QR^1$, $-OSO_2R^2$, $-QR^7CO-Q^1R^8$, $-QR^7CO_2N=C(R^9)(R^{10})$, $-QR^7C(CH_3)=R^{11}$, or $-QR^7CON(R^{12})(R^{13})$ in which

Q and $Q^1$ are independently oxygen or sulfur,

$R^1$ is alkyl, haloalkyl, hydroxyalkyl, cyanoalkyl, alkoxyalkyl, alkylthioalkyl, alkylsulfinylalkyl, alkylsulfonyalkyl, alkanoyl, alkenyl, haloalkenyl, alkenyloxyalkyl, alkynyl, haloalkynyl, alkoxycarbonyl, a three- to eight-membered ring heterocyclic group having one or two similar or different ring heteroatoms that are oxygen or sulfur, or an alkyl radical substituted with such a heterocyclic group,

$R^2$ is alkyl, haloalkyl, cyanoalkyl, phenylalkyl, cyclic alkyl, alkenyl, haloalkenyl, phenylalkenyl, alkynyl, haloalkynyl, phenylalkynyl, phenyl, or a group of the formula $-(CH_2)_mNR^3R^4$ or $(CH_2)_n-Y-R^5$ where

m is 0 or an integer from 1 to 5,

n is an integer from 1 to 5,

$R^3$ is hydrogen or alkyl,

$R^4$ is alkyl or a group $-CH_2-Y-R^5$,

$R^5$ is alkyl, alkenyl, alkynyl, or a radical $-CH(R^{18})CO_2R^{19}$,

$R^{18}$ and $R^{19}$ are independently hydrogen or alkyl, and

Y is oxygen or $S(O)_r$ where R is 0, 1 or 2,

$R^7$ is an alkylene or fluoroalkylene radical,

$R^8$ is hydrogen, alkyl, substituted alkyl, alkenyl, alkynyl, phenyl, 3-tetrahydrofuranyl, 2-oxo-3-tetrahydrofuranyl, 3-tetrahydrothienyl or the oxide or dioxide thereof, 3-pyridyl, cyclopropyl, cyclopentyl, cyclohexyl, cyclopropylmethyl, tetrahydrofurfuryl, furfuryl, thenyl, or benzyl,

one of $R^9$ and $R^{10}$ is alkyl and the other is alkyl or alkylthio,

$R^{11}$ is oxygen or $N-OR^{20}$ in which $R^{20}$ is hydrogen or alkyl, and

one of $R^{12}$ and $R^{13}$ is hydrogen, alkyl, or alkenyl and the other is hydrogen, alkyl, cyanoalkyl, alkoxyalkyl, alkenyl, alkynyl, alkylsulfonyl, arylsulfonyl (in which aryl is unsubstituted phenyl or phenyl substituted with halogen or alkyl), haloalkylsulfonyl, alkylaminosulfonyl, dialkylaminosulfonyl, or CH(H or $CH_3$)$CO_2$alkyl, or $N(R^{12})(R^{13})$ is a pyrrolidino, piperidino, or morpholino ring; or

(c) a group $-N(R^{14})(R^{15})$, $-SO_2R^{16}$, or $-CO-R^{17}$ in which

$R^{14}$ and $R^{15}$ are independently hydrogen, alkyl, alkenyl, alkynyl, cyanoalkyl, acetyl, alkoxycarbonyl, alkoxyalkyl, aminocarbonyl, alkylaminocarbonyl, dialkylaminocarbonyl, alkylsulfonyl, haloalkylsulfonyl, arylsulfonyl (in which aryl is unsubstituted phenyl or phenyl substituted with halogen or alkyl) or CH(H or $CH_3$)$CO_2$-alkyl, or $N(R^{14})(R^{15})$ is a group $N=C(R^9)(R^{10})$ in which one of $R^9$ and $R^{10}$ is alkyl and the other is hydrogen, alkyl or alkylthio, or $N(R^{14})(R^{15})$ is a tetrahydrophthalimido or 2-oxopyrrolidino group,

$R^{16}$ is hydroxy, amino, alkylamino, dialkylamino, or arylamino in which aryl is unsubstituted phenyl or phenyl substituted with halogen or alkyl, and

$R^{17}$ is hydroxy, alkoxy, alkylthio, amino, alkylamino, dialkylamino, or arylamino in which aryl is unsubstituted phenyl or phenyl substituted with halogen or alkyl.

In a first group of preferred compounds of this embodiment of the invention, W is oxygen and Z is nitro or $-QR^1$, especially alkoxyalkoxy, e.g. methoxymethoxy.

In a second such group, W is oxygen and Z is $-N(R^{14})(R^{15})$, where $R^{14}$ and $R^{15}$ are as defined above, or are salts thereof when the compound contains a free acidic hydrogen atom. $R^{14}$ and $R^{15}$ can, for example, both be hydrogen, or at least one of them can be alkylsulfonyl, haloalkylsulfonyl, $-CH(CH_3)CO_2$alkyl or $-CH_2CO_2$alkyl. Thus one may be hydrogen and the other alkylsulfonyl or $-CH(CH_3)_2CO_2$alkyl.

Preferred values of R are *n*-propyl and 3-fluoropropyl, with $X^1$ being fluorine or chlorine and $X^2$ chlorine or bromine. Especially preferred is the case where R is 3-fluoropropyl, $X^1$ is fluorine and $X^2$ is chlorine.

The present invention also provides compounds that are salts of compounds of Formula I having a free acidic hydrogen atom.

In a second embodiment of the invention, compounds have Formula I in which W, $X^1$ and $X^2$ are as defined above, and

(a) R is alkyl, haloalkyl, alkoxyalkyl, alkylthioalkyl, cyanoalkyl, haloalkoxyalkyl, trifluoromethylthio, alkenyl, haloalkenyl, 2-oxopropyl, or 3-oxopropyl; and Z is hydroxy, mercapto, or benzyloxy; or

(b) R is hydrogen and Z is fluorine, chlorine, bromine, cyano, nitro, alkyl, haloalkyl, alkynyl, or $QR^1$ in which Q is sulfur or oxygen and $R^1$ is alkyl, benzyl, haloalkyl, cyanoalkyl, alkoxyalkyl, alkylthioalkyl, alkylsulfinylalkyl, alkylsulfonylalkyl, alkenyl, haloalkenyl, alkenyloxyalkyl, alkynyl, or haloalkynyl or 2 to 5 carbon atoms.

In preferred compounds of this embodiment of the invention, W is oxygen, $X^1$ is fluorine or chlorine, $X^2$ is chlorine or bromine, and

(a) R is n-propyl or 3-fluoropropyl and Z is hydroxy, mercapto, or benzyloxy, or

(b) R is hydrogen and Z is benzyloxy or $C_{1-4}$alkoxy.

Separate aspects of the invention pertain to sub-genera for the individual substituent groups above. In one such aspect W will be oxygen.

In a sub-genus for R, that group may be alkyl or 1 to 6 carbon atoms such as n-$C_3H_7$, a fluoroalkyl radical of 1 to 5 carbon atoms and one or more fluorine atoms, especially a fluoropropyl radical such as 3-fluoropropyl, alkoxyalkyl or alkylthioalkyl or 2 to 4 carbon atoms such as methoxymethyl or its thio analog, cyanoalkyl of 1 to 3 alkyl carbon atoms such as cyanomethyl, fluoroalkoxyalkyl of 2 to 4 carbon atoms, for example 2-(difluoromethoxy)ethyl, alkenyl of 3 to 5 carbon atoms such as 2-propenyl, or haloalkenyl of 3 to 5 carbon atoms, for example, a fluoroalkenyl such as 3-fluoro-2-propenyl. In a preferred embodiment R is n-$C_3H_7$ or a fluoropropyl radical such as 3-fluoropropyl. Typical R groups include

$$-CH_3,\ -CH_2CH_3,\ -(CH_2)_2CH_3,\ -CH(CH_3)_2,\ -(CH_2)_3CH_3,\ -CH_2CH(CH_3)_2$$

$$-CH(CH_3)CH_2CH_3,\ -CHF_2,\ -CH_2CH_2F,\ -(CH_2)_2CH_2F,\ -(CH_2)_2CH_2Br,$$

$$-(CH_2)_2CHF_2,\ -CH_2CHFCH_3,\ -CH_2CF_2CH_3,\ -CH_2OCH_3,\ -CH_2OCH_2CH_3,$$

$$-CH_2SCH_3,\ -CH_2CN,\ -CH_2CH_2OCHF_2,\ -SCF_3,\ -CH{=}CH_2,\ -CH_2CH{=}CH_2,\text{ and }-CH_2CH{=}CHF.$$

With respect to sub-genera for $X^1$ and $X^2$, $X^1$ may be fluorine, chlorine, bromine, methyl, or trifluoromethyl and $X^2$ may be fluorine, chlorine, bromine, methyl, ethyl, bromomethyl, fluoromethyl, trifluoromethyl, or nitro, one of $X^1$ and $X^2$ being fluorine, chlorine, or bromine. In a preferred embodiment, $X^1$ and $X^2$ independently will be selected from fluorine, chlorine, and bromine. In a particularly preferred embodiment $X^1$ will be chlorine or, especially, fluorine and $X^2$ will be chlorine.

It will be understood that any alkyl, alkenyl or alkynyl groups of the compound may be straight chain or branched chain radicals. Thus, 1-methylethyl, 2-methyl-2-propenyl, and 1-methyl-2-propynyl are branched chain examples of alkyl, alkenyl, and alkynyl radicals respectively. The halogen may be fluorine, chlorine, bromine or iodine. The haloalkyl radical may have one or more same or different halogen atoms.

In one aspect of the invention, Z is hydrogen and W, R, $X^1$, and $X^2$ are as defined above. Preferably W is oxygen and R is n-propyl or a fluoropropyl radical such as 3-fluoropropyl. $X^1$ and $X^2$ are preferably independently selected from fluorine, chlorine, and bromine. More preferably, $X^1$ is chlorine or, especially, fluorine and $X^2$ is chlorine. The compounds in which Z is hydrogen are generally less active herbicidally than the corresponding compounds in which Z is one of the substituent groups described below.

Z may also be selected from fluorine, chlorine, bromine, cyano, nitro, alkyl (preferably of 1 to 6 carbon atoms) such as methyl, alkyl (preferably of 1 to 5 carbon atoms) substituted with one or more fluorine, chlorine, or bromine atoms or alkoxy groups of 1 to 4 carbons, and alkynyl (preferably of 3 to 5 carbon atoms) such as 2-propynyl. Examples of substituted alkyl include $-CF_3$ and acetal and ketal groups such as $-CH(OC_2H_5)_2$ and $-C(CH_3)(OC_2H_5)_2$. W, $X^1$, $X^2$, and R are as defined above, both generally and otherwise.

Z may also be a radical selected from the group consisting of

$$-QR^1,\ -OSO_2R^2,\ -Q-R^7-CO-Q^1R^8,\ -Q-R^7-CO_2-N{=}C(R^9)(R^{10}),$$

$$-Q-R^7-C(CH_3){=}R^{11},\ -Q-R^7-CO-N(R^{12})(R^{13}),\ -N(R^{14})(R^{15}),\ -SO_2R^{16},\text{ and }-CO-R^{17},$$

the substituents W, $X^1$, $X^2$, and R being as defined above both generically and otherwise.

Z is $-QR^1$

Q is sulfur or, preferably, oxygen and $R^1$ is alkyl (preferably of 1 to 6, more preferably of 1 to 4, carbon atoms), haloalkyl (preferably of 1 to 5 carbon atoms), particularly a fluoroalkyl, hydroxyalkyl (preferably of 1 to 5, more preferably of 1 to 3, carbon atoms), cyanoalkyl (preferably of 1 to 5 alkyl carbon atoms), alkoxyalkyl (preferably of 2 to 6 carbon atoms), alkylthioalkyl, alkylsulfinylalkyl, or alkylsulfonylalkyl (each preferably of 2 to 6 carbon atoms), alkanoyl (preferably of 2 to 5 carbon atoms), alkenyl (preferably of 2 to 5, more preferably of 3 to 5, carbon atoms), haloalkenyl (preferably of 2 to 5, more preferably of 3 to 5, carbon atoms) such as a halo-2-propenyl, alkenyloxyalkyl (preferably of 3 to 6 carbon atoms), alkynyl (preferably of 2 to 5, more preferably 3 to 5, carbon atoms), haloalkynyl (preferably of 2 to 5, more preferably 3 to 5, carbon atoms), alkoxycarbonyl (preferably of 1 to 4 alkyl carbon atoms), or a three- to eight-membered ring heterocyclic group of one or two, same or different (preferably the same), ring heteroatoms selected from oxygen and sulfur or an alkyl radical (preferably of 1 to 4, more preferably 1, carbon atoms) substituted with said heterocyclic group. $R^1$ is preferably 2-propynyl or 3-halo-2-propynyl such as 3-iodo-2-propynyl or 3-bromo-2-propynyl.

When $R^1$ is a heterocyclic group or an alkyl radical substituted therewith, the heterocyclic group may be saturated, unsaturated, or aromatic, it may be substituted with halogen (generally fluorine, chlorine, or bromine), alkyl, (preferably of 1 to 4 carbon atoms), or haloalkyl (preferably of 1 to 4 carbon atoms), and it may be adjoined to a benzene ring at two adjacent ring carbon atoms to form a benzo-heterocycle bicyclic group, for example a 1,4-benzodioxanyl group. In sulfur-containing heterocycles, the sulfur may be present in divalent form or as the S-oxide or S-dioxide. In a sub-genus of particular interest, the heterocyclic group

is saturated, unsubstituted, of 5 or 6 ring atoms including 1 or 2 same ring heteroatoms (sulfur or oxygen), and is connected directly to the oxygen moiety of the Z group or indirectly via methylene group. In this subgenus Z is advantageously 3-tetrahydrofuranyloxy or tetrahydrofurfuryloxy.

Examples of Z groups when Z is —$QR^1$ and $R^1$ is other than (and does not contain) a heterocyclic group include such radicals as

$—OCH_3$, $—OC_2H_5$, —O—n-$C_3H_7$, $—OCH(CH_3)_2$, —O—n-$C_4H_9$, $—OCH_2CH(CH_3)_2$, $—SCH_3$, $—SCH(CH_3)_2$, $—OCHF_2$, $—SCHF_2$, $—OCH_2CH_2F$, $—OCH_2CF_3$, $—SCH_2CF_3$, $—O(CH_2)_2CH_2F$, $—OCH_2CH_2Cl$, $—OCH_2CH_2Br$, $—OCH_2CH_2OH$, $—SCH_2CH_2OH$, $—OCH_2CN$, $—SCH_2CN$, $—OCH(CH_3)CN$, $—OCH(CN)CH(CH_3)_2$, $—OCH_2OCH_3$, $—OCH_2OC_2H_5$, $—SCH_2OCH_3$, $—O(CH_2)_2OCH_3$, $—OCH(CH_3)OCH_3$, $—O(CH_2)_2OC_2H_5$, $OCO_2C_2H_5$, $—S(CH_2)_2OCH_3$, $—OCH(CH_3)CH_2OCH_3$, $—OCH_2SCH_3$, $—SCH_2SCH_3$, $—OCH_2S(O)CH_3$, $—OCH(CH_3)SCH_3$, $—OCH(CH_3)S(O)CH_3$, $—OCH(CH_3)S(O)_2CH_3$, $—O(CH_2)_2OCH{=}CH_2$, $—S(CH_2)_2OCH{=}CH_2$, $—O(CH_2)_2OCH_2CH{=}CH_2$, $—OCOCH_3$, $—SCOCH_3$, $—OCOCH(CH_3)_2$, $—OCH_2CH{=}CH_2$, $OCH(CH_3)C{=}CH_2$, $—SCH_2CH{=}CH_2$, $—OCH_2C(Cl){=}CH_2$, $—SCH_2C(Cl){=}CH_2$, $—OCH_2CBr{=}CHBr$, $—OCH_2C{\equiv}CH$, $—SCH_2C{\equiv}CH$, $—OCH_2C{\equiv}CCH_3$, $—OC(CH_3)_2C{\equiv}CH$, $—OCH(CH_3)C{\equiv}CH$, $—OCH_2C{\equiv}CCl$, and $—OCH_2C{\equiv}CBr$.

Examples of Z when $R^1$ is or contains a heterocyclic group include

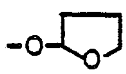 , 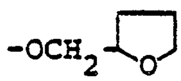 , 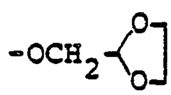 , 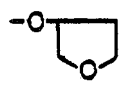 , 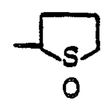 ,

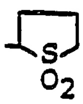 , 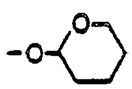 , 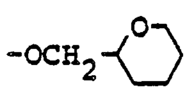 , 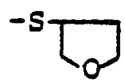 , 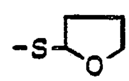 ,

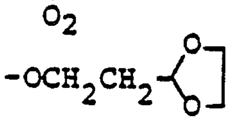 , 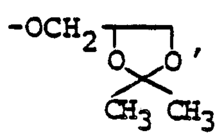 , 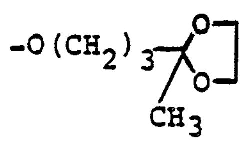 , 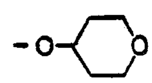 ,

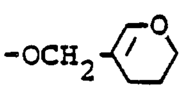 , 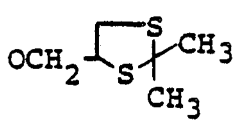 , 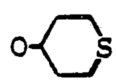 ,

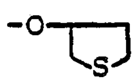 , 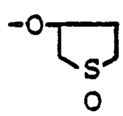 , 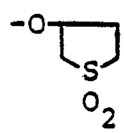 , 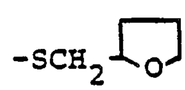 ,

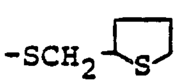 , 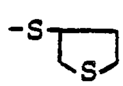 , 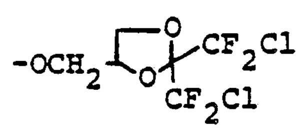 ,

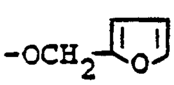 , 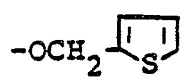 , 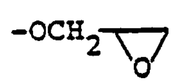 ,

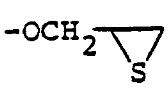 , 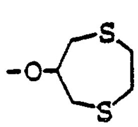 , 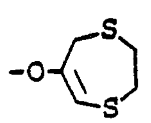 , and 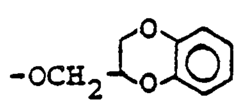 .

Z is $—OSO_2R^2$

$R^2$ is alkyl (preferably of 1 to 8 carbon atoms), haloalkyl, cyanoalkyl, or arylalkyl wherein each alkyl is preferably of 1 to 5 carbon atoms, cyclic alkyl (preferably of 3 to 8 carbon atoms), alkenyl, haloalkenyl, or arylalkenyl wherein each alkenyl is preferably of 2 to 5 carbon atoms, alkynyl, haloalkynyl, or arylalkynyl wherein each alkynyl is preferably of 2 to 5 carbon atoms, aryl such as phenyl, or a group of the formula $—(CH_2)_mNR^3R^4$ or $(CH_2)_n—Y—R^5$ wherein m is 0 to 5 (preferably 0 to 3) and n is 1 to 5 (preferably 1 to 3);

$R^3$ is hydrogen or alkyl (preferably of 1 to 5 carbon atoms);

$R^4$ is alkyl (preferably of 1 to 5 carbon atoms) or a group $—CH_2—Y—R^5$;

$R^5$ is alkyl (preferably of 1 to 5 carbon atoms), alkenyl or alkynyl (preferably of 2 to 5, more preferably of 3 to 5, carbon atoms), or a radical $—CH(R^{18})CO_2R^{19}$;

$R^{18}$ and $R^{19}$ are independently hydrogen or alkyl (preferably of 1 to 4 carbon atoms); and

Y is oxygen or $S(O)_r$ in which r is 0 to 2.

A number of interesting compounds within the above subgenus comprise the series wherein $R^2$ is alkyl of 1 to 8 carbon atoms, haloalkyl, cyanoalkyl, or arylalkyl wherein each alkyl is of 1 to 5 carbon atoms, cyclic alkyl of 3 to 8 carbon atoms, alkenyl or haloalkenyl of 3 to 5 carbon atoms, alkynyl or haloalkynyl of 3 to 5 carbon atoms, or a group $(CH_2)_mNR^3R^4$ or $(CH_2)_n—Y—R^5$ wherein m is 0 to 3, n is 1 to 3, and $R^3$ and $R^4$ are independently alkyl of 1 to 5 carbon atoms.

Frequently, $R^2$ will be alkyl of 1 to 5 carbon atoms, especially methyl; haloalkyl of 1 to 3 carbon atoms, having one or more fluorine, chlorine, or bromine atoms; cyanoalkyl or phenylalkyl of 1 to 3 alkyl carbon atoms; cyclic alkyl of 3 to 6 carbon atoms; alkenyl or alkynyl of 3 to 5 carbon atoms; haloalkenyl of 3 to 5 carbon atoms such as a halopropenyl, for example, a halo-2-propenyl having one or more halogen atoms such as chlorine; haloalkynyl of 3 to 5 carbon atoms, especially a 3-halo-2-propynyl, a group of the formula $(CH_2)_mN(R^3)_2$ in which m is 0 or 2 and $R^3$ is alkyl of 1 to 5 carbon atoms such as methyl; or a group of the formula $(CH_2)_n—Y—R^5$ in which n is 1 or 2, especially 2, Y is oxygen or sulfur, and $R^5$ is alkyl of 1 to 5 carbon atoms such as methyl or ethyl, alkenyl or alkynyl of 3 to 5 carbon atoms, or a radical $—CH(R^{18})CO_2R^{19}$ in which $R^{18}$ is hydrogen or methyl and $R^{19}$ is alkyl of 1 to 4 carbon atoms such as methyl or ethyl.

Examples of specific $R^2$ groups include

$CH_3$, $C_2H_5$, n-$C_3H_7$, $CH(CH_3)_2$, n-$C_4H_9$, $CH_2CH(CH_3)_2$, $CH(CH_3)CH_2CH_3$,

$(CH_2)_2CH(CH_3)_2$, $CF_3$, $CHF_2$, $CH_2Cl$, $CHCl_2$, $CH_2Br$, $(CH_2)_2CH_2Cl$, $CH_2CN$,

$CH_2C_6H_5$, $CHCH_2CH_2$, $CH=CH_2$, $CH_2CH=CH_2$, $CH_2C(Cl)=CCl_2$, $CH=CHC_6H_5$,

$CH_2C\equiv CH$, $CH_2C\equiv CCl$, $C_6H_5$, $NHCH_3$, $N(CH_3)_2$, $(CH_2)_2N(CH_3)_2$,

$(CH_2)_2OCH_3$, $(CH_2)_2OCH_2CH_3$, $(CH_2)_2OCH_2CH=CH_2$, $(CH_2)_2SCH_2CH=CH_2$,

$(CH_2)_2OCH_2C\equiv CH$, $(CH_2)_2OCH_2CO_2CH_3$, and $(CH_2)_2SCH_2CO_2CH_3$.

Z is $—Q—R^7—CO—Q^1R^8$

Q and $Q^1$ are independently oxygen or sulfur.

$R^7$ is an alkylene or haloalkylene (such as fluoroalkylene) radical, preferably of 1 to 3 carbon atoms, for example, $—CH_2—$, $—CH(CH_3)—$, or $—CHF—$.

$R^8$ is hydrogen, alkyl or substituted alkyl (preferably of 1 to 8 carbon atoms), alkenyl or alkynyl (preferably of 2 to 5, more preferably 3 to 5, carbon atoms), for example, 2-propenyl, 1-methyl-2-propenyl, or 2-propynyl, or a monovalent cyclic group of 3 to 7 ring atoms which may be an aromatic (such as phenyl), heterocyclic (such as 3-tetrahydrofuranyl, 2-oxo-3-tetrahydrofuranyl, 3-tetrahydrothienyl or the oxide or dioxide thereof, or 3-pyridyl), or alicyclic (such as cyclopropyl, cyclopentyl, or cyclohexyl) radical, the valence being on a carbon atom of said cyclic group, or an alkyl radical of 1 to 3 (preferably 1) carbon atoms substituted with said cyclic group, for example, cyclopropylmethyl, tetrahydrofurfuryl, furfuryl, thenyl, or benzyl.

When $R^8$ is substituted alkyl, the alkyl substituent(s) will frequently be selected from nitro, halo (such as chloro, bromo, or, particularly, fluoro), cyano, alkoxy or alkyl(thio, sulfinyl, or sulfonyl) of 1 to 4 alkyl carbon atoms, phenyl(thio, sulfinyl, or sulfonyl), alkylamino or dialkylamino in which each alkyl independently is preferably of 1 to 4 carbon atoms, aminocarbonyl, or alkylaminocarbonyl or dialkylaminocarbonyl in which each alkyl independently is preferably of 1 to 4 carbon atoms.

Thus, in this aspect of the invention, Z may be $—OR^7CO_2R^8$, $—SR^7—CO—SR^8$, $—OR^7—CO—SR^8$, or $—SR^7CO_2R^8$.

In a narrower aspect of the invention Q is oxygen or sulfur (frequently oxygen), $Q^1$ is oxygen, $R^7$ is $—CH_2—$ or $—CH(CH_3)—$, and $R^8$ is hydrogen, alkyl, haloalkyl (particularly a fluoroalkyl), cyanoalkyl, alkoxyalkyl, cyclic alkyl such as cyclopentyl, (cyclic alkyl)alkyl such as cyclopropylmethyl, or alkynyl.

Examples of Z groups when Z is $—Q—R^7—CO—Q^1R^8$ include

$OCH_2CO_2H$, $OCH_2CO_2C_2H_5$, $SCH_2CO_2H$, $SCH_2—CO—SCH_3$, $—OCH(CH_3)CO_2C_2H_5$, $OCH(CH_3)CO_2CH(CH_3)_2$, $OCH(CH_3)CO_2C(CH_3)_3$, $OCH(CH_3)CO_2CH(CH_2CH_3)_2$, $OCH(CH_3)CO_2CH(CH_3)CH(CH_3)_2$, $OCH(CH_3)CO_2CH[CH(CH_3)_2]_2$, $OCH(CH_3)CO_2CH(CH_3)CH_2CH_3$, $OCH(CH_3)CO_2\underline{CH(CH_2)_3CH_2}$, $OCH(CH_3)CO_2H$, $SCH(CH_3)CO_2CH(CH_3)_2$, $OCH(CH_3)CO_2CH_2\underline{CHCH_2CH_2}$, $SCH(CH_3)CO_2H$, $OCH(CH_3)CO_2C(CN)(CH_3)_2$, $OCH(CH_3)CO_2CH(CH_2OCH_3)_2$, $OCH(CH_3)CO_2CH(CH_3)C{\equiv}CH$, $OCH(CH_3)CO_2C(CH_3)_2C{\equiv}CH$, $OCH(CH_3)CO_2CH_2CH_2F$, $OCH(CH_3)CO_2CH_2CF_3$, $OCH(CH_3)CO_2CH(CH_2F)_2$, $OCHFCO_2H$, $SCHFCO_2H$, $OCHFCO_2CH_3$, $OCH_2CO—SC_2H_5$, $OCH(CH_3)—CO—SC_2H_5$, $—OCH(CH_3)CO_2CH_2CH{=}CH_2$, $—SCH(CH_3)CO_2CH_2CH{=}CH_2$,

$-OCH_2CO_2C_6H_5$, 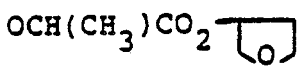 , 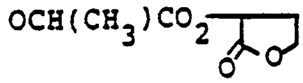 ,

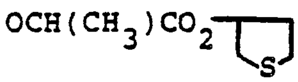 , 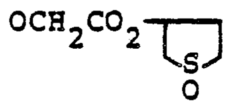 ,

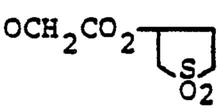 , 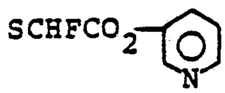 , $OCH(CH_3)CO_2\underline{CH(CH_2)_4CH_2}$,

$SCH_2CO_2\underline{CHCH_2CH_2}$, 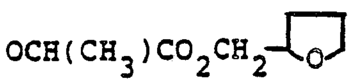 , 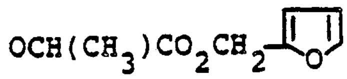 ,

$OCH(CH_3)CO_2CH_2C_6H_5$, 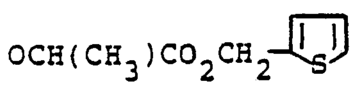 ,

$OCH(CH_3)CO_2CH_2CH_2N(CH_3)_2$, $OCH(CH_3)CO_2CH_2CON(C_2H_5)_2$, $—SCH(CH_3)CO_2CH_2OCH_3$, $OCH(CH_3)CO_2CH_2SCH_3$, $OCH(CH_3)CO_2CH_2SOCH_3$, $OCH(CH_3)CO_2CH_2SO_2CH_3$, $OCH(CH_3)CO_2CH_2CONH_2$, $OCH(CH_3)CO_2CH_2CH(NO_2)CH_3$, $OCH(CH_3)CO_2CH_2CN$, and $OCH(CH_3)CO_2CH_2SC_6H_5$.

The free acids (Z is $—QR^7CO_2H$) may be converted into their salts such as their sodium, potassium, calcium, ammonium, magnesium, or mono-, di-, or tri($C_1$ to $C_4$ alkyl)ammonium salts which may also be used as herbicides.

Z is $—Q—R^7—CO_2—N{=}C(R^9)(R^{10})$

Q and $R^7$ are as defined above.

One of $R^9$ and $R^{10}$ is alkyl and the other is alkyl or alkylthio, each alkyl (independently) preferably being of 1 to 4 carbon atoms.

Examples of Z include

$OCH(CH_3)CO_2N{=}C(CH_3)_2$, $SCH_2CO_2N{=}C(CH_3)_2$, $OCHFCO_2N{=}C(SCH_3)(CH_3)$, $OCH(CH_3)CO_2N{=}C(SCH_3)(CH_3)$, $OCH(CH_3)CO_2N{=}C(CH_3)(C_2H_5)$, and $OCH_2CO_2N{=}C(CH_3)_2$.

Z is $—Q—R^7—C(CH_3){=}R^{11}$

Q and $R^7$ are as defined above.

$R^{11}$ is oxygen or $N—OR^{20}$ in which $R^{20}$ is hydrogen or alkyl (preferably of 1 to 4 carbon atoms).

Examples of Z substituents for this sub-genus include

$OCH(CH_3)C(CH_3)=N—OH$, $SCH(CH_3)C(CH_3)=N—OH$, $OCH_2C(CH_3)=N—OH$, $OCHFC(CH_3)=N—OH$,

$SCH(CH_3)C(CH_3)=N—OCH_3$, $OCH(CH_3)C(CH_3)=N—OCH(CH_3)_2$, $OCH(CH_3)COCH_3$, $SCH(CH_3)COCH_3$,

$OCHFCOCH_3$, $OCH_2COCH_3$, and $SCH_2COCH_3$.

Z is —Q—$R^7$—CO—N($R^{12}$)($R^{13}$)

Q and $R^7$ as defined above.

N($R^{12}$)($R^{13}$) is $NH_2$ or the residue of a primary or secondary amine or of a sulfonamide. For example, one of $R^{12}$ and $R^{13}$ may be hydrogen, alkyl (preferably of 1 to 4 carbon atoms), or alkenyl (preferably of 3 to 5 carbon atoms) such as 2-propenyl, and the other may be hydrogen, alkyl (preferably of 1 to 4 carbon atoms), cyanoalkyl (preferably of 1 to 4 alkyl carbon atoms), alkoxyalkyl (preferably of 2 to 4 carbon atoms), alkenyl (preferably of 3 to 5 carbon atoms) such as 2-propenyl, alkynyl (preferably of 3 to 5 carbon atoms) such as 2-propynyl, alkylsulfonyl (preferably of 1 to 4 carbon atoms), arylsulfonyl in which aryl is unsubstituted phenyl or phenyl substituted with halogen (such as fluorine or chlorine) or alkyl (preferably of 1 to 4 carbon atoms such as methyl), haloalkylsulfonyl (preferably of 1 to 4 carbon atoms), for example, a fluoroalkylsulfonyl such as trifluoromethylsulfonyl, alkylaminosulfonyl or dialkylaminosulfonyl in which each alkyl independently is preferably of 1 to 4 carbon atoms, or CH(H or $CH_3$)$CO_2$alkyl in which alkyl is preferably of 1 to 4 carbon atoms. $R^{12}$ and $R^{13}$ may also be taken together and with the nitrogen atom form a 5- or 6-membered heterocyclic ring which may contain an oxygen atom in the ring, for example, N($R^{12}$)($R^{13}$) may be a pyrrolidino, piperidino, or morpholino ring.

In one aspect of this embodiment, $R^{12}$ and $R^{13}$ will be the same or will be taken together to form a heterocyclic ring. In another aspect one of $R^{12}$ and $R^{13}$ will be alkyl or, frequently, hydrogen and will be different from the other.

Examples of Z substituents for this embodiment include

$OCH(CH_3)CONH_2$, $OCH(CH_3)CON(CH_3)_2$, $OCH(CH_3)CON(CH_2CH=CH_2)_2$, $OCH(CH_3)CONHSO_2C_6H_5$,

$OCH(CH_3)CONHSO_2—C_6H_4(2\text{-}Cl)$, $OCH(CH_3)CONHSO_2—C_6H_4(4\text{-}Cl)$, $OCH_2CON(CH_3)(C_2H_5)$,

$OCH(CH_3)CONHCH(CH_3)_2$, $OCHFCONHSO_2—n\text{-}C_3H_7$,

$OCH(CH_3)CONHCH_2C\equiv CH$, $OCH(CH_3)CONHCH_2CO_2CH_3$,

$OCH(CH_3)CONHSO_2—C_6H_4(4\text{-}CH_3)$, $OCH(CH_3)CONHC(CH_3)_2C\equiv CH$, $OCH(CH_3)CONHC(CH_3)_2CN$,

$OCH(CH_3)CONHSO_2CF_3$, $OCH(CH_3)CONHSO_2NHCH_3$, $OCH(CH_3)CONHSO_2N(CH_3)_2$,

$OCH(CH_3)CONHCH_2CH_2OCH_3$, $OCH_2CONH_2$, $OCHFCONHCH_3$, $OCH(CH_3)CONHCH(CH_3)CO_2C_2H_5$,

$SCH(CH_3)CON(CH_3)$, $SCH(CH_3)CONHCH_2CH=CH_2$, $OCH(CH_3)CON(CH_2)_4CH_2$,

$OCH(CH_3)CON(CH_2)_3CH_2$, $OCH(CH_3)CON(CH_2)_2OCH_2CH_2$, and $SCH_2CONHSO_2CF_3$.

Z is N($R^{14}$)($R^{15}$)

$R^{14}$ and $R^{15}$ are independently selected from hydrogen, alkyl (preferably from 1 to 4 carbon atoms), alkenyl or alkynyl (preferably of 3 to 5 carbon atoms), cyanoalkyl (preferably of 1 to 4 alkyl carbon atoms), acetyl, alkoxycarbonyl (preferably of 1 to 4 alkyl carbon atoms), alkoxyalkyl (preferably of 2 to 4 carbon atoms), aminocarbonyl, alkylaminocarbonyl or dialkylaminocarbonyl in which each alkyl is preferably of 1 to 4 carbon atoms, alkylsulfonyl (preferably of 1 to 4 carbon atoms), haloalkylsulfonyl (preferably of 1 to 4 carbon atoms), for example, a fluoroalkylsulfonyl such as trifluoromethylsulfonyl, arylsulfonyl in which aryl is unsubstituted phenyl or phenyl substituted with halogen (such as fluorine or chlorine) or alkyl (preferably of 1 to 4 carbon atoms such as methyl), and CH(H or $CH_3$)$CO_2$—alkyl in which alkyl is preferably of 1 to 4 carbon atoms, or N($R^{14}$)($R^{15}$) is a group N=C($R^9$)($R^{10}$) in which one of $R^9$ and $R^{10}$ is alkyl and the other is hydrogen, alkyl, or alkylthio, each alkyl (independently) preferably being of 1 to 4 carbon atoms. N($R^{14}$)($R^{15}$) may also represent a cyclic group such as tetrahydrophthalimido or 2-oxopyrrolidino.

Examples of Z groups for this embodiment include

$NHCON(CH_3)_2$, $NHCONH_2$, $NHCONHCH_2CH(CH_3)_2$, $NHCONHCH_3$, $NH_2$, $N(CH_3)_2$, $NHCH_3$,

$N(C_2H_5)_2$, $NHCH_2CH=CH_2$, $NHCH_2C\equiv CH$, $N(CH_2CN)COCH_3$, $N(C_2H_5)COCH_3$, $NHCOCH_3$,

$NHCH_2CO_2C_2H_5$, $NHCO_2CH_3$, $NHCH(CH_3)CO_2C_2H_5$, $NHCH_2CH_2OCH_3$, $NHSO_2CH_3$,

$N(CH_3)SO_2CH_3$, $N(C_2H_5)SO_2CH_3$, $N(SO_2CH_3)_2$, $NHSO_2CF_3$, $N(SO_2CF_3)_2$,

$NHSO_2C_6H_5$, $NHSO_2—C_6H_4(4\text{-}Cl)$, $NHSO_2—C_6H_4(4\text{-}CH_3)$, $N{=}C(CH_3)_2$,

$N{=}CHCH_3$, $N{=}C(CH_3)(C_2H_5)$,

$N{=}C(SCH_3)(CH_3)$, 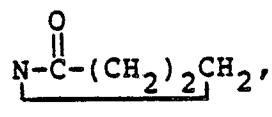, and 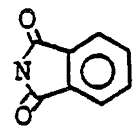.

Z is $SO_2R^{16}$

$R^{16}$ is hydroxy, amino, alkylamino or dialkylamino in which each alkyl is preferably of 1 to 4 carbon atoms, or arylamino in which aryl is unsubstituted phenyl or phenyl substituted with halogen (such as fluorine or chlorine) or alkyl (preferably of 1 to 4 carbon atoms such as methyl).

Examples of Z groups for this embodiment include

$SO_3H$, $SO_2HN_2$, $SO_2NHCH_3$, $SO_2N(C_2H_5)_2$, $SO_2N(CH_3)(C_2H_5)$, $SO_2NHCH(CH_3)_2$,

$SO_2NHC_6H_5$, $SO_2NHC_6H_4(4\text{-}Cl)$, and $SO_2NHC_6H_4(4\text{-}CH_3)$.

Z is $CO—R^{17}$

$R^{17}$ is hydroxy, alkoxy or alkylthio (preferably of 1 to 4 carbon atoms), amino, alkylamino or dialkylamino in which each alkyl is preferably of 1 to 4 carbon atoms, or arylamino in which aryl is unsubstituted phenyl or phenyl substituted with halogen (such as fluorine or chlorine) or alkyl (preferably of 1 to 4 carbon atoms, such as methyl). Acid salts (same as above) are also useful.

Examples of Z groups for this embodiment include

$CO_2H$, $CO_2CH_3$, $CO_2CH(CH_3)_2$, $CO—SCH_3$, $CONH_2$, $CONHCH_3$, $CON(C_2H_5)_2$,

$CON(CH_3)(C_2H_5)$, $CONHCH(CH_3)_2$, $CONHC_6H_5$, $CONHC_6H_4(4\text{-}Cl)$, and $CONHC_6H_4(4\text{-}CH_3)$.

For each of the Z substituents described above, a preferred embodiment comprises the compounds in which W is oxygen, R is n-propyl or, particularly, a fluoropropyl such as 3-fluoropropyl, $X^1$ is chlorine or, especially, fluorine, and $CO_2$ is chlorine. The compounds in which Z is $OR^1$ have very high herbicidal activity, especially where $R^1$ is 2-propynyl (or a 3-halo-2-propynyl such as 3-iodo- or bromo-2-propynyl).

Any free acid compound of formula I may be converted into a salt such as a sodium, potassium, calcium, ammonium, magnesium, or mono-, di- or tri($C_1$ to $C_4$ alkyl)ammonium salt which may also be used as an herbicide.

Many of the present herbicidal compounds may be prepared by the methods illustrated in the chemical equations below.

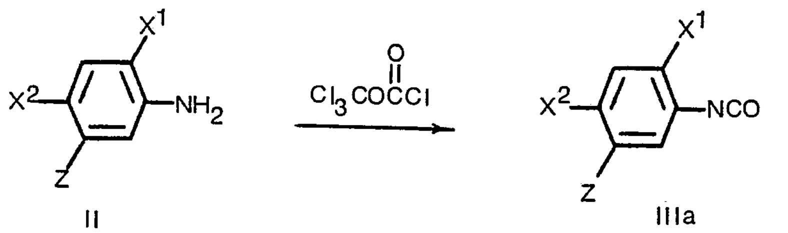


II IIIa

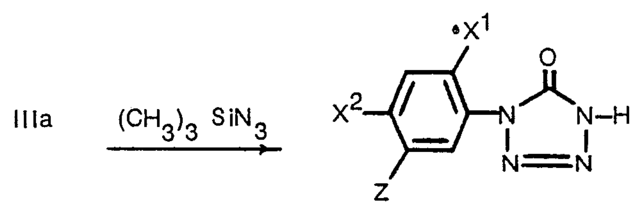


IVa

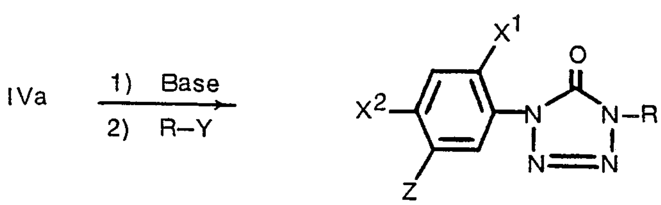


Ia

Treatment of an appropriately substituted phenylamine, II, with trichloromethyl chloroformate gives the corresponding isocyanate IIIa, which upon treatment with trimethylsilyl azide in the manner of O. Tsuge et al., *J. Org. Chem.*, *45*, 5130 (1980), incorporated herein by reference, affords the intermediate tetrazolinone IVa. Intermediate IVa may also be prepared by treatment of the isocyanate IIIa with aluminum azide in the manner of J. Horwitz et al., *J. Am. Chem. Soc.*, *81*, 3076 (1959), incorporated herein by reference. Treatment of the N-4 unsubstituted tetrazolinone IVa with R—Y, wherein Y is a good leaving group, in the presence of a base gives compound Ia. The leaving group Y will generally be a chlorine, bromine, or iodine atom, but may be any readily displaceable group used in the art in similar reactions. The use of sodium hydride base in dimethylformamide has been found to give satisfactory results. This method is generally useful where compound II is readily available, either commercially or by preparation, and is particularly useful where Z is hydrogen, halogen, cyano alkyl, substituted alkyl, or —$OR^1$ wherein $R^1$ is alkyl, especially lower alkyl such as methyl, or benzyl (a useful intermediate discussed below).

The corresponding thiones Ib (I, W is sulfur) may be prepared in a similar manner by treating the appropriately substituted phenylamine II with thiophosgene in the presence of triethylamine to give corresponding isothiocyanate (IIIb) and treating that first with sodium azide and water, then with acid (e.g. $HCl/H_2O$) to give the thione (IVb) which may be concerted by reaction with R—Y (as above for IVa) to the thione (Ib).

In some instances the desired Z group may be unstable under the conditions used in preparing II or in converting II into I. In such cases or where it is otherwise not desirable or convenient to proceed by the methods outlined above, it may be advantageous to add the desired Z substituent to the molecule subsequent to the addition of the desired R group (or a group that can be subsequently converted into the desired R group). In such cases a useful and versatile intermediate is compound I in which Z is —QH (the 5-hydroxyphenyl compound, V, or the corresponding 5-mercaptophenyl compound, VI).

Compound V (Z is OH) is readily prepared from the corresponding compound I in which Z is a lower alkoxy group or a benzyloxy group by treatment with an acidic reagent such as concentrated sulfuric acid, concentrated hydrobromic acid, or a mixture of hydrobromic and acetic acids to effect dealkylation, or, where Z is benzyloxy, by hydrogenolysis over palladium on charcoal ($H_2/Pd/C/C_2H_5OH$).

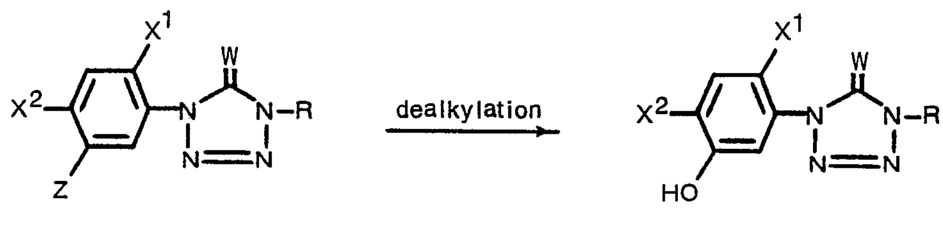


I(Z=O—Alkyl or $OCH_2C_6H_5$) V

The 5-mercaptophenyl compound (VI) may be prepared from the corresponding compound in which Z is hydrogen by the following sequence of steps: nitration (e.g. $HNO_3/H_2SO_4$) to give the corresponding 5-$NO_2$ compound (I, Z is $NO_2$), reduction (e.g. $H_2/PTO_2/C_2H_5OH$) to give the corresponding 5-$NH_2$ compound (I, Z is $NH_2$), treatment first with $NaNO_2/HCl$ then with $SO_2/CuCl$ to give the corresponding 5-$SO_2Cl$ compound (I, Z is $SO_2Cl$), and, finally, reduction (e.g. Sn/HCl) to give the 5-SH compound VI (I, Z is SH).

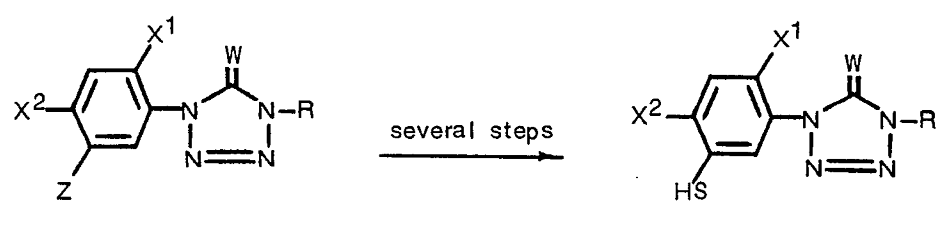


I(Z=H) VI

The appropriate compound V (I, Z = OH) or compound VI (I, Z = SH) is then treated in the presence of a base with the appropriate Y—$R^1$, Y—$SO_2R^2$, Y—$R^7$—CO—$QR^8$($R^8 \neq$H), Y—$R^7$—$CO_2$—N=C($R^9$)($R^{10}$), or Y—$R^7$—C($CH_3$)=$R^{11}$, wherein Y is a good leaving group, to give compound I in which Z is —$QR^1$, —$OSO_2R^2$, —$QR^7$CO—$QR^8$($R^8 \neq$H), —$QR^7CO_2N$=C($R^9$)($R^{10}$), or —$QR^7C(CH_3)=R^{11}$.

Alternatively, compounds of formula I in which Z is —$QR^7$—CO—$SR^8$ or —$QR^7CO_2N$=C($R^9$)($R^{10}$) may be prepared from the corresponding compound in which Z is —$QR^7CO_2R^8$ (ester, prepared as described above) by hydrolysis to the acid (Z is —$QR^7CO_2H$), followed by conversion to the acid chloride (Z is —$QR^7COCl$), then either by treatment with $R^8SH$ to give I in which Z is —$QR^7$—CO—$SR^8$ or by treatment with ($R^9$)($R^{10}$)C=N—OH to give I in which Z is —$QR^7CO_2N$=C($R^9$)($R^{10}$).

Similarly, compounds of formula I in which Z is $—QR^7CON(R^{12})(R^{13})$ may be prepared from the corresponding compound in which Z is $—QR^7COCl$ (prepared as described above) by treatment $HN(R^{12})(R^{13})$.

An alternative method for preparing the compounds of formula I in which Z is $—QR^7C(CH_3)=R^{11}$ wherein $R^{11}$ is $N—OR^{20}$ is by reacting the corresponding compound in which $R^{11}$ is oxygen (prepared as described above) with $H_2N—OR^{20}$.

In the conversion of V or VI into I described above, the base and the leaving group Y may be selected from those used in the art for similar reactions. The leaving group Y in $R^2SO_2Y$ will usually be chlorine or bromine. Examples of suitable bases for the reaction with $R^2SO_2Y$ are sodium hydride and triethylamine. For $R^1—Y$, Y will generally be chlorine, bromine, iodine, or 4-methylphenylsulfonyloxy. Suitable bases include sodium hydride and, where Y is bromine, chlorine, or iodine, potassium carbonate and potassium fluoride.

Compound I in which Z is $SO_2R^{16}$ may be prepared from the corresponding compound in which Z is $SO_2Cl$ (prepared as described above) by treatment with water ($R^{16}$ is hydroxy), ammonia ($R^{16}$ is $NH_2$) or an appropriate amine ($R^{16}$ is alkylamino, dialkylamino, or arylamino).

Compounds of formula I in which Z is cyano (which may be prepared as described above from II) are useful intermediates for the corresponding compounds in which Z is $—CO—R^{17}$ by hydrolysis followed by esterification or amide formation as the case may be.

Compounds of formula I in which Z is $—N(R^{14})(R^{15})$ may be prepared by reduction of the corresponding nitro compound ($Z=NO_2$, prepared as described above) to give the amino derivative ($Z=NH_2$, discussed above) followed by appropriate alkylation, acylation, or sulfonylation as the case may be. The amino compound may also be converted into the corresponding isocyanate (Z=NCO) by treatment with trichloromethyl chloroformate. Treatment of the isocyanate (Z=NCO) with an appropriate amine gives the corresponding compound in which Z is $—N(R^{14})(R^{15})$ and one of $R^{14}$ and $R^{15}$ is hydrogen and the other is aminocarbonyl, alkylaminocarbonyl or dialkylaminocarbonyl. The amino compound ($Z=NH_2$) may also be used to prepare the compounds in which $N(R^{14})(R^{15})$ is the group $N=C(R^9)(R^{10})$ (by treatment with $R^9R^{10}C=O$) or a cyclic group such as a tetrahydrophthalimido (by treatment with tetrahydrophthalic anhydride).

Compounds of formula I in which Z and $X^1$ are halogen atoms and $X^2$ is nitro may be prepared by nitration (e.g. $HNO_3/H_2SO_4$) of the corresponding compound in which $X^2$ is hydrogen (e.g. compound 226 in the tables is converted by nitration to compound 217). The Z halogen in the nitro compounds (Z and $X^1$ are halogen, $X^2$ is nitro) may be readily displaced to produce other herbicidal compounds of the invention, for example, by reaction with $R^1—OH$ in the presence of a base (e.g. NaH) to give the corresponding compound in which Z is $—OR^1$.

Certain of the present compounds may be prepared from intermediate V by the method illustrated in the following chemical equation

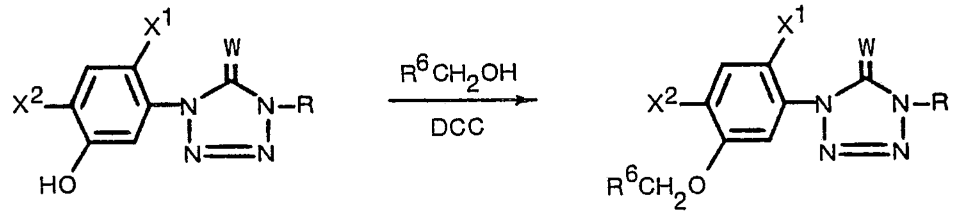


V Ic

The moiety $R^6CH_2$ represents appropriate $R^1$ substituents. The reaction is conducted in the presence of a dehydration reagent such as dicyclohexylcarbodiimide (DCC).

Where $R^1$ is a saturated oxygen- or sulfur-containing heterocycle, a further method of preparation involves addition of the hydroxy group of V or the mercapto group of VI across the double bond of a dihydroheterocycle as shown in the equation below for 2-tetrahydrofuranyl.

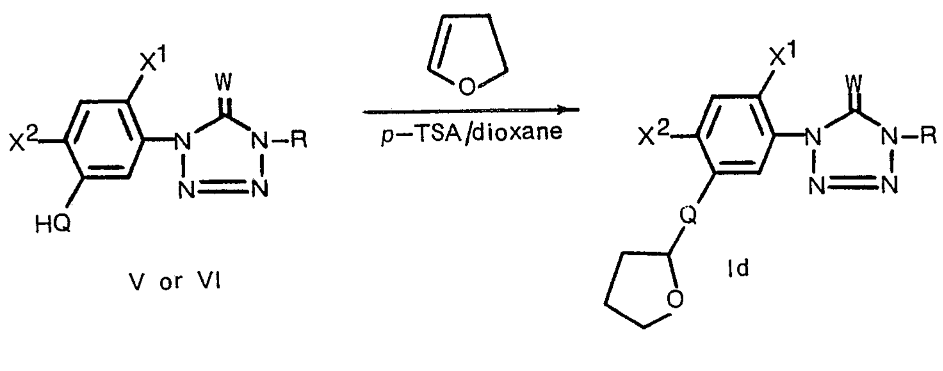


V or VI Id

The compounds in which R is a difluoroalkyl radical in which both fluorine atoms are on the same carbon atoms such as $—(CH_2)_2CHF_2$ or $—CH_2CF_2CH_3$ may be prepared from the corresponding aldehyde or ketone (e.g. $—CH_2CH_2CHO$ or $—CH_2COCH_3$) by treatment with diethylaminosulfur trifluoride ($ET_2N—SF_3$, "DAST") in the presence of methylene chloride.

The methods described above and in the examples for preparing compound I, and intermediates therefor, comprise a further aspect of the present invention.

An additional aspect of the invention pertains to the intermediates themselves, particularly compounds IV, V, VI, and VII.

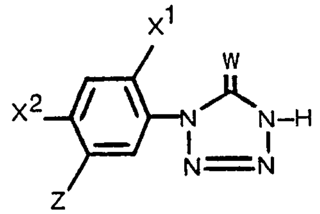

IV

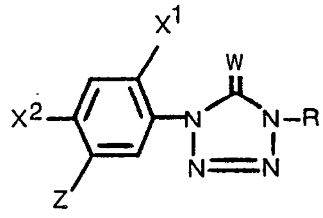

V (Z=OH)
VI (Z-SH)
VII ($Z=OCH_2C_6H_5$)

With respect to compound IV, W is sulfur or, preferably, oxygen, one of $X^1$ and $X^2$ is fluorine, chlorine, or bromine and the other is fluorine, chlorine, bromine, alkyl (preferably of 1 to 6 carbon atoms, more preferably of 1 to 4 carbon atoms) such as methyl, or haloalkyl (preferably of 1 to 5 carbon atoms) particularly fluoromethyl, trifluoromethyl, or bromomethyl, (or $X^2$ may be $NO_2$ when $X^1$ is F, Cl, or Br), and Z is hydrogen, fluorine, chlorine, bromine, cyano, nitro, alkyl (preferably of 1 to 6 carbon atoms) such as methyl, haloalkyl (preferably of 1 to 5 carbon atoms) such as trifluoromethyl, alkynyl (preferably of 3 to 5 carbon atoms), or $QR^1$ in which Q is sulfur or, preferably, oxygen and $R^1$ is alkyl (preferably of 1 to 6, more preferably of 1 to 4, carbon atoms), haloalkyl (preferably of 1 to 5 carbon atoms), particularly a fluoroalkyl, cyanoalkyl (preferably of 1 to 5 carbon atoms), alkoxyalkyl (preferably of 2 to 6 carbon atoms), alkylthioalkyl, alkylsulfinylalkyl, or alkylsulfonylalkyl (each preferably of 2 to 6 carbon atoms), alkenyl (preferably of 2 to 5, more preferably of 3 to 5, carbon atoms), haloalkenyl (preferably of 2 to 5, more preferably of 3 to 5, carbon atoms) such as a halo-2-propenyl, alkenyloxyalkyl (preferably of 3 to 6 carbon atoms), alkynyl (preferably of 2 to 5, more preferably 3 to 5, carbon atoms) or haloalkynyl (preferably of 2 to 5, more preferably 3 to 5, carbon atoms). $R^1$ is advantageously benzyl or lower alkyl.

With respect to compounds V, VI, and VII, W, $X^1$, and $X^2$ are as defined above for compound IV and R is alkyl (preferably of 1 to 6 carbon atoms), fluoroalkyl (preferably of 1 to 5 carbon atoms), alkoxyalkyl (preferably of 2 to 6 carbon atoms), alkylthioalkyl (preferably of 2 to 6 carbon atoms), cyanoalkyl (preferably of 1 to 5 alkyl carbon atoms), haloalkoxyalkyl (preferably of 2 to 6 carbon atoms), trifluoromethylthio, alkenyl (preferably of 2 to 5 carbon atoms), 2-oxopropyl, 3-oxopropyl, or haloalkenyl (preferably of 2 to 5 carbon atoms). Preferably R is n-$C_3H_7$ or, particularly, a fluoroalkyl such as 3-fluoropropyl.

Representative compounds of the invention are shown in Table 1. Characterizing data for many of the compounds are given in Table 2.

The preparation and herbicidal activity of representative compounds of this invention are illustrated further in the examples below. All temperatures are in degrees Celsius, and all pressures are in mm Hg.

### Example 1

### 1,4-Dihydro-4-propyl-1-(2,4,5-trichlorophenyl)-5H-tetrazol-5-one

*Step A:* 1,4-Dihydro-1-(2,4,5-trichlorophenyl)-5H-tetrazol-5-one

In a manner similar to that disclosed by Tsuge et al., *J. Org. Chem., 45,* 5230 (1980), the reaction of 6.2 g (0.028 mole) of 2,4,5-trichlorophenyl isocyanate and 6.4 g (0.056 mole) of trimethylsilyl azide produced 1,4-dihydro-1-(2,4,5-trichlorophenyl)-5H-tetrazol-5-one as a solid, 175—176°C.

The nmr and ir spectra were consistent with the proposed structure.

| | | | |
|---|---|---|---|
| Analysis calc'd for $C_7H_3Cl_3N_4O$: | C 31.66; | H 1.14; | N 21.10; |
| Found: | C 34.78; | H 1.62; | N 18.02. |

*Step B:* 1,4-Dihydro-4-propyl-1-(2,4,5-trichlorophenyl)-5H-tetrazol-5-one

To a stirred mixture of 0.24 g (0.005 mole) of sodium hydride (60% oil suspension) in 45 mL of N,N-dimethylformamide was added 1.3 g (0.005 mole) of 1,4-dihydro-1-(2,4,5-trichlorophenyl)-5H-tetrazol-5-one. The mixture was stirred at room temperature for 15 minutes, then 0.85 g (0.005 mole) of 1-iodopropane and several drops of 1,4,7,10,13,16-hexaoxacyclooctadecane (18-crown-6) were added. After complete addition, the reaction mixture was stirred at room temperature for 4 days. The reaction mixture was poured into ice water and the resulting mixture extracted with diethyl ether. The ether extract was

dried over anhydrous magnesium sulfate, filtered, and the filtrate evaporated under reduced pressure to leave a solid. The solid was purified by column chromatography on silica gel, eluting with methylene chloride. Recrystallization from ethanol gave 0.3 g of 1,4-dihydro-4-propyl-1-(2,4,5-trichlorophenyl)-5H-tetrazol-5-one as a solid, mp 89—90°C.

The nmr and ir spectra were consistent with the proposed structure.

The tetrazolinone of Example 1 is listed in Table 1 as compound 1. Compounds 2, 3 and 109—114 were prepared by an analogous method.

Example 2

1-(2,4-Dichloro-5-methylphenyl)-1,4-dihydro-4-propyl-5H-tetrazol-5-one

*Step A:* 3-Acetamidotoluene

Acetic anhydride (176 mL) was added slowly to a stirred mixture of 103.2 mL of m-toluidine in 300 mL of water. After complete addition, the mixture was stirred for 4 hours at room temperature. The mixture was cooled in an ice bath, then extracted with diethyl ether. The ether extract was dried over anhydrous sodium sulfate, filtered, and the filtrate evaporated under reduced pressure to leave an oily residue which solidified. The solid was collected, washed with water, then dissolved in diethyl ether. The etheral solution was dried over anhydrous sodium sulfate, filtered, and the filtrate evaporated under reduced pressure to yield 3-acetamidotoluene as a solid, mp 59—61°C.

*Step B:* 2,4-Dichloro-5-methylacetanilide

Sulfuryl chloride (120 mL) was added dropwise to 75.0 g (0.05 mole) of 3-acetamidotoluene with stirring. After complete addition, the reaction mixture became solid and was allowed to stand at room temperature for approximately 60 hours. The solid mixture was heated until a slurry formed, then stirred for approximately 5 hours. The mixture was cooled to room temperature and treated with ice water and methylene chloride. The resulting two-phase mixture was filtered, and the organic phase was separated, dried over anhydrous sodium sulfate, then filtered. The solvent was evaporated from the filtrate under reduced pressure to leave a solid. The solid was purified by recrystallization from ethyl acetate to yield 10.5 g of 2,4-dichloro-5-methylacetanilide, mp 133—135°C.

*Step C:* 2,4-Dichloro-5-methylaniline

A solution of 3.7 g (0.09 mole) of sodium hydroxide in 96 mL of water was added to a stirred suspension of 9.0 g (0.0041 mole) of 2,4-dichloro-5-methylacetanilide in 96 mL of ethanol. After complete addition, the reaction mixture was heated at reflux for 2 hours. The reaction mixture was cooled, diluted with 200 mL of water, and the resultant mixture stirred at room temperature for approximately 60 hours. A precipitate had formed and was collected by filtration and purified by recrystallization from ethyl acetate to yield 4.0 g of 2,4-dichloro-5-methylaniline.

*Step D:* 2,4-Dichloro-5-methylphenyl isocyanate

In a manner similar to Kurita, et al., *J. Org. Chem.*, *41,* 2070 (1976), incorporated herein by reference, the reaction of 3.43 g (0.0156 mole) of 2,4-dichloro-5-methylaniline and 3.5 g (0.018 mole) of trichloromethyl chloroformate in 50 mL of toluene produced 3.4 g of 2,4-dichloro-5-methylphenyl isocyanate.

*Step E:* 1-(2,4-Dichloro-5-methylphenyl)-1,4-dihydro-5H-tetrazol-5-one

In the manner of Example 1, Step A, the reaction of 3.4 g (0.016 mole) of 2,4-dichloro-5-methylphenyl isocyanate and 3.6 g (0.031 mole) of trimethylsilyl azide produced 1.07 g of 1-(2,4-dichloro-5-methylphenyl)-1,4-dihydro-5H-tetrazol-5-one as a solid, mp 135—137°C.

The nmr and ir spectra were consistent with the proposed structure.

*Step F:* 1-(2,4-Dichloro-5-methylphenyl)-1,4-dihydro-4-propyl-5H-tetrazol-5-one

Under a dry nitrogen atmosphere, 0.06 g (0.0012 mole) of sodium hydride (50% oil suspension) was washed with petroleum ether to remove the oil, then suspended in 5 mL of anhydrous N,N-dimethylformamide. To the suspension was added 0.3 g (0.0012 mole) of 1-(2,4-dichloro-5-methylphenyl)-1,4-dihydro-4H-tetrazol-5-one. After complete addition, the mixture was stirred at room temperature for 30 minutes then heated at 60°C for 15 minutes. The mixture was cooled to room temperature and 0.4 g (0.0024 mole) of 1-iodopropane added. The resultant mixture was stirred at room temperature for approximately 60 hours then poured into ice water. The mixture was extracted with ether acetate and the organic phase washed with a saturated aqueous solution of sodium chloride. The organic phase was dried over anhydrous sodium sulfate, filtered, and the filtrate evaporated to leave an oil. The oil was purified by column chromatography on silica gel, eluting with ethyl acetate:n-hexane (15:85), to yield 0.12 g of 1-(2,4-dichloro-5-methylphenyl)-1,4-dihydro-4-propyl-5H-tetrazol-5-one as a solid, mp 47—48°C.

The nmr and ir spectra were consistent with the proposed structure.

The tetrazolinone prepared in Example 2 is listed as compound 4 in Table 1. Compounds 5 and 90 were prepared by a similar method starting from the appropriately substituted aniline.

Example 3

1-(2,4-Dichloro-5-methoxyphenyl)-1,4-dihydro-4-methyl-5H-tetrazol-5-one

*Step A:* 3-Hydroxyacetanilide

In the manner of Example 2, Step A, the reaction of 66.0 g (0.60 mole) of 3-aminophenol and 77.8 g (0.76 mole) of acetic anhydride in 180 mL of water gave 81.0 g of 3-hydroxyacetanilide as a solid, mp 144—146°C. The reaction was repeated to obtain an additional quantity of product.

*Step B:* 2,4-Dichloro-5-hydroxyacetanilide

In the manner of Example 2, Step B, the chlorination of 100.0 g (0.66 mole) of 3-hydroxyacetanilide with 179.8 g (1.33 mole) of sulfuryl chloride in 1570 mL of glacial acetic acid produced 63.5 g of 2,4-dichloro-5-hydroxyacetanilide as a solid, mp 224—226°C.

*Step C:* 2,4-Dichloro-5-methoxyacetanilide

A stirred mixture of 35.0 g (0.16 mole) of 2,4-dichloro-5-hydroxyacetanilide, 33.1 g (0.24 mole) of potassium carbonate, and 34.1 g (0.24 mole) of methyl iodide in 300 mL of acetone was heated at reflux for approximately 18 hours. The mixture was cooled and filtered. The filtrate was evaporated under reduced pressure to leave 37.4 g of 2,4-dichloro-5-methoxyacetanilide as a solid.

*Step D:* 2,4-Dichloro-5-methoxyaniline

In the manner of Example 2, Step C, the reaction of 37.4 g (0.16 mole) of 2,4-dichloro-5-methoxyacetanilide with 12.8 g (0.32 mole) of sodium hydroxide in 30 mL of water and 30 mL of ethanol produced 16.7 g of 2,4-dichloro-5-methoxyaniline.

1-(2,4-Dichloro-5-methoxyphenyl)-1,4-dihydro-4-methyl-5H-tetrazol-5-one (compound 6) and compounds 7, 9 and 14—20 of Table 1 were prepared from 2,4-dichloro-5-methoxyaniline using the method described in Steps D, E and F of Example 2. Compounds 21—27, 30, 37, 73, 77, 100 and 101 were also prepared by the method of Example 2, Steps D, E, and F from the corresponding 2,4-dichloro-5-alkoxyaniline compounds which were prepared in the manner of Example 3.

## Example 4

### 1-(2,4-Difluoro-5-methoxyphenyl)-1,4-dihydro-4-ethyl-5H-tetrazol-5-one

*Step A:* 2,4-Difluoro-5-methoxyaniline

Hydrogenation of 3.0 g (0.017 mole) of 2,4-difluoro-5-methoxy-1-nitrobenzene in the presence of 0.1 g of platinum oxide and 0.3 mL of morpholine in 110 mL of absolute ethanol produced 1.5 g of 2,4-difluoro-5-methoxyaniline as a solid, mp 43—45°C.

The nmr and ir spectra were consistent with the proposed structure.

1-(2,4-Difluoro-5-methoxyphenyl)-1,4-dihydro-4-ethyl-5H-tetrazol-5-one (compound 8) and compound 10 of Table 1 were prepared from 2,4-difluoro-5-methoxyaniline by the method of Example 2, Steps D, E, and F.

## Example 5

### 1-(4-Chloro-2-fluoro-5-methoxyphenyl)-1,4-dihydro-4-propyl-5H-tetrazol-5-one

*Step A:* 2-Chloro-4-fluorophenyl 4-methylphenylsulfonate

A stirred solution of 20.0 g (0.137 mole) of 2-chloro-4-fluorophenol in 150 mL of pyridine was cooled in an ice bath. To this solution was added portionswise 31.2 g (0.164 mole) of 4-methylphenylsulfonyl chloride. After complete addition, the mixture was stirred for 2 hours, then allowed to stand in a freezer for approximately 16 hours. The mixture was poured into ice water, filtered, and the filter cake washed with water. The solid was dried in a dessicator to yield 41.0 g of 2-chloro-4-fluorophenyl 4-methylphenylsulfonate, mp 92—94°C.

*Step B:* 2-Chloro-4-fluoro-5-nitrophenyl 4-methyl-3-nitrophenylsulfonate

Nitration of 41.0 g (0.16 mole) of 2-chloro-4-fluorophenyl 4-methylphenylsulfonate in 340 mL of fuming nitric acid produced a solid which was recrystallized from ethanol to yield 40.0 g of 2-chloro-4-fluoro-5-nitrophenyl 4-methyl-3-nitrophenylsulfonate.

*Step C:* 2-Chloro-4-fluoro-5-nitrophenol

A solution of 12.84 g (0.195 mole) of potassium hydroxide in 200 mL of water was added to a vigorously stirred solution of 34.0 g (0.097 mole) of 2-chloro-4-fluoro-5-nitrophenyl 4-methyl-3-nitrophenylsulfonate in 190 mL of p-dioxane. The resultant mixture was stirred at room temperature for approximately 18 hours. The reaction mixture was filtered and the filtrate acidified with concentrated hydrochloric acid. The acidic solution was extracted with diethyl ether. The ether extract was dried ove anhydrous sodium sulfate, filtered, and the solvent evaporated under reduced pressure to leave a solid. The solid was washed with petroleum ether to yield 15.07 g of 2-chloro-4-fluoro-5-nitrophenol.

*Step D:* 1-Chloro-5-fluoro-2-methoxy-4-nitrobenzene

To a stirred solution of 8.0 g (0.042 mole) of 2-chloro-4-fluoro-5-nitrophenol in 200 mL of acetone was added 6.09 g (0.0435 mole) of potassium carbonate. The mixture was heated at reflux temperature for 15 minutes, and 8.9 g (0.063 mole) of iodomethane was added. The resultant mixture was heated at reflux temperature for 5 hours, then stirred at room temperature for approximately 18 hours. The mixture was filtered and the filtrate evaporated under reduced pressure to leave a residue. The residue was dissolved in diethyl ether, filtered, and the filtrate washed with a saturated sodium chloride solution. The ether solution was dried over anhydrous sodium sulfate, filtered, and the filtrate evaporated to yield 6.6 g of 1-chloro-5-fluoro-2-methoxy-4-nitrobenzene as a solid, 63—65°C.

The nmr spectrum was consistent with the proposed structure.

*Step E:* 4-Chloro-2-fluoro-5-methoxyaniline

Hydrogenation of 6.6 g (0.032 mole) of 1-chloro-5-fluoro-2-methoxy-4-nitrobenzene in the presence of 0.2 g of platinum oxide and 0.7 g (0.008 mole) of pyridine in 200 mL of absolute ethanol produced 6.5 g of 4-chloro-2-fluoro-5-methoxyaniline.

1-(4-Chloro-2-fluoro-5-methoxyphenyl)-1,4-dihydro-4-propyl-5H-tetrazol-5-one (Compound 11) was prepared by the method described in Example 2, Steps D, E, and F from 4-chloro-2-fluoro-5-methoxyaniline.

Example 6

1-(4-Bromo-2-chloro-5-methoxyphenyl)-1,4-dihydro-4-propyl-5H-tetrazol-5-one

*Step A:* 4-Bromo-2-chloro-5-methoxyaniline

A stirred solution of 9.7 g (0.050 mole) of 2-chloro-5-methoxyaniline hydrochloride in 120 mL of acetic acid was cooled to 15°C. To this cold mixture was added 4.0 g (0.025 mole) of bromine. After complete addition, the mixture was allowed to warm to room temperature and was stirred for approximately 18 hours. The mixture was poured into ice water. To the resultant mixture was added a solution of 2.0 g (0.05 mole) of sodium hydroxide in 10 mL of water. This mixture was extracted with 200 mL of diethyl ether. The ether extract was dried over anhydrous magnesium sulfate, filtered, and the filtrate evaporated under reduced pressure to leave an oil. The oil was purified by column chromatography on silica gel, eluting with methylene chloride, to yield 7.3 g of 4-bromo-2-chloro-5-methoxyaniline as a solid, mp 40—41°C.

1-(4-Bromo-2-chloro-5-methoxyphenyl)-1,4-dihydro-4-propyl-5H-tetrazol-5-one (compound 12) was prepared from 4-bromo-2-chloro-5-methoxyaniline by the method of Example 2, Steps D, E, and F.

Example 7

1-(2-Bromo-4-chloro-5-methoxyphenyl)-1,4-dihydro-4-propyl-5H-tetrazol-5-one

*Step A:* 5-Methoxyacetanilide

In the manner of Example 2, Step A, the reaction of 103.1 g (0.837 mole) of 3-methoxyaniline and 170.5 g (1.67 moles) of acetic anhydride in 300 mL of water produced 138.0 g of 5-methoxyacetanilide as a solid, mp 60—62°C.

*Step B:* 4-Chloro-5-methoxyacetanilide

In the manner of Example 2, Step B, the reaction of 50.0 g (0.303 mole) of 5-methoxyacetanilide with 40.9 g g (0.303 mole) of sulfuryl chloride in 300 mL of chloroform produced a solid. The solid was triturated in ethyl acetate:n-hexane and collected on a filter paper to give 17.0 g of a solid. Recrystallization from ethyl acetate:n-hexane provided 4.3 g of 4-chloro-5-methoxyacetanilide, mp 83—85°C.

The nmr spectrum was consistent with the proposed structure.

*Step C:* 2-Bromo-4-chloro-5-methoxyacetanilide

Bromination of 4.0 g (0.03 mole) of 4-chloro-5-methoxyacetanilide with 3.2 g (0.02 mole) of bromine in 50 mL of glacial acetic acid produced 3.9 g of 2-bromo-4-chloro-5-methoxyacetanilide as a solid, mp 143—145°C.

The nmr spectrum was consistent with the proposed structure.

*Step D:* 2-Bromo-4-chloro-5-methoxyaniline

In the manner of Example 2, Step C, the reaction of 3.9 g (0.013 mole) of 2-bromo-4-chloro-5-methoxyacetanilide with 1.13 g (0.028 mole) of sodium hydroxide in 30 mL of water and 25 mL of ethanol produced 2.9 g of 2-bromo-4-chloro-5-methoxyaniline.

The nmr and ir spectra were consistent with the proposed structure.

1-(2-Bromo-4-chloro-5-methoxyphenyl)-1,4-dihydro-4-propyl-5H-tetrazol-5-one (compound 13) was prepared from 2-bromo-4-chloro-5-methoxyaniline using the method of Example 2, Steps D, E, and F.

Example 8

1-[2,4-Dibromo-5-(1-methylethoxy)phenyl]-1,4-dihydro-4-(2-propenyl)-5H-tetrazol-5-one

*Step A:* 2,4-Dibromo-5-hydroxyacetanilide

Using the method of Example 6, the bromination of 30.2 g (0.20 mole) of 3-hydroxyacetanilide (Example 3, Step A) with 32 g (0.20 mole) of bromine in 100 mL of glacial acetic acid produced a solid. The solid was treated with an additional 32.0 g (0.20 mole) of bromine in 400 mL of glacial acetic acid to yield 12.5 g of 2,4-dibromo-5-hydroxyacetanilide as a solid, mp 233—240°C.

*Step B:* 2,4-Dibromo-5-(1-methylethoxy)acetanilide

To a stirred mixture of 12.0 g (0.038 mole) of 2,4-dibromo-5-hydroxyacetanilide in 80 mL of acetone was added 7.0 g (0.050 mole) of 2-iodopropane. The mixture was heated at reflux temperature for approximately 18 hours, cooled, then filtered. The filtrate was evaporated to leave a solid which was recrystallized from ethanol to produce 11.7 g of 2,4-dibromo-5-(1-methylethoxy)acetanilide, mp 129—130°C.

*Step C:* 2,4-Dibromo-5-(1-methylethoxy)aniline

A stirred mixture of 11.7 g (0.038 mole) of 2,4-dibromo-5-(1-methylethoxy)acetanilide in 100 mL of dilute hydrochloric acid was heated at reflux temperature for 30 minutes. The reaction mixture was cooled, filtered, and the filter cake suspended in 100 mL of water. To the aqueous suspension was added 4.0 g (0.038 mole) of sodium carbonate, and the resulting mixture was stirred for approximately 1 hour. The mixture was filtered and the filter cake dried under reduced pressure to yield 6.2 g of 2,4-dibromo-5-(1-methylethoxy)aniline.

1-[2,4-Dibromo-5-(1-methylethoxy)phenyl]-1,4-dihydro-4-(2-propenyl)-5H-tetrazol-5-one (compound 28) and compound 29 of Table 1 were prepared from 2,4-dibromo-5-(1-methylethoxy)aniline by the method of Example 2, Steps D, E, and F.

Example 9

1-(4-Chloro-5-methoxy-2-methylphenyl-1,4-dihydro-4-propyl-5H-tetrazol-5-one

*Step A:* 1-Methoxy-4-methyl-3-nitrobenzene

In the manner of Example 8, Step C, the reaction of 25.0 g (0.162 mole) of 4-methyl-3-nitrophenol, 23.3 g (0.168 mole) of potassium carbonate, and 34.1 g (0.24 mole) of methyl iodide produced 26.4 g of 1-methoxy-4-methyl-3-nitrobenzene as an oil.

The nmr spectrum was consistent with the proposed structure.

*Step B:* 5-Methoxy-2-methylaniline

Hydrogenation of 26.4 g (0.157 mole) of 1-methoxy-4-methyl-3-nitrobenzene in the presence of 0.5 g of platinum oxide in 200 mL of absolute ethanol produced 20.2 g of 5-methoxy-2-methylaniline as a solid, mp 36—38°C.

The nmr spectrum was consistent with the proposed structure.

*Step C:* 5-Methoxy-2-methylacetanilide

In the manner of Example 2, Step A, the reaction of 19.4 g (0.14 mole) of 5-methoxy-2-methylaniline with 28.2 g (0.28 mole) of acetic anhydride in 100 mL of water produced 17.1 g of 5-methoxy-2-methylacetanilide as a solid, 68—70°C.

The nmr spectrum was consistent with the proposed structure.

*Step D:* 4-Chloro-5-methoxy-2-methylacetanilide

In the manner of Example 2, Step B, the chlorination of 16.0 g (0.089 mole) of 5-methoxy-2-methylacetanilide with 12.0 g (0.089 mole) of sulfuryl chloride in 150 mL of chloroform produced 13.3 g of 4-chloro-5-methoxyacetanilide as a solid, mp 170—171°C.

The nmr spectrum was consistent with the proposed structure.

*Step E:* 2-Chloro-5-methoxy-2-methylaniline

In the manner of Example 2, Step C, the reaction of 13.3 g (0.062 mole) of 4-chloro-5-methoxyacetanilide with 5.0 g (0.125 mole) of sodium hydroxide in 125 mL of water and 125 mL of ethanol produced 4.5 g of 2-chloro-5-methoxy-2-methylaniline as a solid, mp 83—85°C.

1-(4-Chloro-5-methoxy-2-methylphenyl)-1,4-dihydro-4-propyl-5H-tetrazol-5-one (compound 95) and compound 96 of Table 1 were produced from 2-chloro-5-methoxy-2-methylaniline by the method of Example 2, Steps D, E, and F.

Example 10

1-[2,4-Dichloro-5-(3-iodo-2-propynyloxy)phenyl]-1,4-dihydro-4-propyl-5H-tetrazol-5-one

*Step A:* 1-(2,4-Dichloro-5-hydroxyphenyl)-1,4-dihydro-4-propyl-5H-tetrazol-5-one

A stirred mixture of 2.0 g (0.0066 mole) of 1-(2,4-dichloro-5-methoxyphenyl)-1,4-dihydro-4-propyl-5H-tetrazol-5-one (compound 9) and 30 mL of concentrated hydrobromic acid was heated at reflux temperature for approximately 18 hours. The reaction mixture was cooled, filtered, and the filter cake dissolved in methylene chloride. The methylene chloride solution was evaporated under reduced pressure to leave 1.35 g of 1-(2,4-dichloro-5-hydroxyphenyl)-1,4-dihydro-4-propyl-5H-tetrazol-5-one as a solid, mp 125—128°C.

The nmr spectrum was consistent with the proposed structure.

*Step B:* 1-[2,4-Dichloro-5-(2-propynyloxy)phenyl]-1,4-dihydro-4-propyl-5H-tetrazol-5-one

To a stirred solution of 0.5 g (0.0017 mole) of 1-(2,4-dichloro-5-hydroxyphenyl)-1,4-dihydro-4-propyl-5H-tetrazol-5-one in 15 mL of anhydrous N,N-dimethylformamide was added 0.11 g (0.0019 mole) of potassium fluoride, several drops of 18-crown-6, and 0.14 g (0.0019 mole) of propargyl chloride. The mixture was stirred at 80°C for approximately 20 hours, then cooled to room temperature. A portion of the solvent was removed by distillation under reduced pressure, and the remainder of the mixture was poured into water. The aqueous mixture was extracted with ethyl acetate, and the organic layer washed with a saturated aqueous solution of sodium chloride. The organic phase was dried over anhydrous sodium sulfate, filtered, and the filtrate evaporated under reduced pressure to leave a residue. The residue was

purified by column chromatography on silica gel, eluting with ethyl acetate:n-hexane (15:85), to yield 0.27 g of 1-[2,4-dichloro-5-(2-propynyloxy)phenyl]-1,4-dihydro-4-propyl-5H-tetrazol-5-one as a solid, mp 70—72°C.

The nmr spectrum was consistent with the proposed structure.

This experiment was repeated on a larger scale to obtain additional quantities of 1-[2,4-dichloro-5-(2-propynyloxy)phenyl]-1,4-dihydro-4-propyl-5H-tetrazol-5-one.

The tetrazolinone prepared in Example 10, Steps A and B is listed in Table 1 as compound 45. Other tetrazolinones prepared in a similar manner are compounds 31, 32, 34, 38—42, 45, 46, 49, 50, 57, 58, 63—65, 80 and 91—93. In some instances 18-crown-6 was not used. Also, in some instances, acetone was used as the solvent in place of N,N-dimethylformamide.

*Step C:* 1-[2,4-Dichloro-5-(3-iodo-2-propynyloxy)phenyl]-1,4-dihydro-4-propyl-5H-tetrazol-5-one

To a cold solution of 0.5 g (0.00153 mole) of 1-[2,4-dichloro-5-(2-propynyloxy)phenyl]-1,4-dihydro-4-propyl-5H-tetrazol-5-one in 20 mL of methanol was added 2 mL of a saturated aqueous solution of sodium hydroxide and an additional 10 mL of methanol. To this mixture was added a solution of 0.39 g (0.00153 mole) of iodine in 5 mL of methanol. After complete addition, the reaction mixture was allowed to warm to room temperature and was stirred for approximately 18 hours. The reaction mixture was filtered and a solid collected. The solid was purified by column chromatography to yield 0.14 g of 1-[2,4-dichloro-5-(3-iodo-2-propynyloxy)phenyl]-1,4-dihydro-4-propyl-5H-tetrazol-5-one as a solid, mp 134—138°C.

The nmr spectrum was consistent with the proposed structure.

The tetrazolinone prepared in Step C is listed in Table 1 as compound 61.

## Example 11

1-[2,4-Difluoro-5-(1-methylethoxy)phenyl]-1,4-dihydro-4-propyl-5H-tetrazol-5-one

*Step A:* 1-(2,4-Difluoro-5-hydroxyphenyl)-1,4-dihydro-4-propyl-5H-tetrazol-5-one

Under a dry nitrogen atmosphere a stirred solution of 0.18 g (0.00067 mole) of 1-(2,4-difluoro-5-methoxyphenyl)-1,4-dihydro-4-propyl-5H-tetrazol-5-one (compound 10) in 5 mL of methylene chloride was cooled to −40°C. To this cold solution was added dropwise a solution of 0.5 g (0.002 mole) of boron tribromide in 5 mL of methylene chloride. After complete addition, the mixture was allowed to warm to room temperature and was stirred for approximately 70 hours. The mixture was poured into ice water and the two phases separated. The aqueous phase was extracted with methylene chloride. The organic phases were combined, dried over anhydrous sodium sulfate, and filtered. The filtrate was evaporated to leave 0.14 g of 1-(2,4-difluoro-5-hydroxyphenyl)-1,4-dihydro-4-propyl-5H-tetrazol-5-one as a solid, mp 65—67°C.

The nmr spectrum was consistent with the proposed structure.

The reaction was repeated to obtain additional quantities of product.

*Step B:* 1-(2,4-Difluoro-5-(1-methylethoxy)phenyl]-1,4-dihydro-4-propyl-5H-tetrazol-5-one

A stirred mixture of 0.87 g (0.0034 mole) of 1-(2,4-difluoro-5-hydroxyphenyl)-1,4-dihydro-4-propyl-5H-tetrazol-5-one, 0.7 g (0.0051 mole) of potassium carbonate, and 1.2 g (0.007 mole) of 2-iodopropane in 40 mL of acetone was heated at reflux for approximately 18 hours. The mixture was cooled, filtered, and the solvent evaporated under reduced pressure to leave an oil. The oil was purified by filtration through a small amount of silica gel followed by crystallization from heptane to yield 0.38 g of 1-[2,4-difluoro-5-(1-methylethoxy)phenyl]-1,4-dihydro-4-propyl-5H-tetrazol-5-one, mp 76.5—77°C.

The tetrazolinone prepared in Example 11 is listed in Table 1 as compound 35. Other tetrazolinones prepared by this method were compounds 36, 43, 48, 51—53, 55, 56, 59, 60, 67, 74, 75, 78, 83—85 and 97—99. In some cases the intermediate 5-hydroxyphenyl compound was prepared by the method of Example 10, Step A.

## Example 12

1-[2,4-Dichloro-5-(2-butynyloxy)phenyl]-1,4-dihydro-4-propyl-5H-tetrazol-5-one

To a stirred solution of 0.5 g (0.0017 mole) of 1-(2,4-dichloro-5-hydroxyphenyl)-1,4-dihydro-4-propyl-5H-tetrazol-5-one (Example 10, Step A) and 0.15 g (0.0021 mole) of 2-butyn-1-ol in 5 mL of acetonitrile was added 0.33 g (0.0016 mole) of dicyclohexylcarbodiimide. The reaction mixture was heated at reflux temperature for 4 days, cooled to room temperature, and filtered. The filtrate was washed with 10 mL of a 10% aqueous sodium hydroxide solution, diluted with methylene chloride, and washed with 10 mL of 6N hydrochloric acid. The washed organic phase was dried over anhydrous magnesium sulfate, filtered, and the filtrate evaporated to leave an oil. The oil was purified by column chromatography on silica gel, eluting with ethyl acetate:heptane (20:80), to yield 0.12 g of 1-[2,4-dichloro-5-(2-butynyloxy)phenyl]-1,4-dihydro-4-propyl-5H-tetrazol-5-one as a solid, mp 58—60°C.

The nmr and ir spectra were consistent with the proposed structure.

The tetrazolinone prepared in Example 12 is listed as compound 62 in Table 1.

Example 13

1-(2,4-Dichloro-5-methoxymethoxyphenyl)-1,4-dihydro-4-ethyl-5H-tetrazol-5-one

*Step A:* 1-(2,4-Dichloro-5-hydroxyphenyl)-1,4-dihydro-4-ethyl-5H-tetrazol-5-one

In the manner of Example 11, Step A, the reaction of 1.56 g (0.0054 mole) of 1-(2,4-dichloro-5-methoxyphenyl)-1,4-dihydro-4-ethyl-5H-tetrazol-5-one (compound 7) and 4.06 g (0.016 mole) of boron tribromide in 20 mL of methylene chloride produced 1.25 g of 1-(2,4-dichloro-5-hydroxyphenyl)-1,4-dihydro-4-ethyl-5H-tetrazol-5-one, mp 149—150°C.

*Step B:* 1-(2,4-Dichloro-5-methoxymethoxyphenyl)-1,4-dihydro-4-ethyl-5H-tetrazol-5-one

To a stirred mixture of 0.8 g (0.0029 mole) of 1-(2,4-dichloro-5-hydroxyphenyl)-1,4-dihydro-4-ethyl-5H-tetrazol-5-one, 2.8 g (0.032 mole) of N,N-dimethylacetamide, and 0.14 g (0.0029 mole) of sodium hydride (50% in oil) in 25 mL of toluene was added 0.47 g (0.0058 mole) of chloromethyl methyl ether. The reaction mixture was stirred at room temperature for approximately 18 hours then poured into ice water and extracted with diethyl ether. The extract was dried over anhydrous magnesium sulfate, filtered, and the filtrate evaporated to leave a solid. The solid was purified by recrystallization from heptane to yield 0.19 g of 1-(2,4-dichloro-5-methoxymethoxyphenyl)-1,4-dihydro-4-ethyl-5H-tetrazol-5-one, mp 112—113°C.

The nmr spectrum was consistent with the proposed structure.

The tetrazolinone prepared in Example 13 is listed as compound 66 in Table 1. Other tetrazolinones prepared by this method were compounds 68—72.

Example 14

1-[2,4-Dichloro-5-(2-tetrahydrofuranyloxy)phenyl]-1,4-dihydro-4-propyl-5H-tetrazol-5-one

To a stirred solution of 1.0 g (0.0035 mole) of 1-(2,4-dichloro-5-hydroxyphenyl)-1,4-dihydro-4-propyl-5H-tetrazol-5-one (Example 10, Step A) and 0.05 g (0.00026 mole) of 4-methylphenylsulfonic acid in 40 mL of p-dioxane was added slowly 0.88 g (0.0126 mole) of dihydrofuran in 20 mL of p-dioxane. The mixture was stirred at room temperature for approximately 18 hours, and an additional 0.88 g of dihydrofuran was added. The mixture was stirred at room temperature for an additional 48 hours. One mL of methanol saturated with ammonia was added to the mixture and stirring was continued for a short period of time. The reaction mixture was evaporated under reduced pressure to leave a residue. The residue was purified by column chromatography on silica gel, eluting with methylene chloride, to yield 0.58 g of 1-[2,4-dichloro-5-(2-tetrahydrofuranyloxy)phenyl]-1,4-dihydro-4-propyl-5H-tetrazol-5-one as a solid, mp 89—90°C.

The nmr spectrum was consistent with the proposed structure.

The tetrazolinone of Example 14 is listed as compound 76 in Table 1. Compounds 79, 102, and 103 were also prepared by this process.

Example 15

1-(5-Acetyloxy-2,4-dichlorophenyl)-1,4-dihydro-4-propyl-5H-tetrazol-5-one

To a stirred mixture of 0.1 g (0.0021 mole) of sodium hydride (50% in oil) in 30 mL of tetrahydrofuran was added slowly 0.5 g (0.0017 mole) of 1-(2,4-dichloro-5-hydroxyphenyl)-1,4-dihydro-5H-tetrazol-5-one (Example 10, Step A). The mixture was stirred for 15 minutes and 0.16 g (0.0021 mole) of acetyl chloride was slowly added. The reaction mixture was stirred at room temperature for approximately 18 hours. The mixture was poured into ice water, and the resulting precipitate was collected on a filter paper. Recrystallization from ethyl acetate:heptane gave 0.15 g of 1-(5-acetoxy-2,4-dichlorophenyl)-1,4-dihydro-4-propyl-5H-tetrazol-5-one, mp 91—92°C.

The nmr spectrum was consistent with the proposed structure.

The tetrazolinone prepared in Example 15 is listed in Table 1 as compound 81.

Example 16

2,4-Dichloro-5-(1,4-dihydro-5-oxo-4-propyltetrazol-1-yl)phenoxyacetic acid

A stirred solution of 0.5 g (0.0014 mole) of methyl 2,4-dichloro-5-(1,4-dihydro-5-oxo-4-propyltetrazol-1-yl)phenoxyacetate (compound 83) and 0.08 g (0.0021 mole) of sodium hydroxide in 10 mL of methanol was heated at reflux temperature for 1.5 hours. The reaction mixture was cooled, then evaporated under reduced pressure to leave a solid residue. The residue was dissolved in 20 mL of water, and the solution acidified with concentrated hydrochloric acid. A solid precipitate formed and was collected by filtration and washed with cold water. Recrystallization from diethyl ether:pentane provided 0.2 g of 2,4-dichloro-5-(1,4-dihydro-5-oxo-4-propyltetrazol-1-yl)phenoxyacetic acid, mp 134—136°C.

The nmr spectrum was consistent with the proposed structure.

The tetrazolinone prepared in Example 16 is listed in Table 1 as compound 82.

Example 17

1-(2,4-Dichloro-5-methylsulfonyloxyphenyl)-1,4-dihydro-4-propyl-5H-tetrazol-5-one

A stirred solution of 0.5 g (0.0017 mole) of 1-(2,4-dichloro-5-hydroxyphenyl)-1,4-dihydro-4-propyl-5H-tetrazol-5-one (Example 10, Step A) and 0.26 g (0.0026 mole) of triethylamine in 30 mL of methylene chloride was cooled to 0°C. Methanesulfonyl chloride (0.22 g, 0.0019 mole) was added slowly to the reaction mixture. After complete addition, the mixture was allowed to warm to room temperature and was

stirred for several days. The mixture was poured into ice water and extracted with diethyl ether. The ether extract was dried over anhydrous magnesium sulfate, filtered, and the filtrate evaporated under reduced pressure to leave a residue. The residue was purified by column chromatography on silica gel to yield 0.33 g of 1-(2,4-dichloro-5-methylsulfonyloxyphenyl)-1,4-dihydro-4-propyl-5H-tetrazol-5-one was a solid, mp 64—65°C.

The nmr spectrum was consistent with the proposed structure.

The tetrazolinone prepared in Example 17 is listed in Table 1 as compound 86. Compounds 87—89 were also prepared by this method.

Example 18

1-(2-chloro-5-methoxy-4-trifluoromethylphenyl)-1,4-dihydro-4-propyl-5H-tetrazol-5-one

Under a dry nitrogen atmosphere a stirred mixture of 6.0 g (0.044 mole) of sodium trifluoroacetate and 4.18 g (0.022 mole) of copper (I) iodide in 40 mL of N,N-dimethylacetamide was heated at 150°C. To the hot mixture was added 3.3 g (0.011 mole) of 1-(4-bromo-2-chloro-5-methoxyphenyl)-1,4-dihydro-4-propyl-5H-tetrazol-5-one (compound 12). After complete addition, the hot mixture was stirred for 3 hours then allowed to cool to room temperature. The mixture was filtered and the filtrate diluted with ice water to form a precipitate. The solid was collected by filtration and purified, first by column chromatography on silica gel eluting with methylene chloride:heptane (80:20), then by recrystallization from heptane to yield 0.7 g of 1-(2-chloro-5-methoxy-4-trifluoromethylphenyl)-1,4-dihydro-4-propyl-5H-tetrazol-5-one, mp 82—83°C.

The nmr spectrum was consistent with the proposed structure.

The tetrazolinone prepared in Example 18 is listed in Table 1 as compound 94. Compound 108 was also prepared by this method.

Example 19

1-[2-Chloro-4-fluoro-5-(2-propynyloxy)phenyl]-1,4-dihydro-4-ethyl-5H-tetrazol-5-one

*Step A:* Di-4-chloro-2-fluorophenyl carbonate

A stirred solution of 11.5 g (0.078 mole) of 2-fluoro-4-chlorophenol sodium salt in 80 mL of p-dioxane and 30 mL of water was cooled to 5—10°C. A solution of phosgene in toluene (35 mL of a 12.5% solution, 0.039 mole phosgene) was added slowly to the cold reaction mixture. After complete addition, the mixture was stirred at room temperature for approximately 18 hours. The mixture was poured into ice water and the organic phase separated. The organic phase was dried over anhydrous magnesium sulfate, filtered, and the filtrate evaporated to leave an oil. The oil was purified by crystallization from petroleum ether to yield 6.0 g of di-4-chloro-2-fluorophenyl carbonate, mp 89—90°C.

*Step B:* Di-4-chloro-2-fluoro-5-nitrophenyl carbonate

To 50 mL of fuming nitric acid, cooled to 10°C, was added 6.0 g (0.02 mole) of di-4-chloro-2-fluorophenyl carbonate. After complete addition, the mixture was stirred for 30 minutes at 10°C then poured into ice water. A solid formed and was collected by filtration and rinsed with water to yield 7.5 g of di-4-chloro-2-fluoro-5-nitrophenyl carbonate.

*Step C:* 4-Chloro-2-fluoro-5-n-nitrophenol

A solution of 1.2 g (0.03 mole) of sodium hydroxide in 30 mL of water was added to a stirred solution of 7.5 g (0.019 mole) of di-4-chloro-2-fluoro-5-nitrophenyl carbonate in 60 mL of p-dioxane. After complete addition, the mixture was stirred for 1 hour. The mixture was diluted with 100 mL of water and the resulting solution neutralized with concentrated hydrochloric acid. A solid formed and was collected by filtration and washed with petroleum ether to yield 6.0 g of 4-chloro-2-fluoro-5-nitrophenol, mp 85—86°C.

The nmr spectrum was consistent with the proposed structure.

*Step D:* 1-Chloro-3-fluoro-4-methoxy-6-nitrobenzene

In the manner of Example 3, Step C, the reaction of 6.0 g (0.031 mole) of 4-chloro-2-fluoro-5-nitrophenol, 6.35 g (0.046 mole) of potassium carbonate, and 8.8 g (0.062 mole) of methyl iodide in 80 mL of acetone produced 6.1 g of 1-chloro-3-fluoro-4-methoxy-6-nitrobenzene as a solid, mp 80—81°C.

*Step E:* 2-Chloro-4-fluoro-5-methoxyaniline

The hydrogenation of 4.5 g (0.022 mole) of 1-chloro-3-fluoro-4-methoxy-6-nitrobenzene in the presence of 0.15 g of platinum oxide in 200 mL of absolute ethanol produced 3.2 g of 2-chloro-4-fluoro-5-methoxyaniline.

*Step F:* 2-Chloro-4-fluoro-5-methoxyphenyl isocyanate

In the manner of Example 2, Step D, the reaction of 3.0 g (0.017 mole) of 2-chloro-4-fluoro-5-methoxyaniline with 1.68 g (0.0085 mole) of trichloromethyl chloroformate in 20 mL of toluene produced 3.0 g of 2-chloro-4-fluoro-5-methoxyphenyl isocyanate.

*Step G:* 1-(2-Chloro-4-fluoro-5-methoxyphenyl)-1,4-dihydro-5H-tetrazol-5-one

In the manner of Example 2, Step E, the reaction of 3.2 g (0.016 mole) of 2-chloro-4-fluoro-5-methoxyphenyl isocyanate with 3.2 g (0.028 mole) of trimethylsilyl azide produced 3.0 g of 1-(2-chloro-4-fluoro-5-methoxyphenyl)-1,4-dihydro-5H-tetrazol-5-one.

*Step H:* 1-(2-Chloro-4-fluoro-5-methoxyphenyl)-1,4-dihydro-4-ethyl-1,4-dihydro-5H-tetrazol-5-one

In the manner of Example 2, Step F, the reaction of 1.33 g (0.0054 mole) of 1-(2-chloro-4-fluoro-5-methoxyphenyl)-1,4-dihydro-5H-tetrazol-5-one with 0.26 g (0.0054 mole) of sodium hydride and 1.6 g (0.01 mole) of iodoethane in 60 mL of dimethylsulfoxide produced 1.26 g of 1-(2-chloro-4-fluoro-5-methoxyphenyl)-1,4-dihydro-4-ethyl-5H-tetrazol-5-one, compound 105, 79—80°C. Compound 106 was prepared in a similar manner.

*Step I:* 1-(2-Chloro-4-fluoro-5-hydroxyphenyl)-1,4-dihydro-4-ethyl-5H-tetrazol-5-one

In the manner of Example 11, Step A, the reaction of 0.9 g (0.0033 mole) of 1-(2-chloro-4-fluoro-5-methoxyphenyl)-1,4-dihydro-4-ethyl-5H-tetrazol-5-one with 2.5 g (0.01 mole) of boron tribromide in 15 mL of methylene chloride produced 0.8 g of 1-(2-chloro-4-fluoro-5-hydroxyphenyl)-1,4-dihydro-4-ethyl-5H-tetrazol-5-one.

*Step J:* 1-[2-Chloro-4-fluoro-5-(2-propynyloxy)phenyl]-1,4-dihydro-4-ethyl-5H-tetrazol-5-one

In the manner of Example 8, Step B, the reaction of 0.8 g (0.0031 mole) of 1-(2-chloro-4-fluoro-5-hydroxyphenyl)-1,4-dihydro-4-ethyl-5H-tetrazol-5-one with 0.9 g (0.0062 mole) of 1-bromo-2-propyne and 0.63 g (0.0046 mole) of potassium carbonate in 25 mL of acetone produced 0.6 g of 1-[2-chloro-4-fluoro-5-(2-propynyloxy)phenyl]-1,4-dihydro-4-ethyl-5H-tetrazol-5-one, mp 88—89°C.

The nmr spectrum was consistent with the proposed structure.

The tetrazolinone prepared in Example 19 is listed in Table 1 as compound 44. Compound 47 was also prepared by the process described in this Example.

Example 20

1-(2,4-Dichloro-5-methoxyphenyl)4-ethenyl-1,4-dihydro-5H-tetrazol-5-one

*Step A:* 1-(2,4-Dichloro-5-methoxyphenyl)-1,4-dihydro-4-(2-hydroxyethyl)-5H-tetrazol-5-one

In the manner of Example 10, Step B, 3.3 g (0.058 mole) of potassium fluoride was reacted with 3.0 g (0.0115 mole) of 1-(2,4-dichloro-5-methoxyphenyl)-1,4-dihydro-5H-tetrazol-5-one (prepared from 2,4-dichloro-5-methoxyaniline, Example 3, using the process of Example 2, Steps D and E) in 45 mL of N,N-dimethylformamide. Subsequent treatment with 5.9 g (0.035 mole) of 2-iodoethanol produced 1.1 g of 1-(2,4-dichloro-5-methoxyphenyl)-2,5-dihydro-4-(2-hydroxyethyl)-5H-tetrazol-5-one as a solid, mp 109—110°C.

*Step B:* 1-(2,4-Dichloro-5-methoxyphenyl)-1,4-dihydro-4-[2-(methylsulfonyloxy)ethyl]-5H-tetrazol-5-one

A stirred solution of 0.9 g (0.003 mole) of 1-(2,4-dichloro-5-methoxyphenyl)-1,4-dihydro-4-(2-hydroxyethyl)-5H-tetrazol-5-one and 0.45 g (0.0044 mole) of triethylamine in 35 mL of methylene chloride was cooled to 0°C. To the cool solution was added 0.37 g (0.0033 mole) of methanesulfonyl chloride. After complete addition, the mixture was allowed to warm to room temperature and was stirred for approximately 18 hours. The mixture was poured into ice water. Sodium chloride was added and the mixture was extracted with diethyl ether. The ether extract was dried over anhydrous magnesium sulfate, filtered, and the filtrate evaporated under reduced pressure to yield 1.0 g of 1-(2,4-dichloro-5-methoxyphenyl)-1,4-dihydro-4-[2-(methylsulfonyloxy)ethyl]-5H-tetrazol-5-one as an oil.

*Step C:* 1-(2,4-Dichloro-5-methoxyphenyl)-4-ethenyl-1,4-dihydro-5H-tetrazol-5-one

A stirred mixture of 0.38 g (0.0066 mole) of potassium fluoride and 0.85 g (0.0022 mole) of 1-(2,4-dichloro-5-methoxyphenyl)-1,4-dihydro-4-[2-(methylsulfonyloxy)ethyl]-5H-tetrazol-5-one in 35 mL of N,N-dimethylformamide was heated at 120—130°C for 2 hours, then stirred at room temperature for 3 days. The reaction mixture was poured into ice water then extracted with diethyl ether. The ether extract was dried over anhydrous magnesium sulfate, filtered, and the filtrate evaporated under reduced pressure to leave a residue. The residue was purified by column chromatography on silica gel, eluting with methylene chloride, then recrystallized from ethyl acetate:heptane to yield 0.25 g of 1-(2,4-dichloro-5-methoxyphenyl)-4-ethenyl-1,4-dihydro-5H-tetrazol-5-one, mp 142—143°C.

The nmr and ir spectra were consistent with the proposed structure.

The tetrazolinone prepared in Example 20 is listed as compound 104 in Table 1.

Example 21

1-(4-Bromomethyl-2-chloro-5-isopropoxyphenyl)-1,4-dihydro-4-propyl-5H-tetrazol-5-one

Under a dry nitrogen atmosphere, a stirred solution of 4.4 g (0.014 mole) of 1-(2-chloro-4-methyl-5-isopropoxyphenyl)-1,4-dihydro-4-propyl-5H-tetrazol-5-one (compound 91), 2.5 g (0.014 mole) of N-bromosuccinimide, and 0.06 g (0.00025 mole) of benzoyl peroxide in 80 mL of carbon tetrachloride was heated at reflux temperature for two days. The reaction mixture was allowed to cool to room temperature, filtered, and the filtrate washed with a saturated aqueous sodium chloride solution. The washed filtrate was dried over anhydrous magnesium sulfate, filtered, and the filtrate evaporated under reduced pressure to leave an oil. The oil was crystallized from petroleum ether to yield 2.7 g of 1-(4-bromomethyl-2-chloro-5-isopropoxyphenyl)-1,4-dihydro-4-propyl-5H-tetrazol-5-one, mp 68—70°C.

The nmr spectrum was consistent with the proposed structure.

The tetrazolinone of Example 21 is listed as compound 107 in Table 1.

Herbicidal Activity

The plant test species used in demonstrating the herbicidal activity of compounds of this invention include cotton (*Gossypium hirsutum* var. Stoneville), soybean (*Glycine max* var. Williams), field corn (*Zea mays* var. Agway 595S), rice (*Oryza sativa* var. Labelle), wheat (*Triticum aestivium* var. Prodax), field bindweed (*Convolvulus arvensis*), morningglory (*Ipomea lacunosa* or *Ipomea hederacea*), velvetleaf (*Abutilon theophrasti*), barnyardgrass (*Echinochloa crus galli*), green foxtail (*Setaria viridis*), johnsongrass (*Sorghum halepense*), and yellow nutsedge (*Cyperus esculentus*).

Seeds or tubers of the plant test species were planted in furrows in steam sterilized sand loam soil contained in disposable fiber flats. A topping soil of equal portions of sand and sandy loam soil was placed uniformly on top of each flat to a depth of approximately 0.5 cm.

The flats for the preemergence test were watered, then drenched with the appropriate amount of a solution of the test compound in a mixture of acetone and water containing a small amount (up to 0.5% v/v) of sorbitan monolaurate emulsifier/solubilizer. The concentration of the test compound in solution was varied to give a range of application rates, generally 8.0 kg/ha and submultiples thereof. The flats were placed in a greenhouse and watered regularly at the soil surface for 21 days at which time phytotoxicity data were recorded.

The flats for the postemergence test were placed in a greenhouse and watered for 8—10 days, then the foliage of the emerged test plants was sprayed with a solution of the test compound in acetone-water containing up to 0.5% sorbitan monolaurate. After spraying, the foliage was kept dry for 24 hours, then watered regularly for 21 days, and phytotoxicity data recorded.

Phytotoxicity data were taken either as percent kill or percent control. Percent control was determined by a method similar to the 0 to 100 rating system disclosed in "Research Methods in Weed Science," 2nd ed., B. Truelove, Ed.; Southern Weed Science Society; Auburn University, Auburn, Alabama, 1977. The present rating system is as follows:

| Rating Percent Control | Description of Main Categories | Crop Description | Weed Description |
|---|---|---|---|
| 0 | No effect | No crop reduction or injury | No weed control |
| 10 | | Slight discoloration or stunting | Very poor weed control |
| 20 | Slight effect | Some discoloration, stunting or stand loss | Poor weed control |
| 30 | | Crop injury more pronounced but not lasting | Poor to deficient weed control |
| 40 | | Moderate injury, crop usually recovers | Deficient weed control |
| 50 | Moderate effect | Crop injury more lasting, recovery | Deficient to moderate weed control |
| 60 | | Lasting crop injury no recovery | Moderate weed control |

| Rating Percent Control | Description of Main Categories | Crop Description | Weed Description |
|---|---|---|---|
| 70 | | Heavy injury and stand loss | Control somewhat less than satisfactory |
| 80 | Severe | Crop nearly destroyed a few survivors | Satisfactory to good weed control |
| 90 | | Only occasional live plants left | Very good to excellent control |
| 100 | Complete effect | Complete crop destruction | Complete weed destruction |

Herbicidal data at selected application rates are given for various compounds of the invention in Table 3 and 4 below. The test compounds are identified in the tables below by numbers which correspond to those in Table 1 above.

In the tables of herbicidal data below:

"kg/ha" is kilograms per hectare,

"% K" is percent kill, and

"% C" is percent control.

It is clear that the generic class of aryltetrazolinones and thiones described and illustrated herein is characterized by herbicidal activity, and that the degree of this activity varies among specific compounds within this class and to some extent among the species of plant to which these compounds may be applied. Thus, selection of a specific herbicidal compound for control of a specific plant may readily be made.

For herbicidal application, the active compounds as above defined are formulated into herbicidal compositions by admixture in herbicidally effective amounts with adjuvants and carriers normally employed in the art for facilitating the dispersion of active ingredients for the particular utility desired, recognizing the fact that the formulation and mode of application of a toxicant may affect the activity of the material in a given application. Thus, for agricultural use the present herbicidal compounds may be formulated as granules of relatively large particle size, water-soluble or water-dispersible granules, as powdery dusts, as wettable powders, as emulsifiable concentrates, as solutions or as any of several other known types of formulations, depending on the desired mode of application.

For preemergence application these herbicidal compositions are usually applied either as sprays, dusts, or granules to the areas in which suppression of vegetation is desired. For postemergence control of established plant growth, sprays or dusts are most commonly used. These formulations may contain as little as 0.5% to as much as 95% or more by weight of active ingredient.

Dusts are free flowing admixtures of the active ingredient with finely divided solids such as talc, natural clays, kieselguhr, flours such as walnut shell and cottonseed flours, and other organic and inorganic solids which act as dispersants and carriers for the toxicant; these finely divided solids have an average particle size of less than about 50 microns. A typical dust formulation useful herein is one containing 1.0 part of the herbicidal compound and 99.0 parts of talc.

Wettable powders, also useful formulations for both pre- and postemergence herbicides, are in the form of finely divided particles which disperse readily in water or other dispersant. The wettable powder is ultimately applied to the soil either as a dry dust or as an emulsion in water or other liquid. Typical carriers for wettable powders inclue Fuller's earth, kaolin clays, silicas, and other highly absorbent, readily wet inorganic diluents. Wettable powders normally are prepared to contain about 5—80% of active ingredient, depending on the absorbency of the carrier, and usually also contain a small amount of a wetting, dispersing or emulsifying agent to facilitate dispersion. For example, a useful wettable powder formulation contains 80.8 parts of the herbicidal compound, 17.9 parts of Palmetto clay, and 1.0 part of sodium lignosulfonate and 0.3 part of sulfonated aliphatic polyester as wetting agents. Frequently, additional wetting agent and/or oil will be added to the tank-mix for post-emergence application to facilitate dispersion on the foliage and absorption by the plant.

Other useful formulations for herbicidal applications are emulsifiable concentrates. Emulsifiable concentrates are homogeneous liquid or past compositions dispersible in water or other dispersant, and may consist entirely of the herbicidal compound and a liquid or solid emulsifying agent, or may also contain a

liquid carrier, such as xylene, heavy aromatic naphthas, isophorone, or other non-volatile organic solvent. For herbicidal application these concentrates are dispersed in water or other liquid carrier, and normally applied as a spray to the area to be treated. The percentage by weight of the essential active ingredient may vary according to the manner in which the composition is to be applied, but in general comprises 0.5 to 95% of active ingredient by weight of the herbicidal composition.

Typical wetting, dispersing or emulsifying agents used in agricultural formulations include, for example, the alkyl or alkylaryl sulfonates and sulfates and their sodium salts; polyhydric alcohols; and other types of surface active agents, many of which are available in commerce. The surface active agent, when used, normally comprises 1% to 15% by weight of the herbicidal composition.

Other useful formulations for herbicidal applications include simple solutions of the active ingredient in a dispersant in which it is completely soluble at the desired concentration, such as acetone, alkylated naphthalenes, xylene or other organic solvents. Granular formulations, wherein the toxicant is carried on relatively coarse particles, are of particular utility for aerial distribution or for penetration of cover crop canopy. Pressurized sprays, typically aerosols wherein the active ingredient is dispersed in finely divided form as a result of vaporization of a low boiling dispersant solvent carrier, such as the Freons, may also be used. Water-soluble or water-dispersible granules are also useful formulations for herbicidal application of the present compounds. Such granular formulations are free-flowing, non-dusty, and readily water-soluble or water-miscible. This soluble or dispersible granular formulations described in U.S. patent No. 3,920,442, incorporated herein by reference are useful herein with the present herbicidal compounds.

The active herbicidal compounds of this invention may be formulated and/or applied with insecticides, fungicides, nematicides, plant growth regulators, fertilizers, or other agricultural chemicals and may be used as effective soil sterilants as well as selective herbicides in agriculture. In applying an active compound of this invention, whether formulated alone or with other agricultural chemicals, an effective amount and concentration of the active compound is of course employed.

The active herbicidal compounds of this invention may be used in combination with other herbicides, e.g. they may be mixed with, say, an equal or larger amount of a known herbicide such as chloroacetanilide herbicides such as 2-chloro-N-(2,6-diethylphenyl)-N-(methoxymethyl)acetamide (alachlor), 2-chloro-N-(2-ethyl-6-methylpheny)-N-(2-methoxy-1-methylethyl)acetamide (metolachlor), and N-chloroacetyl-N-(2,6-diethylphenyl)glycine (diethatyl-ethyl); benzothiadiazinone herbicides such as 3-(1-methylethyl)-(1H)-2,1,3-benzothiadiazin-4-(3H)-one-2,2-dioxide (bentazon); triazine herbicides such as 6-chloro-N-ethyl-N-(1-methylethyl)-1,3,5-triazine-2,4-diamine (atrazine), and 2-{[4-chloro-6-(ethylamino)-1,3,5-triazin-2-yl]-amino}-2-methylpropanenitrile (cyanazine); dinitrolaniline herbicides such as 2,6-dinitro-N,N-dipropyl-4-(trifluoromethyl)benzeneamine (trifluralin); and aryl urea herbicides such as N'-(3,4-dichlorophenyl)-N,N-dimethylurea (diuron) and N,N-dimethyl-N'-[3-(trifluoromethyl)phenyl]urea (fluometuron).

It is apparent that various modifications may be made in the formulation and application of the compounds of this ivnention without departing from the inventive concepts herein as defined in the claims.

TABLE 1
Representative Compounds

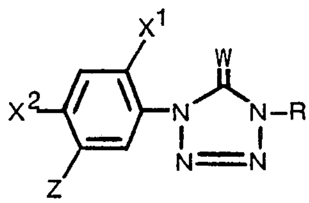

| Cpd No. | $X^1$ | $X^2$ | Z | R | W |
|---|---|---|---|---|---|
| 1 | Cl | Cl | Cl | n-$C_3H_7$ | O |
| 2 | Cl | Cl | Cl | $CH_2CH=CH_2$ | O |
| 3 | Cl | Cl | Cl | $CH_2OCH_3$ | O |
| 4 | Cl | Cl | $CH_3$ | n-$C_3H_7$ | O |
| 5 | Cl | Cl | $CH_3$ | $CH_2CH=CH_2$ | O |
| 6 | Cl | Cl | $OCH_3$ | $CH_3$ | O |
| 7 | Cl | Cl | $OCH_3$ | $C_2H_5$ | O |
| 8 | F | F | $OCH_3$ | $C_2H_5$ | O |
| 9 | Cl | Cl | $OCH_3$ | n-$C_3H_7$ | O |
| 10 | F | F | $OCH_3$ | n-$C_3H_7$ | O |
| 11 | F | Cl | $OCH_3$ | n-$C_3H_7$ | O |
| 12 | Cl | Br | $OCH_3$ | n-$C_3H_7$ | O |
| 13 | Br | Cl | $OCH_3$ | n-$C_3H_7$ | O |
| 14 | Cl | Cl | $OCH_3$ | n-$C_4H_9$ | O |
| 15 | Cl | Cl | $OCH_3$ | $CH(CH_3)_2$ | O |
| 16 | Cl | Cl | $OCH_3$ | $CH_2CH(CH_3)_2$ | O |
| 17 | Cl | Cl | $OCH_3$ | $CHF_2$ | O |
| 18 | Cl | Cl | $OCH_3$ | $CH_2CH_2F$ | O |
| 19 | Cl | Cl | $OCH_3$ | $(CH_2)_2CH_2F$ | O |
| 20 | Cl | Cl | $OCH_3$ | $CH_2CH=CH_2$ | O |
| 21 | Cl | Cl | $OC_2H_5$ | $CH_2CH=CH_2$ | O |
| 22 | Cl | Cl | $OC_2H_5$ | $CH_2OCH_3$ | O |
| 23 | Cl | Cl | O-n-$C_3H_7$ | $CH_3$ | O |

TABLE 1 (Continued)

| Cpd No. | $X^1$ | $X^2$ | Z | R | W |
|---|---|---|---|---|---|
| 24 | Cl | Cl | O-n-$C_3H_7$ | n-$C_3H_7$ | O |
| 25 | Cl | Cl | O-n-$C_3H_7$ | $CH_2CH{=}CH_2$ | O |
| 26 | Cl | Cl | $OCH(CH_3)_2$ | n-$C_3H_7$ | O |
| 27 | Cl | Cl | $OCH(CH_3)_2$ | $CH_2CH{=}CH_2$ | O |
| 28 | Br | Br | $OCH(CH_3)_2$ | $CH_2CH{=}CH_2$ | O |
| 29 | Br | Br | $OCH(CH_3)_2$ | $CH_2OCH_3$ | O |
| 30 | Cl | Cl | O-n-$C_4H_9$ | n-$C_3H_7$ | O |
| 31 | Cl | Cl | $OCH_2CH_2F$ | n-$C_3H_7$ | O |
| 32 | Cl | Cl | $O(CH_2)_2CH_2F$ | n-$C_3H_7$ | O |
| 33 | Cl | Cl | $OCHF_2$ | n-$C_3H_7$ | O |
| 34 | Cl | Cl | $OCH_2CF_3$ | n-$C_3H_7$ | O |
| 35 | F | F | $OCH(CH_3)_2$ | n-$C_3H_7$ | O |
| 36 | Cl | Br | $OCH(CH_3)_2$ | n-$C_3H_7$ | O |
| 37 | Cl | Cl | $OCH_2CH(CH_3)_2$ | n-$C_3H_7$ | O |
| 38 | Cl | Cl | $OCH_2CH_2OH$ | n-$C_3H_7$ | O |
| 39 | Cl | Cl | $OCH_2CH{=}CH_2$ | n-$C_3H_7$ | O |
| 40 | Cl | Cl | $OCH_2C(Cl){=}CH_2$ | n-$C_3H_7$ | O |
| 41 | Cl | Cl | $OCH_2C{\equiv}CH$ | $CH_3$ | O |
| 42 | Cl | Cl | $OCH_2C{\equiv}CH$ | $C_2H_5$ | O |
| 43 | F | F | $OCH_2C{\equiv}CH$ | $C_2H_5$ | O |
| 44 | Cl | F | $OCH_2C{\equiv}CH$ | $C_2H_5$ | O |
| 45 | Cl | Cl | $OCH_2C{\equiv}CH$ | n-$C_3H_7$ | O |
| 46 | F | F | $OCH_2C{\equiv}CH$ | n-$C_3H_7$ | O |
| 47 | Cl | F | $OCH_2C{\equiv}CH$ | n-$C_3H_7$ | O |
| 48 | F | Cl | $OCH_2C{\equiv}CH$ | n-$C_3H_7$ | O |
| 49 | Cl | Br | $OCH_2C{\equiv}CH$ | n-$C_3H_7$ | O |
| 50 | Br | Cl | $OCH_2C{\equiv}CH$ | n-$C_3H_7$ | O |
| 51 | Cl | Cl | $OCH_2C{\equiv}CH$ | $CH(CH_3)_2$ | O |
| 52 | Cl | Cl | $OCH_2C{\equiv}CH$ | n-$C_4H_9$ | O |
| 53 | Cl | Cl | $OCH_2C{\equiv}CH$ | $CH_2CH(CH_3)_2$ | O |
| 54 | Cl | Cl | $OCH_2C{\equiv}CH$ | $CHF_2$ | O |
| 55 | Cl | Cl | $OCH_2C{\equiv}CH$ | $CH_2CH_2F$ | O |

TABLE 1 (continued)

| Cpd No. | $X^1$ | $X^2$ | Z | R | W |
|---|---|---|---|---|---|
| 56 | Cl | Cl | $OCH_2C{\equiv}CH$ | $(CH_2)_2CH_2F$ | O |
| 57 | Cl | Cl | $OCH_2C{\equiv}CH$ | $(CH_2)_2CH_2Br$ | O |
| 58 | Cl | Cl | $OCH_2C{\equiv}CH$ | $CH_2{=}CH_2$ | O |
| 59 | Cl | Cl | $OCH_2C{\equiv}CH$ | $CH_2OCH_3$ | O |
| 60 | Cl | Cl | $OCH_2C{\equiv}CH$ | $CH_2OC_2H_5$ | O |
| 61 | Cl | Cl | $OCH_2C{\equiv}CI$ | n-$C_3H_7$ | O |
| 62 | Cl | Cl | $OCH_2C{\equiv}CCH_3$ | n-$C_3H_7$ | O |
| 63 | Cl | Cl | $OCH_2CN$ | n-$C_3H_7$ | O |
| 64 | Cl | Cl | $OCH(CH_3)CN$ | n-$C_3H_7$ | O |
| 65 | Cl | Cl | $OCH(CN)CH(CH_3)_2$ | n-$C_3H_7$ | O |
| 66 | Cl | Cl | $OCH_2OCH_3$ | $C_2H_5$ | O |
| 67 | Cl | Cl | $OCH_2OCH_3$ | n-$C_3H_7$ | O |
| 68 | F | F | $OCH_2OCH_3$ | n-$C_3H_7$ | O |
| 69 | F | Cl | $OCH_2OCH_3$ | n-$C_3H_7$ | O |
| 70 | Cl | Br | $OCH_2OCH_3$ | n-$C_3H_7$ | O |
| 71 | Cl | Cl | $OCH_2OC_2H_5$ | n-$C_3H_7$ | O |
| 72 | Cl | Cl | $OCH_2OCH_3$ | $(CH_2)_2CH_2F$ | O |
| 73 | Cl | Cl | $O(CH_2)_2OC_2H_5$ | n-$C_3H_7$ | O |
| 74 | Cl | Br | $O(CH_2)_2OC_2H_5$ | n-$C_3H_7$ | O |
| 75 | Cl | Cl | $O(CH_2)_2OCH{=}CH_2$ | n-$C_3H_7$ | O |
| 76 | Cl | Cl | O 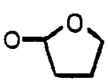 | n-$C_3H_7$ | O |
| 77 | Cl | Cl | $OCH_2$ 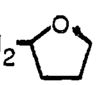 | n-$C_3H_7$ | O |
| 78 | Cl | Cl | $OCH_2$ 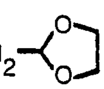 | n-$C_3H_7$ | O |
| 79 | Cl | Cl | O 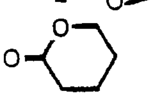 | n-$C_3H_7$ | O |
| 80 | Cl | Cl | $OCH_2$ 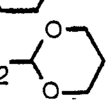 | n-$C_3H_7$ | O |
| 81 | Cl | Cl | $O-\overset{O}{\overset{\|}{C}}CH_3$ | n-$C_3H_7$ | O |

TABLE 1 (continued)

| Cpd No. | $X^1$ | $X^2$ | Z | R | W |
|---|---|---|---|---|---|
| 82 | Cl | Cl | $OCH_2CO_2H$ | n-$C_3H_7$ | O |
| 83 | Cl | Cl | $OCH_2CO_2CH_3$ | n-$C_3H_7$ | O |
| 84 | Cl | Cl | $OCH_2CO_2C_2H_5$ | n-$C_3H_7$ | O |
| 85 | Cl | Cl | $OCH_2SCH_3$ | n-$C_3H_7$ | O |
| 86 | Cl | Cl | $OSO_2CH_3$ | n-$C_3H_7$ | O |
| 87 | Cl | Br | $OSO_2CH_3$ | n-$C_3H_7$ | O |
| 88 | Cl | Cl | $OSO_2CH(CH_3)_2$ | n-$C_3H_7$ | O |
| 89 | Cl | Cl | $OSO_2CF_3$ | n-$C_3H_7$ | O |
| 90 | Cl | $CH_3$ | $OCH_3$ | n-$C_3H_7$ | O |
| 91 | Cl | $CH_3$ | $OCH(CH_3)_2$ | n-$C_3H_7$ | O |
| 92 | Cl | $CH_3$ | $OCH_2C{\equiv}CH$ | n-$C_3H_7$ | O |
| 93 | Cl | $CH_3$ | $O(CH_2)_2OC_2H_5$ | n-$C_3H_7$ | O |
| 94 | Cl | $CF_3$ | $OCH_3$ | n-$C_3H_7$ | O |
| 95 | $CH_3$ | Cl | $OCH_3$ | n-$C_3H_7$ | O |
| 96 | $CH_3$ | Cl | $OCH_3$ | $CH_2CH{=}CH_2$ | O |
| 97 | $CH_3$ | Cl | $OCH_2C{\equiv}CH$ | n-$C_3H_7$ | O |
| 98 | $CH_3$ | Cl | $OCH_2C{\equiv}CH$ | $CH_2CH{=}CH_2$ | O |
| 99 | $CF_3$ | Cl | $OCH_2OCH_3$ | n-$C_3H_7$ | O |
| 100 | Cl | Cl | $OCH(CH_3)_2$ | $CH(CH_3)_2$ | O |
| 101 | Cl | Cl | $OCH(CH_3)_2$ | $CH_2OCH_3$ | O |
| 102 | Cl | Br | 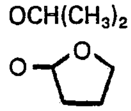 | n-$C_3H_7$ | O |
| 103 | Cl | Br | 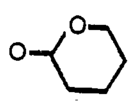 | n-$C_3H_7$ | O |
| 104 | Cl | Cl | $OCH_3$ | $CH{=}CH_2$ | O |
| 105 | Cl | F | $OCH_3$ | $C_2H_5$ | O |
| 106 | Cl | F | $OCH_3$ | n-$C_3H_7$ | O |
| 107 | Cl | $CH_2Br$ | $OCH(CH_3)_2$ | n-$C_3H_7$ | O |
| 108 | Cl | $CF_3$ | $OCH(CH_3)_2$ | n-$C_3H_7$ | O |
| 109 | Cl | Cl | H | n-$C_3H_7$ | O |
| 110 | Cl | Cl | H | $CH(CH_3)_2$ | O |

TABLE 1 (continued)

| Cpd No. | $X^1$ | $X^2$ | Z | R | W |
|---|---|---|---|---|---|
| 111 | Cl | Cl | H | $CH_2OCH_3$ | O |
| 112 | Cl | Cl | H | $CH_2SCH_3$ | O |
| 113 | Cl | Cl | H | $CH_2CH=CH_2$ | O |
| 114 | Cl | Cl | H | $CH_2CN$ | O |
| 115 | F | Cl | $OCH_2C\equiv CI$ | $n-C_3H_7$ | O |
| 116 | F | Cl | $OCH_2C\equiv CH$ | $C_2H_5$ | O |
| 117 | Cl | Cl | $OCH_2OC_2H_5$ | $(CH_2)_2CH_2F$ | O |
| 118 | F | Cl | $OCH_3$ | $C_2H_5$ | O |
| 119 | Cl | Cl | $OCH_2C_6H_5$ | $CH_2C(O)CH_3$ | O |
| 120 | F | Cl | $OCH_2C_6H_5$ | $(CH_2)_2CH_2F$ | O |
| 121 | F | Cl | $OCH_2C\equiv CH$ | $(CH_2)_2CH_2F$ | O |
| 122 | Cl | Cl | $OCH_2CBr=CHBr$ | $n-C_3H_7$ | O |
| 123 | Cl | Cl | $OCH_2C_6H_5$ | $CH_2CH_2OH$ | O |
| 124 | Cl | Cl | $OCH(CH_3)CO_2C_2H_5$ | $n-C_3H_7$ | O |
| 125 | F | Br | $OCH_2C\equiv CH$ | $n-C_3H_7$ | O |
| 126 | Cl | Cl | $OCH(CH_3)CO_2C_2H_5$ | $(CH_2)_2CH_2F$ | O |
| 127 | Cl | Cl | $OCO_2C_2H_5$ | $(CH_2)_2CH_2F$ | O |
| 128 | F | Cl | $OCH(CH_3)CO_2C_2H_5$ | $(CH_2)_2CH_2F$ | O |
| 129 | Cl | Cl | $OCH(CH_3)C(O)CH_3$ | $(CH_2)_2CH_2F$ | O |
| 130 | Cl | Cl | $OCH(CH_3)CO_2CH(CH_3)_2$ | $(CH_2)_2CH_2F$ | O |
| 131 | Cl | Cl | $OCH_3$ | $CH_2CH_2C(O)H$ | O |
| 132 | Cl | Cl | $OCH(CH_3)CO_2CH_2CH_2F$ | $(CH_2)_2CH_2F$ | O |
| 133 | F | Cl | $OCH_2C\equiv CI$ | $(CH_2)_2CH_2F$ | O |
| 134 | Cl | Cl | $OCH(CH_3)CO_2CH_2CF_2$ | $(CH_2)_2CH_2F$ | O |
| 135 | Cl | Cl | $OCH(CH_3)CO_2CH(CH_2F)_2$ | $(CH_2)_2CH_2F$ | O |
| 136 | Cl | Cl | $OCH(CH_3)CN$ | $(CH_2)_2CH_2F$ | O |
| 137 | F | Cl | $OCH(CH_3)CO_2C(CH_3)_3$ | $(CH_2)_2CH_2F$ | O |
| 138 | F | Cl | $OCH(CH_3)CO_2CH(CH_3)_2$ | $(CH_2)_2CH_2F$ | O |
| 139 | F | Cl | $OCH(CH_3)CN$ | $(CH_2)_2CH_2F$ | O |
| 140 | F | Cl | $OCH(CH_3)CO_2CH(C_2H_5)_2$ | $(CH_2)_2CH_2F$ | O |
| 141 | F | Cl | $OCH_2C\equiv CH$ | $(CH_2)_2CHF_2$ | O |
| 142 | F | Cl | $OCH(CH_3)CO_2CH(CH_3)CH(CH_3)_2$ | $(CH_2)_2CH_2F$ | O |

TABLE 1 (continued)

| Cpd No. | $X^1$ | $X^2$ | Z | R | W |
|---|---|---|---|---|---|
| 143 | F | Cl | $OCH(CH_3)_2$ | H | S |
| 144 | F | Cl | OH | $(CH_2)_2CH_2F$ | O |
| 145 | Cl | Cl | $OSO_2CF_3$ | $(CH_2)_2CH_2F$ | O |
| 146 | Cl | Cl | $OCH_3$ | $(CH_2)_2CH_2F$ | O |
| 147 | Cl | Cl | $NO_2$ | n-$C_3H_7$ | O |
| 148 | Cl | Cl | $OCH_3$ | $CH_2CH_2C(O)H$ | S |
| 149 | Cl | Cl | $OCH_3$ | $(CH_2)_2CHF_2$ | S |
| 150 | F | Cl | $OCH_2C_6H_5$ | $CH_2CH_2C(O)H$ | O |
| 151 | Cl | Cl | $OCH(CH_3)_2$ | $CH_2CH_2C(O)H$ | S |
| 152 | F | Cl | $OCH(CH_3)CO_2CH(CH_2F)_2$ | $(CH_2)_2CH_2F$ | O |
| 153 | F | Cl | $OCH(CH_3)CO_2CH[CH(CH_3)_2]_2$ | $(CH_2)_2CH_2F$ | O |
| 154 | F | Cl | $OCH(CH_3)CO_2N{=}C(CH_3)_2$ | $(CH_2)_2CH_2F$ | O |
| 155 | F | Cl | $OCH_2C_6H_5$ | $CH(CH_3)_2$ | O |
| 156 | F | Cl | $OCH(CH_3)_2$ | $CH_2CH_2C(O)H$ | S |
| 157 | F | Cl | $OCH_2C_6H_5$ | $CH_3$ | O |
| 158 | F | Cl | $OCH(CH_3)CO_2CH(CH_3)C_2H_5$ | $(CH_2)_2CH_2F$ | O |
| 159 | F | Cl | $OCH_2C{\equiv}CH$ | $CH(CH_3)_2$ | O |
| 160 | F | Cl | $OCH(CH_3)C(O)NH_2$ | $(CH_2)_2CH_2F$ | O |
| 161 | F | Cl | $OCH_2C{\equiv}CH$ | $CH_3$ | O |
| 162 | F | Cl | $OCH_2C_6H_5$ | n-$C_3H_7$ | O |
| 163 | Cl | Cl | $OCH(CH_3)_2$ | n-$C_3H_7$ | O |
| 164 | F | Cl | $OCH(CH_3)CO_2\underline{CH(CH_2)_3CH_2}$ | $(CH_2)_2CH_2F$ | O |
| 165 | F | Cl | $OCH(C_2H_5)CO_2CH(CH_3)_2$ | $(CH_2)CH_2F$ | O |
| 166 | F | Cl | $OCH(CH_3)CO_2CH(CH_3)C{\equiv}CH$ | $(CH_2)_2CH_2F$ | O |
| 167 | F | Cl | $OCH(CH_3)CO_2C(CH_3)_2C{\equiv}CH$ | $(CH_2)_2CH_2F$ | O |
| 168 | F | Cl | $OCH_2C{\equiv}CH$ | n-$C_4H_9$ | O |
| 169 | F | Cl | $OCH(CH_3)C{\equiv}CH$ | $(CH_2)_2CH_2F$ | O |
| 170 | Cl | Cl | $OCH(CH_3)C{\equiv}CH$ | n-$C_3H_7$ | O |
| 171 | F | Cl | $OCH(CH_3)CO_2CH_2\underline{CHCH_2CH_2}$ | $(CH_2)_2CH_2F$ | O |
| 172 | F | Cl | $OCH(CH_3)_2$ | $(CH_2)_2CHF_2$ | S |
| 173 | F | Cl | $OCH_2C{\equiv}CH$ | $CH_2CHFCH_3$ | O |
| 174 | F | Cl | $OCH_2C{\equiv}CH$ | $(CH_2)_2CHF_2$ | S |

TABLE 1 (continued)

| Cpd No. | $X^1$ | $X^2$ | Z | R | W |
|---|---|---|---|---|---|
| 175 | F | Cl | $OCH(CH_3)CO_2CH(CH_3)_2$ | $(CH_2)_2CHF_2$ | S |
| 176 | F | Cl | $OCH_2C_6H_5$ | $CH_2C(O)CH_3$ | O |
| 177 | F | Cl | OH | $(CH_2)_2CHF_2$ | S |
| 178 | F | Cl | $OCH_2C_6H_5$ | $CH_2CHFCH_3$ | O |
| 179 | F | Cl | $OCH(CH_3)CO_2H$ | H | O |
| 180 | F | Cl | $OCH_2C_6H_5$ | $CH(CH_3)C_2H_5$ | O |
| 181 | F | Cl | OH | $CH(CH_3)C_2H_5$ | O |
| 182 | F | Cl | $OCH(CH_3)CO_2H$ | $(CH_2)_2CH_2F$ | O |
| 183 | F | Cl | $OCH(CH_3)CO_2CH(CH_2OCH_3)_2$ | $(CH_2)_2CH_2F$ | O |
| 184 | F | Cl | $OCH(CH_3)CO_2C_3H_7$ | $(CH_2)_2CHF$ | O |
| 185 | F | Cl | $OCH_2C\equiv CH$ | $CH(CH_3)C_2H_5$ | O |
| 186 | F | Cl | $NO_2$ | H | O |
| 187 | F | Cl | H | $(CH_2)_2CH_2F$ | O |
| 188 | F | Cl | $OCH(CH_3)CO_2C(CH_3)_2CN$ | $(CH_2)_2CH_2F$ | O |
| 189 | F | Cl | $NO_2$ | $(CH_2)_2CH_2F$ | O |
| 190 | F | Cl | $OCH_2OCH_3$ | $(CH_2)_2CH_2F$ | O |
| 191 | F | Cl | $NHC(O)N(CH_3)_2$ | $(CH_2)_2CH_2F$ | O |
| 192 | F | Cl | $NHC(O)NHCH_2CH(CH_3)_2$ | $(CH_2)_2CH_2F$ | O |
| 193 | F | Cl | $OCH_2C\equiv CH$ | $CH_2OCH_3$ | O |
| 194 | F | Cl | 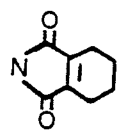 | $(CH_2)_2CH_2F$ | O |
| 195 | F | Cl | $N(SO_2CH_3)_2$ | $(CH_2)_2CH_2F$ | O |
| 196 | F | Cl | OH | $CH_2OCH_3$ | O |
| 197 | F | Cl | $OCH_2C_6H_5$ | $CH_2OCH_3$ | O |
| 198 | F | Cl | $OCH_2C_6H_5$ | $CH_2SCH_3$ | O |
| 199 | F | Cl | $OCH(CH_3)C(O)N(CH_2CH{=}CH_2)_2$ | $(CH_2)_2CH_2F$ | O |
| 200 | F | Cl | $N(CH_2CN)C(O)CH_3$ | $(CH_2)_2CH_2F$ | O |
| 201 | F | Cl | $N(C_2H_5)C(O)CH_3$ | $(CH_2)_2CH_2F$ | O |
| 202 | F | Cl | $NHC(O)CH_3$ | $(CH_2)_2CH_2F$ | O |
| 203 | F | Cl | $O(CH_2)_2OC_2H_5$ | $(CH_2)_2CH_2F$ | O |
| 204 | F | Cl | $OCH(CH_3)_2$ | $(CH_2)_2CH_2F$ | O |

TABLE 1 (continued)

| Cpd No. | $X^1$ | $X^2$ | Z | R | W |
|---|---|---|---|---|---|
| 205 | F | Cl | $OCH(CH_3)CH_2OCH_3$ | $(CH_2)_2CH_2F$ | O |
| 206 | F | Br | $OCH_2C{\equiv}CH$ | $(CH_2)_2CH_2F$ | O |
| 207 | F | Cl | $NH_2$ | $(CH_2)_2CH_2F$ | O |
| 208 | F | Cl | $OCH_2SCH_3$ | $(CH_2)_2CH_2F$ | O |
| 209 | F | Cl | $OCH(CH_3)CO_2C(CH_3)_2C{\equiv}CH$ | $(CH_2)_2CH_2F$ | O |
| 210 | F | Br | OH | $(CH_2)_2CH_2F$ | O |
| 211 | F | Br | $OCH_2C_6H_5$ | H | O |
| 212 | F | Br | $OCH_2C_6H_5$ | $(CH_2)_2CH_2F$ | O |
| 213 | F | Cl | $OCH_2C_6H_5$ | $SCF_3$ | O |
| 214 | F | Cl | $OCH_2C_6H_5$ | $CH_2CN$ | O |
| 215 | F | Cl | $CH_3$ | $(CH_2)_2CH_2F$ | O |
| 216 | F | Cl | $OCH_2S(O)CH_3$ | $(CH_2)_2CH_2F$ | O |
| 217 | F | $NO_2$ | F | $(CH_2)_2CH_2F$ | O |
| 218 | F | Cl | 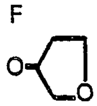 | $(CH_2)_2CH_2F$ | O |
| 219 | F | Cl | $O(CH_2)_2OCH_3$ | $(CH_2)_2CH_2F$ | O |
| 220 | F | $NO_2$ | $OCH_2C{\equiv}CH$ | $(CH_2)_2CH_2F$ | O |
| 221 | F | Cl | $OCH(CH_3)C(O)CH_3$ | $(CH_2)_2CH_2F$ | O |
| 222 | F | Cl | $OCH(CH_3)C(CH_3){=}N\text{-}OH$ | $(CH_2)_2CH_2F$ | O |
| 223 | Cl | Cl | $NHCH_2CO_2C_2H_5$ | $n\text{-}C_3H_7$ | O |
| 224 | F | Br | $OCH_2OCH_3$ | $(CH_2)_2CH_2F$ | O |
| 225 | F | Br | $OCH(CH_3)C{\equiv}CH$ | $(CH_2)_2CH_2F$ | O |
| 226 | F | H | F | $(CH_2)_2CH_2F$ | O |
| 227 | F | $CH_3$ | $OCH_2C_6H_5$ | $(CH_2)_2CH_2F$ | O |
| 228 | F | $CH_3$ | OH | $(CH_2)_2CH_2F$ | O |
| 229 | F | $CH_3$ | $OCH_2C{\equiv}CH$ | $(CH_2)_2CH_2F$ | O |
| 230 | F | $CH_3$ | $OCH(CH_3)CO_2CH(CH_3)_2$ | $(CH_2)_2CH_2F$ | O |
| 231 | F | $C_2H_5$ | $OCH_2C_6H_5$ | $(CH_2)_2CH_2F$ | O |
| 232 | F | $C_2H_5$ | OH | $(CH_2)_2CH_2F$ | O |
| 233 | F | $C_2H_5$ | $OCH_2C{\equiv}CH$ | $(CH_2)_2CH_2F$ | O |
| 234 | F | $C_2H_5$ | $OCH(CH_3)CO_2CH(CH_3)_2$ | $(CH_2)_2CH_2F$ | O |

TABLE 1 (continued)

| Cpd No. | $X^1$ | $X^2$ | Z | R | W |
|---|---|---|---|---|---|
| 235 | F | Cl | $OCH_2C{\equiv}CH$ | $CH_2CF_2CH_3$ | O |
| 236 | F | Cl | $OCH(CH_3)CO_2CH(CH_3)_2$ | $CH_2CF_2CH_3$ | O |
| 237 | F | Cl | $SCH_2CN$ | $(CH_2)_2CH_2F$ | O |
| 238 | F | Cl | $SCH_2C{\equiv}CH$ | $(CH_2)_2CH_2F$ | O |
| 239 | F | Cl | $SCH(CH_3)CO_2CH(CH_3)_2$ | $(CH_2)_2CH_2F$ | O |
| 240 | F | Cl | $N(CH_3)(SO_2CH_3)$ | $(CH_2)_2CH_2F$ | O |
| 241 | F | Cl | $N(C_2H_5)(SO_2CH_3)$ | $(CH_2)_2CH_2F$ | O |
| 242 | F | Cl | $N(CH_3)_2$ | $(CH_2)_2CH_2F$ | O |
| 243 | F | Cl | $N(C_2H_5)_2$ | $(CH_2)_2CH_2F$ | O |
| 244 | F | Cl | $N(SO_2CF_3)_2$ | $(CH_2)_2CH_2F$ | O |
| 245 | F | Cl | $NHCO_2CH_3$ | $(CH_2)_2CH_2F$ | O |
| 246 | F | Cl | $NHCH_2C{\equiv}CH$ | $(CH_2)_2CH_2F$ | O |
| 247 | F | Cl | $NHCH(CH_3)CO_2C_2H_5$ | $(CH_2)_2CH_2F$ | O |
| 248 | F | Cl | $NHCH_2CO_2C_2H_5$ | $(CH_2)_2CH_2F$ | O |
| 249 | F | Cl | 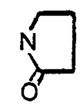 | $(CH_2)_2CH_2F$ | O |
| 250 | F | Cl | $N{=}C(CH_3)_2$ | $(CH_2)_2CH_2F$ | O |
| 251 | F | Cl | $N{=}CHCH_3$ | $(CH_2)_2CH_2F$ | O |
| 252 | F | Cl | $OCH(CH_3)OCH_3$ | $(CH_2)_2CH_2F$ | O |
| 253 | F | Cl | $OCH(CH_3)S(O)CH_3$ | $(CH_2)_2CH_2F$ | O |
| 254 | F | Cl | 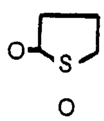 | $(CH_2)_2CH_2F$ | O |
| 255 | F | Cl | $OCH(CH_3)CON(CH_3)_2$ | $(CH_2)_2CH_2F$ | O |
| 256 | F | Cl | $OCH(CH_3)CONHS(O)_2$- 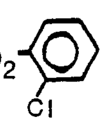 | $(CH_2)_2CH_2F$ | O |
| 257 | F | Cl | $OCH(CH_3)CONHS(O)_2CH_3$ | $(CH_2)_2CH_2F$ | O |
| 258 | $CF_3$ | Cl | $OCH_2C{\equiv}CH$ | $(CH_2)_3CH_2F$ | O |
| 259 | F | Cl | $OCH_2C{\equiv}CH$ | $(CH_2)_2CH_2F$ | S |
| 260 | F | Cl | $OC(CH_3)_2C{\equiv}CH$ | $(CH_2)_2CH_2F$ | O |

TABLE 1 (continued)

| Cpd No. | $X^1$ | $X^2$ | Z | R | W |
|---|---|---|---|---|---|
| 261 | F | Cl | $OCH_2C{\equiv}CH$ | $CH_2CH{=}CHF$ | O |
| 262 | F | Cl | CN | $(CH_2)_2CH_2F$ | O |
| 263 | F | Cl | $CO_2C_2H_5$ | $(CH_2)_2CH_2F$ | O |
| 264 | F | Cl | $CO_2H$ | $(CH_2)_2CH_2F$ | O |
| 265 | F | Cl | $SO_3H$ | $(CH_2)_2CH_2F$ | O |
| 266 | F | Cl | $SO_2N(C_2H_5)_2$ | $(CH_2)_2CH_2F$ | O |
| 267 | F | Cl | $SO_2NHC_6H_5$ | $(CH_2)_2CH_2F$ | O |
| 268 | F | Cl | $SO_2NH_2$ | $(CH_2)_2CH_2F$ | O |
| 269 | F | Cl | $CH(OC_2H_5)_2$ | $(CH_2)_2CH_2F$ | O |
| 270 | F | Cl | $C(CH_3)(OC_2H_5)_2$ | $(CH_2)_2CH_2F$ | O |
| 271 | F | Cl | $CH_2C{\equiv}CH$ | $(CH_2)_2CH_2F$ | O |
| 272 | F | Cl | H | $(CH_2)_2CH_2F$ | S |
| 273 | F | Cl | $OCH_2C_6H_5$ | H | O |
| 274 | Cl | Cl | $OCH_2C{\equiv}CH$ | $CH_2CH_2OCHF_2$ | O |
| 275 | F | Cl | $OCH_2C{\equiv}CH$ | $CH_2CH_2OCHF_2$ | O |
| 276 | Cl | Cl | $NH_2$ | n-$C_3H_7$ | O |
| 277 | Cl | Cl | $NHC(O)CH_3$ | n-$C_3H_7$ | O |
| 278 | F | Cl | $OCH_2C_6H_5$ | $(CH_2)_2CHF_2$ | O |
| 279 | F | Cl | OH | $(CH_2)_2CHF_2$ | O |
| 280 | Cl | Cl | $OCH_2C{\equiv}CH$ | $(CH_2)_2CHF_2$ | S |
| 281 | F | Cl | OH | $CH(CH_3)_2$ | O |
| 282 | F | Cl | OH | $CH_3$ | O |
| 283 | F | Cl | OH | n-$C_3H_7$ | O |
| 284 | F | Cl | $OCH_2C_6H_5$ | $CH_2CF_2CH_3$ | O |
| 285 | F | Cl | OH | $CH_2CF_2CH_3$ | O |
| 286 | F | Cl | OH | $(CH_2)_2CH_2F$ | S |
| 287 | F | Cl | OH | $CH_2CHFCH_3$ | O |
| 288 | F | Cl | $OCH_2CO_2C_2H_5$ | $(CH_2)_2CH_2F$ | O |
| 289 | F | Cl | H | H | O |
| 290 | F | Cl | NCO | $(CH_2)_2CH_2F$ | O |
| 291 | F | Cl | OH | $CH_2SCH_3$ | O |
| 292 | F | Cl | $OCH_2C{\equiv}CH$ | $CH_2SCH_3$ | O |

TABLE 1 (continued)

| Cpd No. | $X^1$ | $X^2$ | Z | R | W |
|---|---|---|---|---|---|
| 293 | F | Cl | $CH_3$ | H | O |
| 294 | Cl | Cl | $OCH_2C{\equiv}CH$ | $CH_2CF_2CH_3$ | O |
| 295 | F | H | F | H | O |
| 296 | F | Cl | $OCH_3$ | H | O |
| 297 | F | Cl | $OCH_2C{\equiv}CBr$ | $(CH_2)_2CH_2F$ | O |
| 298 | F | Cl | $OCH_2CO_2Na$ | $(CH_2)_2CH_2F$ | O |
| 299 | F | Cl | $OCH(CH_3)CO_2K$ | $(CH_2)_2CH_2F$ | O |
| 300 | F | Cl | $OCH(CH_3)CO_2NH_4$ | $(CH_2)_2CH_2F$ | O |
| 301 | F | Cl | $CO_2Na$ | $(CH_2)_2CH_2F$ | O |
| 302 | F | Cl | $SO_3Na$ | $(CH_2)_2CH_2F$ | O |
| 303 | F | Cl | $OCH(CH_3)CO_2H$ | $(CH_2)_2CH_2F$ | O |
| 304 | F | Cl | $OCH(CH_3)CH{=}CH_2$ | $(CH_2)_2CH_2F$ | O |
| 305 | F | Cl | $SCH(CH_3)_2$ | $(CH_2)_2CH_2F$ | O |
| 306 | F | Cl | $SCHF_2$ | $(CH_2)_2CH_2F$ | O |
| 307 | F | Cl | $SCH_2C{\equiv}CI$ | $(CH_2)_2CH_2F$ | O |
| 308 | F | Cl | $SCH_2CF_3$ | $(CH_2)_2CH_2F$ | O |
| 309 | F | Cl | $SCH_2OCH_3$ | $(CH_2)_2CH_2F$ | O |
| 310 | F | Cl | $SCH_2SCH_3$ | $(CH_2)_2CH_2F$ | O |
| 311 | F | Cl | $O(CH_2)_2F$ | $(CH_2)_2CH_2F$ | O |
| 312 | F | Cl | $O(CH_2)_2Br$ | $(CH_2)_2CH_2F$ | O |
| 313 | F | Cl | $OSO_2CH_3$ | $(CH_2)_2CH_2F$ | O |
| 314 | F | Cl | $OSO_2CF_3$ | $(CH_2)_2CH_2F$ | O |
| 315 | F | Cl | $OSO_2CH_2CH(CH_3)_2$ | $(CH_2)_2CH_2F$ | O |
| 316 | F | Cl | $OSO_2CHCl_2$ | $(CH_2)_2CH_2F$ | O |
| 317 | F | Cl | $OSO_2CH_2C_6H_5$ | $(CH_2)_2CH_2F$ | O |
| 318 | F | Cl | $OSO_2CH_2CN$ | $(CH_2)_2CH_2F$ | O |
| 319 | F | Cl | $OSO_2CH_2C{\equiv}CH$ | $(CH_2)_2CH_2F$ | O |
| 320 | F | Cl | $OSO_2CH_2C{\equiv}CI$ | $(CH_2)_2CH_2F$ | O |
| 321 | F | Cl | $OSO_2NHCH_3$ | $(CH_2)_2CH_2F$ | O |
| 322 | F | Cl | $OSO_2(CH_2)_2N(CH_3)_2$ | $(CH_2)_2CH_2F$ | O |
| 323 | F | Cl | $OSO_2(CH_2)_2SCH_2CH{=}CH_2$ | $(CH_2)_2CH_2F$ | O |
| 324 | F | Cl | $OSO_2(CH_2)_2OCH_2C{\equiv}CH$ | $(CH_2)_2CH_2F$ | O |
| 325 | F | Cl | $OSO_2(CH_2)_2OCH_2CO_2CH_3$ | $(CH_2)_2CH_2F$ | O |

TABLE 1 (continued

| Cpd No. | $X^1$ | $X^2$ | Z | R | W |
|---|---|---|---|---|---|
| 326 | F | Cl | $OSO_2(CH_2)_2OCH_2CO_2H$ | $(CH_2)_2CH_2F$ | O |
| 327 | F | Cl | $OSO_2(CH_2)_2OCH(CH_3)CO_2Na$ | $(CH_2)_2CH_2F$ | O |
| 328 | F | Cl | $OSO_2(CH_2)_2SCH(CH_3)CO_2CH_3$ | $(CH_2)_2CH_2F$ | O |
| 329 | F | Cl | $SCH_2CO_2H$ | $(CH_2)_2CH_2F$ | O |
| 330 | F | Cl | $SCH_2CO—SCH_3$ | $(CH_2)_2CH_2F$ | O |
| 331 | F | Cl | $OCH(CH_3)CO_2CH_2CH=CH_2$ | $(CH_2)_2CH_2F$ | O |
| 332 | F | Cl | $OCHFCO_2H$ | $(CH_2)_2CH_2F$ | O |
| 333 | F | Cl | $SCH_2CO_2N=C(CH_3)_2$ | $(CH_2)_2CH_2F$ | O |
| 334 | F | Cl | $OCHFCO_2N=C(SCH_3)(CH_3)$ | $(CH_2)_2CH_2F$ | O |
| 335 | F | Cl | $OCH(CH_3)CO_2N=C(SCH_3)(CH_3)$ | $(CH_2)_2CH_2F$ | O |
| 336 | F | Cl | $OCH(CH_3)CO_2N=C(CH_3)(C_2H_5)$ | $(CH_2)_2CH_2F$ | O |
| 337 | F | Cl | $OCH_2CO_2N=C(CH_3)_2$ | $(CH_2)_2CH_2F$ | O |
| 338 | F | Cl | $SCH(CH_3)C(CH_3)=NOH$ | $(CH_2)_2CH_2F$ | O |
| 339 | F | Cl | $OCH_2C(CH_3)=NOH$ | $(CH_2)_2CH_2F$ | O |
| 340 | F | Cl | $SCH(CH_3)C(CH_3)=N—OCH_3$ | $(CH_2)_2CH_2F$ | O |
| 341 | F | Cl | $OCH(CH_3)C(CH_3)=N—OCH(CH_3)_2$ | $(CH_2)_2CH_2F$ | O |
| 342 | F | Cl | $SCH(CH_3)COCH_3$ | $(CH_2)_2CH_2F$ | O |
| 343 | F | Cl | $OCH_2COCH_3$ | $(CH_2)_2CH_2F$ | O |
| 344 | F | Cl | $SCH_2COCH_3$ | $(CH_2)_2CH_2F$ | O |
| 345 | F | Cl | $N=C(SCH_3)(CH_3)$ | $(CH_2)_2CH_2F$ | O |
| 346 | F | Cl | $CO—SCH_3$ | $(CH_2)_2CH_2F$ | O |
| 347 | F | Cl | $CONH_2$ | $(CH_2)_2CH_2F$ | O |
| 348 | F | Cl | $CONHCH_3$ | $(CH_2)_2CH_2F$ | O |
| 349 | F | Cl | $CON(C_2H_5)_2$ | $(CH_2)_2CH_2F$ | O |
| 350 | F | Cl | $CONHC_6H_5$ | $(CH_2)_2CH_2F$ | O |
| 351 | F | Cl | $OCH_2C≡CH$ | $SCF_3$ | O |
| 352 | F | $CH_2F$ | $OCH_2C≡CH$ | $(CH_2)_2CH_2F$ | O |

TABLE 2
Characterizing Data

| Compound No. | mp(°C) | Empirical Formula | | Elemental Analysis C | H | N |
|---|---|---|---|---|---|---|
| 1 | 89—90 | $C_{10}H_9Cl_3N_4O$ | C | 39.05 | 2.95 | 18.22 |
| | | | F | 39.13 | 3.22 | 17.80 |
| 2 | 100—101 | $C_{10}H_7Cl_3N_4O$ | C | 39.31 | 2.31 | 18.34 |
| | | | F | 40.38 | 2.64 | 18.33 |
| 3 | 114—115 | $C_9H_7Cl_3N_4O_2$ | C | 34.92 | 2.28 | 18.10 |
| | | | F | 36.01 | 2.59 | 17.91 |
| 4 | 47—48 | $C_{11}H_{12}Cl_2N_4O$ | C | | | |
| | | | F | | | |
| 5 | 55—57 | $C_{11}H_{10}Cl_2N_4O$ | C | | | |
| | | | F | | | |
| 6 | 158—159 | $C_9H_8Cl_2N_4O_2$ | C | 39.29 | 2.93 | 20.37 |
| | | | F | 39.35 | 3.14 | 20.30 |
| 7 | 99—100 | $C_{10}H_{10}Cl_2N_4O_2$ | C | 41.54 | 3.49 | 19.38 |
| | | | F | 42.55 | 3.50 | 16.91 |
| 8 | 59—60 | $C_{10}H_{10}F_2O_2N_4$ | C | | | |
| | | | F | | | |
| 9 | 86—87 | $C_{11}H_{12}Cl_2N_4O_2$ | C | 43.57 | 3.99 | 18.48 |
| | | | F | 44.40 | 4.02 | 16.35 |
| 10 | 65—67 | $C_{11}H_{12}F_2N_4O_2$ | C | | | |
| | | | F | | | |
| 11 | 60—62 | $C_{11}H_{12}ClFN_4O_2$ | C | | | |
| | | | F | | | |
| 12 | 89—90 | $C_{11}H_{12}BrClN_4O_2$ | C | 38.00 | 3.48 | 16.11 |
| | | | F | 38.07 | 3.41 | 15.96 |
| 13 | 65—67 | $C_{11}H_{12}BrClN_4O_2$ | C | | | |
| | | | F | | | |
| 14 | 54—56 | $C_{12}H_{14}Cl_2N_4O_2$ | C | | | |
| | | | F | | | |
| 15 | 81—85 | $C_{11}H_{12}Cl_2N_4O_2$ | C | | | |
| | | | F | | | |

TABLE 2 (continued)

Characterizing Data

| Compound No. | mp(°C) | Empirical Formula | | Elemental Analysis C | H | N |
|---|---|---|---|---|---|---|
| 16 | 58—59 | $C_{12}H_{14}Cl_2N_4O_2$ | C | 45.44 | 4.45 | 17.66 |
| | | | F | 45.51 | 4.28 | 17.17 |
| 17 | 144—145 | $C_9H_6Cl_2F_2N_4O_2$ | C | 34.75 | 1.95 | 18.01 |
| | | | F | 34.97 | 2.14 | 18.01 |
| 18 | 110—111 | $C_{10}H_9Cl_2FN_4O_2$ | C | 39.11 | 2.95 | 18.24 |
| | | | F | 39.10 | 2.90 | 18.18 |
| 19 | 80—81 | $C_{11}H_{11}Cl_2FN_4O_2$ | C | 41.14 | 3.45 | 17.44 |
| | | | F | 41.39 | 3.36 | 16.64 |
| 20 | 107—108 | $C_{11}H_{10}Cl_2N_4O_2$ | C | 43.87 | 3.35 | 18.61 |
| | | | F | 43.95 | 3.40 | 17.05 |
| 21 | 103—104 | $C_{12}H_{12}Cl_2N_4O_2$ | C | 45.73 | 3.84 | 17.78 |
| | | | F | 45.99 | 3.85 | 15.99 |
| 22 | 108—109 | $C_{11}H_{12}Cl_2N_4O_3$ | C | 41.39 | 3.79 | 17.56 |
| | | | F | 43.97 | 4.04 | 14.24 |
| 23 | 86—87 | $C_{11}H_{12}Cl_2N_4O_2$ | C | 43.58 | 4.00 | 18.48 |
| | | | F | 43.51 | 4.02 | 18.28 |
| 24 | oil | $C_{13}H_{16}Cl_2N_4O_2$ | C | 47.14 | 4.87 | 16.91 |
| | | | F | 47.40 | 4.94 | 15.75 |
| 25 | 85—86 | $C_{13}H_{14}Cl_2N_4O_2$ | C | 47.43 | 4.29 | 17.02 |
| | | | F | 47.66 | 4.35 | 17.02 |
| 26 | oil | $C_{13}H_{16}Cl_2N_4O_2$ | C | 47.14 | 4.87 | 16.91 |
| | | | F | 47.77 | 4.99 | 15.05 |
| 27 | oil | $C_{13}H_{14}Cl_2N_4O_2$ | C | 47.43 | 4.29 | 17.02 |
| | | | F | 47.72 | 4.15 | 15.80 |
| 28 | oil | $C_{13}H_{14}Br_2N_4O_2$ | C | 37.34 | 3.38 | 13.40 |
| | | | F | 37.51 | 3.48 | 13.32 |
| 29 | 94—95 | $C_{12}H_{14}Br_2N_4O_3$ | C | 34.15 | 3.34 | 13.28 |
| | | | F | 34.28 | 3.41 | 13.28 |
| 30 | 71—72 | $C_{14}H_{18}Cl_2N_4O_2$ | C | 48.79 | 5.26 | 16.23 |
| | | | F | 50.48 | 5.91 | 16.71 |

TABLE 2 (continued)

Characterizing Data

| Compound No. | mp(°C) | Empirical Formula | | Elemental Analysis C | H | N |
|---|---|---|---|---|---|---|
| 31 | 74—75 | $C_{12}H_{13}Cl_2FN_4O_2$ | C | 43.00 | 3.91 | 16.72 |
| | | | F | 42.93 | 3.84 | 16.71 |
| 32 | 78—79 | $C_{13}H_{15}Cl_2FN_4O_2$ | C | 44.71 | 4.33 | 16.05 |
| | | | F | 44.72 | 4.28 | 15.83 |
| 33 | oil | $C_{11}H_{10}Cl_2F_2N_4O_2$ | C | | | |
| | | | F | | | |
| 34 | 83—84 | $C_{12}H_{11}Cl_2F_3N_4O_2$ | C | 38.83 | 2.99 | 15.10 |
| | | | F | 38.84 | 2.90 | 14.99 |
| 35 | 77—78 | $C_{13}H_{16}F_2N_4O_2$ | C | 52.34 | 5.41 | 18.77 |
| | | | F | 52.38 | 5.18 | 18.71 |
| 36 | 49—50 | $C_{13}H_{16}BrClN_4O_2$ | C | 41.56 | 4.29 | 14.92 |
| | | | F | 42.02 | 4.22 | 14.73 |
| 37 | 51—52 | $C_{14}H_{18}Cl_2N_4O_2$ | C | 48.70 | 5.26 | 16.23 |
| | | | F | 48.79 | 5.16 | 16.09 |
| 38 | 87—88 | $C_{12}H_{14}Cl_2N_4O_3$ | C | 43.26 | 4.24 | 16.82 |
| | | | F | 43.63 | 4.29 | 16.86 |
| 39 | 57—58 | $C_{13}H_{14}Cl_2N_4O_2$ | C | 47.43 | 4.29 | 17.02 |
| | | | F | 47.49 | 4.50 | 16.86 |
| 40 | 56—57 | $C_{13}H_{13}Cl_3N_4O_2$ | C | 42.94 | 3.60 | 15.41 |
| | | | F | 43.65 | 3.68 | 14.96 |
| 41 | 155 | $C_{11}H_8Cl_2N_4O_2$ | C | 44.17 | 2.69 | 18.73 |
| | | | F | 44.33 | 2.88 | 18.55 |
| 42 | 120—121 | $C_{12}H_{10}Cl_2N_4O_2$ | C | 46.03 | 3.22 | 17.89 |
| | | | F | 46.60 | 3.20 | 16.37 |
| 43 | 71—72 | $C_{12}H_{10}F_2N_4O_2$ | C | 51.43 | 3.59 | 19.99 |
| | | | F | 51.74 | 3.47 | 19.95 |
| 44 | 88—89 | $C_{12}H_{10}FClN_4O_2$ | C | 48.58 | 3.39 | 18.88 |
| | | | F | 48.70 | 3.37 | 18.90 |
| 45 | 70—72 | $C_{13}H_{12}Cl_2N_4O_2$ | C | | | |
| | | | F | | | |

TABLE 2 (continued)
Characterizing Data

| Compound No. | mp(°C) | Empirical Formula | | Elemental Analysis C | H | N |
|---|---|---|---|---|---|---|
| 46 | oil | $C_{13}H_{12}F_2N_4O_2$ | C | | | |
| | | | F | | | |
| 47 | 72—73 | $C_{13}H_{12}ClFO_2N_4$ | C | 50.25 | 3.89 | 18.03 |
| | | | F | 50.21 | 3.79 | 17.97 |
| 48 | 60—62 | $C_{13}H_{12}ClFN_4O_2$ | C | | | |
| | | | F | | | |
| 49 | 87—88 | $C_{13}H_{12}BrClN_4O_2$ | C | 42.01 | 3.26 | 15.08 |
| | | | F | 41.94 | 3.34 | 14.96 |
| 50 | 70—71 | $C_{13}H_{12}BrClN_4O_2$ | C | | | |
| | | | F | | | |
| 51 | 120—121 | $C_{13}H_{12}Cl_2N_4O_2$ | C | 47.72 | 3.70 | 17.12 |
| | | | F | 47.67 | 3.32 | 16.70 |
| 52 | 73—74 | $C_{14}H_{14}Cl_2N_4O_2$ | C | 49.27 | 4.14 | 16.42 |
| | | | F | 50.04 | 3.94 | 15.07 |
| 53 | 90—91 | $C_{14}H_{14}Cl_2N_4O_2$ | C | 49.28 | 4.14 | 16.42 |
| | | | F | 49.22 | 3.96 | 15.65 |
| 54 | 63—65 | $C_{11}H_6Cl_2F_2N_4O_2$ | C | | | |
| | | | F | | | |
| 55 | 118—120 | $C_{12}H_9Cl_2FN_4O_2$ | C | 44.53 | 2.69 | 16.69 |
| | | | F | 43.52 | 2.74 | 16.92 |
| 56 | 86—87 | $C_{13}H_{11}Cl_2FN_4O_2$ | C | 45.54 | 3.15 | 16.05 |
| | | | F | 45.23 | 3.21 | 16.23 |
| 57 | oil | $C_{13}H_{11}BrCl_2N_4O_2$ | C | 38.44 | 2.73 | 13.80 |
| | | | F | 39.40 | 2.83 | 13.03 |
| 58 | 115—116 | $C_{12}H_8Cl_2N_4O_2$ | C | 46.24 | 2.59 | 17.98 |
| | | | F | 47.12 | 2.83 | 16.93 |
| 59 | 127—128 | $C_{12}H_{10}Cl_2N_4O_3$ | C | 43.79 | 3.06 | 17.02 |
| | | | F | 44.42 | 3.01 | 17.03 |
| 60 | 79—80 | $C_{13}H_{12}Cl_2N_4O_3$ | C | 45.50 | 3.52 | 16.32 |
| | | | F | 45.84 | 3.18 | 17.07 |

TABLE 2 (continued)
Characterizing Data

| Compound No. | mp(°C) | Empirical Formula | | Elemental Analysis C | H | N |
|---|---|---|---|---|---|---|
| 61 | 134—138 | $C_{13}H_{11}Cl_2IN_4O_2$ | C | 34.46 | 2.45 | 12.37 |
| | | | F | 34.85 | 2.46 | 12.37 |
| 62 | 58—60 | $C_{14}H_{14}Cl_2N_4O_2$ | C | | | |
| | | | F | | | |
| 63 | oil | $C_{12}H_{11}Cl_2N_5O_2$ | C | 43.92 | 3.38 | 21.34 |
| | | | F | 43.48 | 3.37 | 19.09 |
| 64 | oil | $C_{13}H_{13}Cl_2N_5O_2$ | C | 45.63 | 3.82 | 20.46 |
| | | | F | 45.66 | 3.72 | 20.10 |
| 65 | oil | $C_{15}H_{17}Cl_2N_5O_2$ | C | | | |
| | | | F | | | |
| 66 | 112—113 | $C_{11}H_{12}Cl_2N_4O_3$ | C | 41.39 | 3.79 | 17.56 |
| | | | F | 42.09 | 3.81 | 17.35 |
| 67 | 76—77 | $C_{12}H_{14}Cl_2N_4O_3$ | C | 43.26 | 4.23 | 16.82 |
| | | | F | 43.73 | 3.97 | 16.82 |
| 68 | oil | $C_{12}H_{14}F_4N_4O_3$ | C | 47.99 | 4.70 | 18.66 |
| | | | F | 48.21 | 4.72 | 18.08 |
| 69 | oil | $C_{12}H_{14}ClFN_4O_3$ | C | | | |
| | | | F | | | |
| 70 | 72—73 | $C_{12}H_{14}BrClN_4O_3$ | C | 38.16 | 3.74 | 14.84 |
| | | | F | 38.14 | 3.88 | 14.95 |
| 71 | 60—61 | $C_{13}H_{16}Cl_2N_4O_3$ | C | 44.90 | 4.64 | 16.13 |
| | | | F | 45.20 | 4.52 | 15.89 |
| 72 | — | $C_{12}H_{13}Cl_2FN_4O_3$ | C | 41.04 | 3.73 | 15.96 |
| | | | F | 35.44 | 3.12 | 11.49 |
| 73 | 60—61 | $C_{14}H_{18}Cl_2N_4O_3$ | C | 46.42 | 5.29 | 15.47 |
| | | | F | 46.44 | 4.93 | 15.06 |
| 74 | 63—64 | $C_{14}H_{13}BrClN_4O_3$ | C | 41.45 | 4.47 | 13.81 |
| | | | F | 41.47 | 4.56 | 13.71 |
| 75 | 87—88 | $C_{14}H_{16}Cl_2N_4O_3$ | C | 46.81 | 4.49 | 15.60 |
| | | | F | 46.95 | 4.39 | 15.60 |

TABLE 2 (continued)

Characterizing Data

| Compound No. | mp(°C) | Empirical Formula | | Elemental Analysis C | H | N |
|---|---|---|---|---|---|---|
| 76 | 89—90 | $C_{14}H_{16}Cl_2N_4O_3$ | C | 46.81 | 4.49 | 15.60 |
| | | | F | 47.00 | 4.20 | 15.44 |
| 77 | 81—82 | $C_{15}H_{18}Cl_2N_4O_3$ | C | 48.26 | 4.86 | 15.01 |
| | | | F | 48.47 | 4.84 | 15.06 |
| 78 | 99—101 | $C_{14}H_{16}Cl_2N_4O_4$ | C | 44.81 | 4.30 | 14.93 |
| | | | F | 44.93 | 4.32 | 14.58 |
| 79 | 83—84 | $C_{15}H_{18}Cl_2N_4O_3$ | C | 48.27 | 4.86 | 15.01 |
| | | | F | 48.44 | 4.85 | 14.78 |
| 80 | oil | $C_{15}H_{18}Cl_2N_4O_4$ | C | | | |
| | | | F | | | |
| 81 | 91—92 | $C_{12}H_{12}Cl_2N_4O_3$ | C | 43.52 | 3.65 | 16.91 |
| | | | F | 43.58 | 3.76 | 16.83 |
| 82 | 134—136 | $C_{12}H_{12}Cl_2N_4O_4$ | C | | | |
| | | | F | | | |
| 83 | 76—78 | $C_{13}H_{14}Cl_2N_4O_4$ | C | | | |
| | | | F | | | |
| 84 | 80—81 | $C_{14}H_{16}Cl_2N_4O_4$ | C | 44.81 | 4.30 | 14.93 |
| | | | F | 45.45 | 4.22 | 13.48 |
| 85 | 58—59 | $C_{12}H_{14}Cl_2N_4O_2S$ | C | 41.28 | 4.04 | 16.05 |
| | | | F | 41.63 | 3.86 | 16.07 |
| 86 | 64—65 | $C_{11}H_{12}Cl_2N_4O_4S$ | C | 35.98 | 3.29 | 15.25 |
| | | | F | 36.37 | 3.28 | 14.55 |
| 87 | 75—76 | $C_{11}H_{12}BrClN_4O_4S$ | C | 32.09 | 2.94 | 13.61 |
| | | | F | 32.15 | 2.84 | 13.59 |
| 88 | 60—61 | $C_{13}H_{16}Cl_2N_4O_4S$ | C | 39.50 | 4.08 | 14.17 |
| | | | F | 39.76 | 4.00 | 13.98 |
| 89 | 52—53 | $C_{11}H_9Cl_2F_3N_4O_4S$ | C | 31.37 | 2.15 | 13.31 |
| | | | F | 31.76 | 2.13 | 13.38 |
| 90 | 74—75 | $C_{12}H_{15}ClN_4O_2$ | C | 50.97 | 5.35 | 19.82 |
| | | | F | 51.04 | 5.40 | 19.77 |

TABLE 2 (continued)
Characterizing Data

| Compound No. | mp(°C) | Empirical Formula | | Elemental Analysis C | H | N |
|---|---|---|---|---|---|---|
| 91 | 67—68 | $C_{14}H_{19}ClN_4O_2$ | C | 54.10 | 6.16 | 18.03 |
| | | | F | 54.39 | 6.22 | 17.95 |
| 92 | 89—90 | $C_{14}H_{15}ClN_4O_2$ | C | 54.81 | 4.93 | 18.27 |
| | | | F | 54.70 | 4.97 | 18.15 |
| 93 | 50—51 | $C_{15}H_{21}ClN_4O_3$ | C | 52.86 | 6.21 | 16.44 |
| | | | F | 53.20 | 6.33 | 16.29 |
| 94 | 82—83 | $C_{12}H_{12}ClF_3N_4O_2$ | C | 42.81 | 3.59 | 16.63 |
| | | | F | 42.05 | 3.42 | 16.02 |
| 95 | 59—60 | $C_{12}H_{15}ClN_4O_2$ | C | | | |
| | | | F | | | |
| 96 | 65—66 | $C_{12}H_{13}ClN_4O_2$ | C | | | |
| | | | F | | | |
| 97 | 100—101 | $C_{14}H_{15}ClN_4O_2$ | C | | | |
| | | | F | | | |
| 98 | 129—130 | $C_{14}H_{13}ClN_4O_2$ | C | | | |
| | | | F | | | |
| 99 | oil | $C_{13}H_{17}ClN_4O_3$ | C | | | |
| | | | F | | | |
| 100 | oil | $C_{13}H_{16}Cl_2N_4O_2$ | C | 47.14 | 4.87 | 16.91 |
| | | | F | 48.14 | 4.84 | 15.01 |
| 101 | 68—69 | $C_{12}H_{14}Cl_2N_4O_3$ | C | 43.26 | 4.24 | 16.81 |
| | | | F | 43.68 | 4.36 | 16.31 |
| 102 | 170—171 | $C_{14}H_{16}BrClN_4O_3$ | C | 41.66 | 3.99 | 13.87 |
| | | | F | 41.66 | 3.79 | 13.69 |
| 103 | 83—84 | $C_{15}H_{18}BrClN_4O_3$ | C | 43.13 | 4.34 | 13.41 |
| | | | F | 43.12 | 4.04 | 13.25 |
| 104 | 142—143 | $C_{10}H_8Cl_2N_4O_2$ | C | 41.83 | 2.81 | 19.51 |
| | | | F | 41.70 | 2.72 | 19.51 |
| 105 | 79—80 | $C_{10}H_{10}ClFN_4O_2$ | C | | | |
| | | | F | | | |

TABLE 2 (continued)
Characterizing Data

| Compound No. | mp(°C) | Empirical Formula | | Elemental Analysis C | H | N |
|---|---|---|---|---|---|---|
| 106 | 85—86 | $C_{11}H_{12}ClFN_4O_2$ | C | | | |
| | | | F | | | |
| 107 | 68—70 | $C_{14}H_{18}BrClN_4O_2$ | C | | | |
| | | | F | | | |
| 108 | oil | $C_{14}H_{16}ClF_3N_4O_2$ | C | 46.10 | 4.41 | 15.35 |
| | | | F | 45.33 | 4.41 | 14.12 |
| 109 | 40—41 | $C_{10}H_{10}Cl_2N_4O$ | C | 43.95 | 3.69 | 20.52 |
| | | | F | 44.42 | 3.84 | 20.34 |
| 110 | 58—59 | $C_{10}H_{10}Cl_2N_4O$ | C | 43.95 | 3.69 | 20.52 |
| | | | F | 43.64 | 3.69 | 20.37 |
| 111 | 116—117 | $C_9H_8Cl_2N_4O_2$ | C | 39.27 | 2.93 | 20.37 |
| | | | F | 39.29 | 2.90 | 20.45 |
| 112 | 99—100 | $C_9H_8Cl_2N_4SO$ | C | 37.12 | 2.77 | 19.24 |
| | | | F | 37.15 | 2.82 | 18.96 |
| 113 | 49—50 | $C_{10}H_8Cl_2N_4O$ | C | 44.30 | 2.97 | 20.66 |
| | | | F | 44.73 | 3.07 | 20.50 |
| 114 | 121—122 | $C_9H_5Cl_2N_5O$ | C | 40.02 | 1.87 | 25.93 |
| | | | F | 40.28 | 1.79 | 25.98 |
| 115 | 104—105 | $C_{13}H_{11}ClFIN_4O_2$ | C | 35.76 | 2.54 | 12.83 |
| | | | F | 38.87 | 3.03 | 12.84 |
| 116 | 90—91 | $C_{12}H_{10}ClFN_4O_2$ | C | 48.58 | 3.40 | 18.89 |
| | | | F | 49.30 | 3.49 | 18.80 |
| 117 | 45—46 | $C_{13}H_{15}Cl_2N_4O_3F$ | C | 42.75 | 4.15 | 15.34 |
| | | | F | 43.37 | 4.30 | 15.85 |
| 118 | 70—72 | $C_{10}H_{10}ClFN_4O_2$ | C | | | |
| | | | F | | | |
| 119 | 154—155 | $C_{17}H_{14}Cl_2N_4O_3$ | C | | | |
| | | | F | | | |
| 120 | 78—79 | $C_{17}H_{14}Cl_2N_4O_3$ | C | | | |
| | | | F | | | |

TABLE 2 (continued)

Characterizing Data

| Compound No. | mp(°C) | Empirical Formula | | Elemental Analysis C | H | N |
|---|---|---|---|---|---|---|
| 121 | 49—51 | $C_{13}H_{11}ClF_2N_4O_2$ | C | 47.50 | 3.38 | 17.04 |
| | | | F | 47.85 | 3.38 | 16.40 |
| 122 | 64—65 | $C_{13}H_{12}Br_2Cl_2N_2O_2$ | C | 32.06 | 2.48 | 11.51 |
| | | | F | 31.93 | 2.53 | 11.27 |
| 123 | 117—118 | $C_{16}H_{14}Cl_2N_4O_3$ | C | | | |
| | | | F | | | |
| 124 | oil | $C_{15}H_{18}Cl_2N_4O_4$ | C | 46.28 | 4.67 | 14.39 |
| | | | F | 48.50 | 5.49 | 11.68 |
| 125 | oil | $C_{13}H_{12}BrFN_4O_2$ | C | | | |
| | | | F | | | |
| 126 | oil | $C_{15}H_{17}Cl_2FN_4O_4$ | C | 44.24 | 4.21 | 13.76 |
| | | | F | 46.66 | 4.87 | 12.32 |
| 127 | oil | $C_{13}H_{13}Cl_2FN_4O_4$ | C | 41.18 | 3.46 | 14.78 |
| | | | F | 41.70 | 3.52 | 14.53 |
| 128 | oil | $C_{15}H_{17}ClF_2N_4O_4$ | C | 46.10 | 4.64 | 14.34 |
| | | | F | 46.95 | 4.16 | 13.65 |
| 129 | oil | $C_{14}H_{15}Cl_2FN_4O_3$ | C | 44.58 | 4.01 | 14.85 |
| | | | F | 44.89 | 3.93 | 14.05 |
| 130 | oil | $C_{16}H_{19}Cl_2FN_4O_4$ | C | 45.62 | 4.55 | 13.30 |
| | | | F | 45.95 | 4.56 | 12.98 |
| 131 | 73—74 | $C_{11}H_{10}Cl_2N_4O_3$ | C | 41.66 | 3.18 | 17.67 |
| | | | F | 42.10 | 3.25 | 16.87 |
| 132 | oil | $C_{15}H_{16}Cl_2F_2N_4O_4$ | C | 42.37 | 3.79 | 13.18 |
| | | | F | 42.97 | 4.10 | 10.89 |
| 133 | 120—123 | $C_{13}H_{10}ClF_2IN_4O_2$ | C | 34.12 | 2.20 | 12.24 |
| | | | F | 34.96 | 2.35 | 11.78 |
| 134 | oil | $C_{15}H_{14}Cl_2F_2O_4N_4$ | C | 39.06 | 3.06 | 12.14 |
| | | | F | 39.43 | 2.99 | 11.26 |
| 135 | oil | $C_{16}H_{17}Cl_2F_3N_4O_4$ | C | 42.03 | 3.75 | 12.25 |
| | | | F | 41.90 | 3.59 | 11.60 |

TABLE 2 (continued)
Characterizing Data

| Compound No. | mp(°C) | Empirical Formula | | Elemental Analysis C | H | N |
|---|---|---|---|---|---|---|
| 136 | oil | $C_{13}H_{12}Cl_2FN_5O_2$ | C | 43.35 | 3.36 | 19.45 |
| | | | F | 43.42 | 3.14 | 18.30 |
| 137 | oil | $C_{17}H_{21}ClF_2N_4O_4$ | C | 48.75 | 5.06 | 13.38 |
| | | | F | 48.55 | 5.01 | 12.52 |
| 138 | oil | $C_{16}H_{19}ClF_2N_4O_4$ | C | 47.47 | 4.74 | 13.84 |
| | | | F | 47.73 | 4.69 | 13.48 |
| 139 | oil | $C_{13}H_{12}ClF_2N_5O_2$ | C | 45.42 | 3.52 | 20.37 |
| | | | F | 47.42 | 3.88 | 18.90 |
| 140 | oil | $C_{18}H_{23}ClF_2N_4O_4$ | C | 49.94 | 5.36 | 12.94 |
| | | | F | 50.64 | 5.75 | 12.28 |
| 141 | 44—47 | $C_{13}H_{10}ClF_3N_4O_2$ | C | | | |
| | | | F | | | |
| 142 | oil | $C_{18}H_{23}ClF_2N_4O_4$ | C | 49.94 | 5.36 | 12.94 |
| | | | F | 49.65 | 5.26 | 12.54 |
| 143 | 134—135 | $C_{10}H_{10}ClFN_4OS$ | C | | | |
| | | | F | | | |
| 144 | 83—85 | $C_{10}H_9ClF_2N_4O_2$ | C | | | |
| | | | F | | | |
| 145 | oil | $C_{11}H_8Cl_2F_4N_4O_4S$ | C | 30.08 | 1.84 | 12.76 |
| | | | F | 30.89 | 1.89 | 12.49 |
| 146 | 81—82 | $C_{11}H_{10}Cl_2F_2N_4O_2$ | C | | | |
| | | | F | | | |
| 147 | 77—78 | $C_{10}H_9Cl_2N_5O_3$ | C | | | |
| | | | F | | | |
| 148 | 80—83 | $C_{11}H_{10}Cl_2N_4O_2S$ | C | | | |
| | | | F | | | |
| 149 | 81—83 | $C_{11}H_{10}Cl_2F_2N_4OS$ | C | | | |
| | | | F | | | |
| 150 | 83—84 | $C_{17}H_{14}ClFN_4O_3$ | C | | | |
| | | | F | | | |

TABLE 2 (continued)
Characterizing Data

| Compound No. | mp(°C) | Empirical Formula | | Elemental Analysis C | H | N |
|---|---|---|---|---|---|---|
| 151 | oil | $C_{15}H_{17}Cl_2N_3O_2S$ | C | | | |
| | | | F | | | |
| 152 | oil | $C_{16}H_{17}ClF_4N_4O_4$ | C | 43.60 | 3.88 | 12.70 |
| | | | F | 44.72 | 3.90 | 12.08 |
| 153 | oil | $C_{20}H_{27}ClF_2N_4O_4$ | C | 52.11 | 5.91 | 12.16 |
| | | | F | 52.76 | 5.83 | 11.37 |
| 154 | oil | $C_{16}H_{18}ClF_2N_5O_4$ | C | 46.00 | 4.34 | 16.79 |
| | | | F | 46.70 | 4.26 | 15.37 |
| 155 | oil | $C_{17}H_{16}ClFN_4O_2$ | C | | | |
| | | | F | | | |
| 156 | oil | $C_{13}H_{14}ClFN_4O_2S$ | C | | | |
| | | | F | | | |
| 157 | 93—95 | $C_{15}H_{12}ClFN_4O_2$ | C | | | |
| | | | F | | | |
| 158 | oil | $C_{17}H_{21}ClF_2N_4O_4$ | C | 48.74 | 5.05 | 13.38 |
| | | | F | 48.57 | 4.76 | 13.10 |
| 159 | oil | $C_{13}H_{12}ClFN_4O_2$ | C | | | |
| | | | F | | | |
| 160 | oil | $C_{13}H_{14}ClF_2N_5O_3$ | C | | | |
| | | | F | | | |
| 161 | 137—138 | $C_{11}H_8ClFN_4O_2$ | C | 46.74 | 2.85 | 19.82 |
| | | | F | 47.14 | 2.93 | 19.77 |
| 162 | oil | $C_{18}H_{18}ClN_4O_2$ | C | | | |
| | | | F | | | |
| 163 | 52—54 | $C_{14}H_{16}Cl_2N_4O_3$ | C | | | |
| | | | F | | | |
| 164 | oil | $C_{18}H_{21}ClF_2N_4O_4$ | C | 50.18 | 4.91 | 13.00 |
| | | | F | 52.44 | 5.27 | 11.26 |
| 165 | oil | $C_{17}H_{21}ClF_2N_4O_4$ | C | 48.74 | 5.05 | 13.38 |
| | | | F | 49.10 | 5.26 | 13.20 |

TABLE 2 (continued)

Characterizing Data

| Compound No. | mp(°C) | Empirical Formula | | Elemental Analysis C | H | N |
|---|---|---|---|---|---|---|
| 166 | oil | $C_{17}H_{17}ClF_2N_4O_4$ | C | 49.22 | 4.13 | 13.51 |
| | | | F | 49.49 | 4.02 | 13.19 |
| 167 | oil | $C_{18}H_{19}ClF_2N_4O_4$ | C | 50.41 | 4.47 | 13.07 |
| | | | F | 53.62 | 4.68 | 10.98 |
| 168 | 58—59 | $C_{14}H_{14}ClFN_4O_2$ | C | 51.78 | 4.35 | 17.25 |
| | | | F | 51.91 | 4.41 | 17.22 |
| 169 | 78—79 | $C_{14}H_{13}ClF_2N_4O_2$ | C | | | |
| | | | F | | | |
| 170 | 80—81 | $C_{14}H_{14}Cl_2N_4O_2$ | C | 49.28 | 4.14 | 16.42 |
| | | | F | 49.45 | 4.19 | 16.59 |
| 171 | oil | $C_{17}H_{19}ClF_2N_4O_4$ | C | 48.98 | 4.60 | 13.44 |
| | | | F | 49.38 | 4.51 | 12.71 |
| 172 | oil | $C_{13}H_{14}ClF_3N_4OS$ | C | | | |
| | | | F | | | |
| 173 | oil | $C_{13}H_{11}ClF_2N_4O_2$ | C | 47.50 | 3.37 | 17.05 |
| | | | F | 47.80 | 3.35 | 16.32 |
| 174 | 86—87 | $C_{13}H_{10}ClF_3N_4OS$ | C | 43.04 | 2.78 | 15.45 |
| | | | F | 43.71 | 2.98 | 15.56 |
| 175 | oil | $C_{16}H_{18}ClF_3N_4O_3S$ | C | 43.78 | 4.14 | 12.77 |
| | | | F | 44.19 | 4.07 | 12.07 |
| 176 | 154—156 | $C_{17}H_{14}ClFN_4O_3$ | C | 54.19 | 3.75 | 14.87 |
| | | | F | 55.15 | 3.75 | 13.83 |
| 177 | 95—97 | $C_{10}H_8ClF_3N_4OS$ | C | | | |
| | | | F | | | |
| 178 | oil | $C_{17}H_{15}ClF_2N_4O_2$ | C | | | |
| | | | F | | | |
| 179 | 122—123 | $C_{10}H_8ClFN_3O_4$ | C | | | |
| | | | F | | | |
| 180 | 68—70 | $C_{18}H_{18}ClFN_4O_2$ | C | | | |
| | | | F | | | |

TABLE 2 (continued)

Characterizing Data

| Compound No. | mp(°C) | Empirical Formula | | Elemental Analysis C | H | N |
|---|---|---|---|---|---|---|
| 181 | 102—105 | $C_{11}H_{12}ClFN_4O_2$ | C | | | |
| | | | F | | | |
| 182 | oil | $C_{13}H_{13}ClF_2N_4O_4$ | C | | | |
| | | | F | | | |
| 183 | oil | $C_{18}H_{23}ClF_2N_4O_6$ | C | 46.51 | 4.98 | 12.05 |
| | | | F | 48.03 | 5.09 | 12.02 |
| 184 | oil | $C_{16}H_{19}ClF_2N_4O_4$ | C | 47.47 | 4.92 | 13.84 |
| | | | F | 47.46 | 4.77 | 13.65 |
| 185 | oil | $C_{14}H_{14}ClFN_4O_2$ | C | 51.78 | 4.35 | 17.25 |
| | | | F | 50.79 | 4.39 | 16.55 |
| 186 | 157—158 | $C_7H_3ClFN_5O_3$ | C | | | |
| | | | F | | | |
| 187 | 62—63 | $C_{10}H_9ClF_2N_4O$ | C | | | |
| | | | F | | | |
| 188 | oil | $C_{17}H_{18}ClF_2N_5O_4$ | C | 47.50 | 4.22 | 16.29 |
| | | | F | 47.57 | 4.05 | 15.48 |
| 189 | 80—81 | $C_{10}H_8ClF_2N_5O_3$ | C | | | |
| | | | F | | | |
| 190 | oil | $C_{12}H_{13}ClF_2N_4O_3$ | C | | | |
| | | | F | | | |
| 191 | oil | $C_{15}H_{19}ClF_2N_6O_2$ | C | 46.33 | 4.93 | 21.61 |
| | | | F | 46.59 | 4.85 | 21.32 |
| 192 | 132—134 | $C_{15}H_{19}ClF_2N_6O_2$ | C | 46.33 | 4.93 | 21.61 |
| | | | F | 46.43 | 4.85 | 21.45 |
| 193 | 115—116 | $C_{12}H_{10}ClFN_4O_3$ | C | 46.09 | 3.22 | 17.92 |
| | | | F | 46.64 | 3.10 | 17.68 |
| 194 | oil | $C_{18}H_{16}ClF_2N_5O_3$ | C | | | |
| | | | F | | | |
| 195 | 92—94 | $C_{12}H_{14}ClF_2N_5O_5S_2$ | C | | | |
| | | | F | | | |

TABLE 2 (continued)

Characterizing Data

| Compound No. | mp(°C) | Empirical Formula | | Elemental Analysis C | H | N |
|---|---|---|---|---|---|---|
| 196 | 130—131 | $C_9H_8ClFN_4O_3$ | C | | | |
| | | | F | | | |
| 197 | oil | $C_{16}H_{14}ClFN_4O_3$ | C | | | |
| | | | F | | | |
| 198 | 67—68 | $C_{16}H_{14}ClFN_4O_2S$ | C | | | |
| | | | F | | | |
| 199 | oil | $C_{19}H_{22}ClF_2N_5O_3$ | C | 51.64 | 5.01 | 15.84 |
| | | | F | 49.12 | 4.55 | 14.44 |
| 200 | 113—115 | $C_{14}H_{13}ClF_2N_6O_2$ | C | | | |
| | | | F | | | |
| 201 | oil | $C_{14}H_{16}ClF_2N_5O_2$ | C | | | |
| | | | F | | | |
| 202 | 112—113 | $C_{12}H_{12}ClF_2N_5O_2$ | C | | | |
| | | | F | | | |
| 203 | oil | $C_{14}H_{17}ClF_2N_4O_3$ | C | 46.35 | 4.72 | 15.45 |
| | | | F | 46.65 | 4.65 | 15.30 |
| 204 | oil | $C_{13}H_{15}ClF_2N_4O_2$ | C | 46.92 | 4.54 | 16.84 |
| | | | F | 47.10 | 4.23 | 16.50 |
| 205 | oil | $C_{14}H_{17}ClF_2N_4O_3$ | C | 46.35 | 4.72 | 15.44 |
| | | | F | 46.25 | 4.36 | 15.15 |
| 206 | 75—76 | $C_{13}H_{11}BrF_2N_4O_2$ | C | 41.84 | 2.96 | 15.01 |
| | | | F | 42.03 | 2.89 | 14.70 |
| 207 | 81—84 | $C_{10}H_{10}ClF_2N_5O$ | C | | | |
| | | | F | | | |
| 208 | 66—67 | $C_{12}H_{13}ClF_2N_4O_2S$ | C | | | |
| | | | F | | | |
| 209 | oil | $C_{18}H_{19}BrF_2N_4O_2$ | C | 45.68 | 4.05 | 11.84 |
| | | | F | 45.98 | 4.20 | 11.37 |
| 210 | 121—122 | $C_{10}H_9BrF_2N_4O_2$ | C | | | |
| | | | F | | | |

TABLE 2 (continued)
Characterizing Data

| Compound No. | mp(°C) | Empirical Formula | | Elemental Analysis C | H | N |
|---|---|---|---|---|---|---|
| 211 | 171—172 | $C_{14}H_{10}BrFN_4O_2$ | C | | | |
| | | | F | | | |
| 212 | 84—86 | $C_{17}H_{15}BrF_2N_4O_2$ | C | | | |
| | | | F | | | |
| 213 | 75—76 | $C_{15}H_9ClF_4N_4O_2$ | C | | | |
| | | | F | | | |
| 214 | 111—112 | $C_{16}H_{11}ClFN_5O_2$ | C | | | |
| | | | F | | | |
| 215 | 44—45 | $C_{11}H_{11}ClF_2N_4O$ | C | | | |
| | | | F | | | |
| 216 | oil | $C_{12}H_{13}ClF_2N_4O_3S$ | C | 39.30 | 3.57 | 15.28 |
| | | | F | 40.07 | 3.89 | 13.94 |
| 217 | oil | $C_{10}H_8F_3N_5O_3$ | C | 39.61 | 2.66 | 23.10 |
| | | | F | 39.55 | 3.09 | 21.27 |
| 218 | oil | $C_{14}H_{15}ClF_2N_4O_3$ | C | 46.60 | 4.19 | 15.53 |
| | | | F | 46.30 | 3.97 | 14.91 |
| 219 | 69—70 | $C_{13}H_{15}ClF_2N_4O_3$ | C | 44.77 | 4.34 | 16.07 |
| | | | F | 44.78 | 4.24 | 16.08 |
| 220 | oil | $C_{13}H_{11}ClF_2N_5O_4$ | C | 46.02 | 3.27 | 20.64 |
| | | | F | 45.59 | 3.45 | 20.85 |
| 221 | oil | $C_{14}H_{15}ClF_2N_4O_3$ | C | 46.61 | 4.19 | 15.53 |
| | | | F | 46.41 | 4.15 | 15.31 |
| 222 | 100—106 | $C_{14}H_{16}ClF_2N_5O_3$ | C | 44.63 | 4.28 | 18.59 |
| | | | F | 44.51 | 4.38 | 17.70 |
| 223 | 81—82 | $C_{14}H_{17}Cl_2N_5O_4$ | C | 44.71 | 4.19 | 18.72 |
| | | | F | 44.93 | 4.58 | 18.72 |
| 224 | oil | $C_{12}H_{13}BrF_2N_4O_3$ | C | 38.01 | 3.46 | 14.77 |
| | | | F | 37.76 | 3.30 | 14.34 |
| 225 | 78—80 | $C_{14}H_{13}BrF_2N_4O_2$ | C | | | |
| | | | F | | | |
| 226 | oil | $C_{10}H_9F_3N_4O$ | C | | | |
| | | | F | | | |

TABLE 3
Preemergence Herbicidal Activity

| Compound No. | 1 | 2 | 3 | 4 | 5 | 6 | 7 | 8 | 9 | 10 | 11 | 12 |
|---|---|---|---|---|---|---|---|---|---|---|---|---|
| Rate (kg/ha) | 4.0 | 4.0 | 4.0 | 4.0 | 4.0 | 4.0 | 4.0 | 4.0 | 4.0 | 4.0 | 0.25 | 4.0 |
| Species | % K | % K | % K | % K | % K | % K | % K | % K | % K | % K | % K | % K |
| Cotton | 0 | 0 | 0 | 0 | 0 | 0 | 0 | 0 | 0 | 0 | 0 | 0 |
| Soybean | 0 | 0 | 0 | 0 | 0 | 0 | 0 | 100 | 60 | 50 | 0 | 0 |
| Field Corn | — | — | — | 0 | 0 | — | — | 0 | — | — | 0 | 0 |
| Rice | 0 | 0 | 0 | 0 | 0 | 0 | 10 | 100 | 100 | 90 | 30 | — |
| Wheat | 0 | 0 | 0 | 0 | 0 | 0 | 20 | 100 | 0 | 100 | 20 | — |
| Field Bindweed | — | — | 0 | 0 | — | — | — | 100 | — | — | 0 | 50 |
| Morningglory | 0 | 0 | 0 | 0 | 0 | 0 | 0 | 0 | 0 | 0 | 0 | 0 |
| Velvetleaf | 100 | 100 | 100 | 80 | 80 | 90 | 100 | 100 | 100 | 100 | 100 | 100 |
| Barnyardgrass | 0 | 0 | 0 | 25 | 0 | 0 | 70 | 100 | 20 | 100 | 80 | 100 |
| Green Foxtail | 90 | 95 | 90 | 85 | 60 | 100 | 100 | 100 | 100 | 100 | 95 | 100 |
| Johnsongrass | 0 | 0 | 0 | 90 | 40 | 0 | 90 | 100 | 95 | 100 | 50 | 100 |
| Yellow Nutsedge | — | — | — | 0 | 0 | — | — | 20 | — | — | 0 | 0 |

TABLE 3 (Continued)

| Compound No. | 13 | 14 | 15 | 16 | 17 | 18 | 19 | 20 | 21 | 22 | 23 | 24 |
|---|---|---|---|---|---|---|---|---|---|---|---|---|
| Rate (kg/ha) | 4.0 | 4.0 | 4.0 | 4.0 | 4.0 | 4.0 | 4.0 | 4.0 | 4.0 | 4.0 | 4.0 | 4.0 |
| Species | % K | % K | % K | % K | % K | % K | % K | % K | % K | % K | % K | % K |
| Cotton | — | 0 | 0 | 0 | 0 | 0 | 40 | 0 | 0 | 0 | 0 | 0 |
| Soybean | — | 0 | 0 | 0 | 0 | 0 | 80 | 0 | 0 | 0 | 0 | 60 |
| Field Corn | — | 0 | 100 | — | — | 60 | 100 | — | — | — | — | — |
| Rice | 30 | 0 | 0 | — | 0 | 0 | 60 | 30 | 20 | 95 | 0 | 95 |
| Wheat | 20 | 100 | 100 | — | 0 | 90 | 100 | 0 | 30 | 85 | 0 | 80 |
| Field Bindweed | — | 0 | 0 | — | — | 0 | 0 | — | — | — | — | — |
| Morningglory | — | 0 | 0 | 0 | 0 | 0 | 40 | 0 | 0 | 20 | 0 | 0 |
| Velvetleaf | 100 | 0 | 100 | 100 | 100 | 100 | 100 | 100 | 100 | 100 | 75 | 100 |
| Barnyardgrass | 95 | 0 | 80 | 0 | 0 | 0 | 100 | 0 | 0 | 0 | 0 | 100 |
| Green Foxtail | 100 | 0 | 100 | 100 | 90 | 100 | 100 | 100 | 100 | 100 | 95 | 100 |
| Johnsongrass | 100 | 0 | 90 | 95 | 60 | 40 | 90 | 95 | 85 | 10 | 40 | 100 |
| Yellow Nutsedge | 100 | 0 | 0 | — | — | 0 | 50 | — | — | — | — | — |

TABLE 3 (Continued)

| Compound No. | 25 | 26 | 27 | 28 | 29 | 30 | 31 | 32 | 33 | 34 | 35 | 36 |
|---|---|---|---|---|---|---|---|---|---|---|---|---|
| Rate (kg/ha) | 4.0 | 4.0 | 4.0 | 4.0 | 4.0 | 4.0 | 4.0 | 4.0 | 4.0 | 4.0 | 4.0 | 4.0 |
| Species | % K | % K | % K | % K | % K | % K | % K | % K | % K | % K | % K | % K |
| Cotton | 20 | 0 | 0 | 0 | 0 | 50 | 0 | 0 | 0 | 0 | 30 | 0 |
| Soybean | 10 | 0 | 0 | 0 | 0 | 0 | 20 | 0 | 0 | 0 | 30 | 20 |
| Field Corn | — | — | 75 | — | — | — | 0 | 60 | — | 40 | 0 | — |
| Rice | 0 | 20 | 0 | 0 | 0 | 0 | 10 | 0 | 20 | 0 | 100 | 0 |
| Wheat | 10 | 70 | 20 | 0 | 0 | 0 | 100 | 90 | 20 | 50 | 95 | 40 |
| Field Bindweed | — | — | 0 | — | — | — | 0 | 0 | — | 0 | 20 | — |
| Morningglory | 0 | 0 | 0 | 0 | 0 | 70 | 0 | 0 | 100 | 0 | 0 | 0 |
| Velvetleaf | 90 | 100 | 100 | 80 | 80 | 90 | 100 | 80 | 100 | 100 | 100 | 100 |
| Barnyardgrass | 0 | 90 | 100 | 0 | 0 | 60 | 90 | 90 | 60 | 80 | 100 | 100 |
| Green Foxtail | 90 | 100 | 100 | 100 | 95 | 100 | 100 | 100 | 100 | 100 | 95 | 100 |
| Johnsongrass | 20 | 70 | 100 | 0 | 0 | 50 | 90 | 90 | 100 | 95 | 30 | 100 |
| Yellow Nutsedge | — | — | 0 | — | — | — | 0 | 30 | — | 30 | 100 | — |

TABLE 3 (Continued)

| Compound No. | 37 | 38 | 39 | 40 | 41 | 42 | 43 | 44 | 45 | 46 | 47 | 48 | 49 |
|---|---|---|---|---|---|---|---|---|---|---|---|---|---|
| Rate (kg/ha) | 4.0 | 4.0 | 4.0 | 4.0 | 4.0 | 0.25 | 0.25 | 4.0 | 0.25 | 0.25 | 4.0 | 0.25 | 0.25 |
| Species | % K | % K | % K | % K | % K | % K | % K | % K | % K | % K | % K | % K | % K |
| Cotton | 0 | 0 | 0 | 0 | 0 | 0 | 0 | 0 | 0 | 0 | 0 | 0 | 0 |
| Soybean | 0 | 0 | 0 | 0 | 0 | 0 | 0 | 30 | 0 | 0 | 0 | 0 | 0 |
| Field Corn | — | 0 | — | 0 | 60 | 0 | 30 | 30 | 0 | 50 | 30 | 30 | — |
| Rice | 0 | 0 | 20 | 0 | 90 | 0 | 30 | 100 | — | 40 | 100 | 90 | 0 |
| Wheat | 0 | 0 | 0 | 70 | 100 | 0 | 0 | 30 | — | 0 | 100 | 80 | 0 |
| Field Bindweed | — | 100 | — | 20 | 70 | 0 | 0 | 100 | 0 | 40 | 90 | 80 | — |
| Morningglory | 0 | 50 | 0 | 0 | 0 | 0 | 0 | 0 | 0 | 0 | 0 | 0 | 0 |
| Velvetleaf | 90 | 0 | 100 | 90 | 100 | 100 | 100 | 100 | 100 | 100 | 100 | 100 | 100 |
| Barnyardgrass | 40 | 0 | 40 | 90 | 100 | 0 | 80 | 100 | 20 | 50 | 100 | 100 | 60 |
| Green Foxtail | 100 | 50 | 100 | 100 | 100 | 90 | 95 | 100 | 95 | 100 | 100 | 100 | 100 |
| Johnsongrass | 20 | 0 | 95 | 60 | 100 | 90 | 30 | 95 | 50 | 100 | 100 | 100 | 30 |
| Yellow Nutsedge | 0 | 0 | — | 0 | 0 | 0 | 0 | 10 | 0 | 20 | 90 | 0 | — |

TABLE 3 (Continued)

| Compound No. | 50 | 51 | 52 | 53 | 54 | 55 | 56 | 57 | 58 | 59 | 60 | 61 |
|---|---|---|---|---|---|---|---|---|---|---|---|---|
| Rate (kg/ha) | 0.25 | 0.25 | 4.0 | 4.0 | 2.0 | 4.0 | 0.25 | 4.0 | 4.0 | 4.0 | 4.0 | 4.0 |
| Species | % K | % K | % K | % K | % K | % K | % K | % K | % K | % K | % K | % K |
| Cotton | 0 | 0 | 0 | 0 | 0 | 0 | 0 | 0 | 0 | 20 | 0 | 0 |
| Soybean | 10 | 0 | 20 | 0 | 0 | 70 | 0 | 0 | 0 | 30 | 20 | 0 |
| Field Corn | 0 | 0 | 30 | 60 | 100 | 30 | 0 | 0 | 0 | 30 | 0 | 90 |
| Rice | 0 | 0 | 0 | 10 | 50 | 10 | 0 | 0 | 30 | 90 | 0 | 90 |
| Wheat | 0 | 0 | 100 | 0 | 90 | 10 | 0 | 0 | 0 | 80 | 0 | 20 |
| Field Bindweed | 0 | 0 | 70 | 0 | 85 | 60 | 0 | 0 | 20 | 100 | 0 | 50 |
| Morningglory | 0 | 0 | 20 | 20 | 10 | 50 | 0 | 0 | 0 | 90 | 0 | 0 |
| Velvetleaf | 95 | 100 | 100 | 100 | 100 | 100 | 100 | 100 | 100 | 100 | 100 | 100 |
| Barnyardgrass | 90 | 40 | 0 | 80 | 100 | 95 | 100 | 0 | 100 | 90 | 50 | 100 |
| Green Foxtail | 100 | 50 | 100 | 100 | 100 | 100 | 100 | 70 | 100 | 100 | 100 | 100 |
| Johnsongrass | 10 | 100 | 30 | 100 | 100 | 100 | 100 | 20 | 90 | 100 | 100 | 100 |
| Yellow Nutsedge | 0 | 0 | 0 | 0 | 10 | 0 | 0 | 0 | 0 | 40 | 0 | 30 |

TABLE 3 (Continued)

| Compound No. | 62 | 63 | 64 | 65 | 66 | 67 | 68 | 69 | 70 | 71 | 72 | 73 |
|---|---|---|---|---|---|---|---|---|---|---|---|---|
| Rate (kg/ha) | 4.0 | 4.0 | 4.0 | 4.0 | 4.0 | 4.0 | 0.25 | 0.25 | 4.0 | 4.0 | 4.0 | 4.0 |
| Species | % K | % K | % K | % K | % K | % K | % K | % K | % K | % K | % K | % K |
| Cotton | 0 | 0 | 0 | 0 | 0 | 0 | 0 | 0 | 0 | 0 | 0 | 0 |
| Soybean | 0 | 0 | 0 | 0 | 0 | 60 | 0 | 0 | 90 | 0 | 0 | 0 |
| Field Corn | 0 | 0 | — | 0 | 75 | 0 | 0 | 60 | — | 0 | 0 | 0 |
| Rice | 0 | — | 80 | 0 | 90 | — | 0 | 90 | 90 | 0 | 0 | — |
| Wheat | 0 | — | 100 | 0 | 100 | — | 0 | 60 | 100 | 0 | 0 | — |
| Field Bindweed | 0 | 0 | — | 0 | 0 | 100 | 0 | 80 | — | 0 | 0 | 100 |
| Morningglory | 0 | 30 | 20 | 20 | 0 | 20 | 0 | 0 | 0 | 0 | 0 | 20 |
| Velvetleaf | 100 | 20 | 100 | 100 | 100 | 100 | 100 | 100 | 100 | 20 | 80 | 100 |
| Barnyardgrass | 10 | 0 | 60 | 90 | 100 | 95 | 90 | 100 | 100 | 95 | 0 | 40 |
| Green Foxtail | 95 | 30 | 100 | 100 | 100 | 100 | 90 | 100 | 100 | 100 | 95 | 100 |
| Johnsongrass | 80 | 0 | 95 | 100 | 100 | 80 | 30 | 90 | 100 | 90 | 0 | 40 |
| Yellow Nutsedge | 0 | 0 | — | 0 | 75 | 40 | 0 | 0 | — | 0 | 0 | 0 |

TABLE 3 (Continued)

| Compound No. | 74 | 75 | 76 | 77 | 78 | 79 | 80 | 81 | 82 | 83 | 84 | 85 |
|---|---|---|---|---|---|---|---|---|---|---|---|---|
| Rate (kg/ha) | 4.0 | 4.0 | 4.0 | 4.0 | 4.0 | 4.0 | 4.0 | 4.0 | 4.0 | 4.0 | 4.0 | 4.0 |
| Species | % K | % K | % K | % K | % K | % K | % K | % K | % K | % K | % K | % K |
| Cotton | 0 | 0 | 0 | 0 | 0 | 0 | 0 | 0 | 0 | 50 | 0 | 0 |
| Soybean | 0 | 0 | 0 | 0 | 0 | 0 | 0 | 0 | 0 | 20 | 0 | 40 |
| Field Corn | — | — | 0 | — | — | 0 | 75 | — | 0 | 0 | — | — |
| Rice | 0 | 0 | 0 | 0 | 95 | 0 | 0 | 0 | 0 | 0 | 0 | 25 |
| Wheat | 60 | 0 | 0 | 30 | 100 | 0 | 0 | 0 | 0 | 0 | 0 | 10 |
| Field Bindweed | — | — | 0 | — | — | 0 | 0 | — | 0 | 80 | — | — |
| Morningglory | 30 | 100 | 0 | 0 | 20 | 0 | 0 | 0 | 0 | 60 | 20 | 100 |
| Velvetleaf | 100 | 80 | 0 | 100 | 100 | 0 | 100 | 100 | 40 | 100 | 0 | 100 |
| Barnyardgrass | 0 | 0 | 0 | 0 | 95 | 0 | 25 | 0 | 0 | 0 | 0 | 65 |
| Green Foxtail | 100 | 40 | 0 | 95 | 100 | 0 | 100 | 0 | 0 | 70 | 0 | 95 |
| Johnsongrass | 10 | 90 | 0 | 0 | 50 | 0 | 100 | 0 | 0 | 0 | 0 | 100 |
| Yellow Nutsedge | — | — | 0 | — | — | 0 | — | — | 0 | 0 | — | — |

TABLE 3 (Continued)

| Compound No. | 86 | 87 | 88 | 89 | 90 | 91 | 92 | 93 | 94 | 95 | 96 | 97 |
|---|---|---|---|---|---|---|---|---|---|---|---|---|
| Rate (kg/ha) | 4.0 | 4.0 | 4.0 | 4.0 | 4.0 | 4.0 | 4.0 | 4.0 | 4.0 | 4.0 | 4.0 | 4.0 |
| Species | % K | % K | % K | % K | % K | % K | % K | % K | % K | % K | % K | % K |
| Cotton | 0 | 0 | 0 | 0 | 0 | 0 | 0 | 0 | 0 | 0 | 0 | 0 |
| Soybean | 0 | 0 | 0 | 0 | 0 | 0 | 0 | 0 | 0 | 0 | 0 | 60 |
| Field Corn | — | — | — | — | — | — | — | — | 80 | 0 | 0 | 0 |
| Rice | 0 | 0 | 0 | 0 | 30 | 10 | 20 | 0 | 0 | 20 | 10 | 50 |
| Wheat | 60 | 30 | 0 | 0 | 0 | 0 | 40 | 0 | 40 | 0 | 0 | 30 |
| Field Bindweed | — | — | — | — | — | — | — | — | 0 | 0 | 0 | 60 |
| Morningglory | 70 | 70 | 0 | 0 | 0 | 0 | 0 | 0 | 0 | 0 | 0 | 70 |
| Velvetleaf | 100 | 100 | 100 | 100 | 90 | 30 | 100 | 100 | 100 | 100 | 100 | 100 |
| Barnyardgrass | 50 | 100 | 100 | 0 | 0 | 90 | 100 | 0 | 50 | 50 | 0 | 100 |
| Green Foxtail | 100 | 100 | 100 | 90 | 0 | 100 | 100 | 30 | 100 | 100 | 100 | 100 |
| Johnsongrass | 100 | 100 | 95 | 0 | 10 | 80 | 100 | 0 | 60 | 90 | 0 | 100 |
| Yellow Nutsedge | — | — | — | — | — | — | — | — | 0 | 0 | 0 | 0 |

TABLE 3 (continued)

| Compound No. | 98 | 99 | 100 | 101 | 102 | 103 | 104 | 105 | 106 | 107 | 108 | 109 |
|---|---|---|---|---|---|---|---|---|---|---|---|---|
| Rate (kg/ha) | 2.0 | 2.0 | 4.0 | 8.0 | 4.0 | 4.0 | 4.0 | 4.0 | 4.0 | 2.0 | 4.0 | 8.0 |
| Species | % K | % K | % K | % K | % K | % K | % K | % K | % K | % K | % K | % K |
| Cotton | 0 | 0 | 0 | 0 | 0 | 0 | 0 | 0 | 0 | 0 | 0 | — |
| Soybean | 20 | 20 | 0 | 0 | 0 | 0 | 0 | 0 | 0 | 0 | 0 | 0 |
| Field Corn | 0 | 35 | 0 | 0 | 0 | 0 | 0 | 0 | 0 | 0 | 0 | 0 |
| Rice | 10 | 75 | 0 | 0 | 0 | 0 | 0 | 0 | 0 | 0 | 0 | — |
| Wheat | 10 | 90 | 0 | 0 | 0 | 0 | 0 | 0 | 0 | 0 | 0 | 0 |
| Field Bindweed | 10 | 0 | 0 | 0 | 0 | 0 | 0 | 0 | 0 | 30 | 0 | 0 |
| Morningglory | 0 | 0 | 0 | 0 | 0 | 0 | 0 | 0 | 0 | 0 | 0 | 0 |
| Velvetleaf | 10 | 80 | 60 | 50 | 0 | 0 | 90 | 95 | 100 | 0 | 0 | 100 |
| Barnyardgrass | 95 | 70 | 0 | 0 | 0 | 0 | 0 | 0 | 0 | 0 | 0 | 100 |
| Green Foxtail | 100 | 100 | 50 | 0 | 0 | 75 | 90 | 0 | 80 | 0 | 100 | 100 |
| Johnsongrass | 60 | 50 | 60 | 0 | 0 | 0 | 0 | 0 | 0 | 30 | 0 | 95 |
| Yellow Nutsedge | 0 | 0 | 0 | 0 | 0 | 0 | 0 | 0 | 0 | 0 | 0 | 0 |

TABLE 3 (Continued)

| Compound No. | 110 | 111 | 112 | 113 | 114 | 115 | 116 | 117 | 118 | 119 | 120 | 121 |
|---|---|---|---|---|---|---|---|---|---|---|---|---|
| Rate (kg/ha) | 8.0 | 8.0 | 8.0 | 8.0 | 8.0 | 4.0 | 2.0 | 4.0 | 8.0 | 8.0 | 8.0 | 2.0 |
| Species | % K | % K | % K | % K | % K | % K | % K | % K | % K | % K | % K | % C |
| Cotton | — | — | — | — | — | 100 | 100 | 0 | 100 | 0 | 0 | 100 |
| Soybean | 30 | 0 | 0 | 0 | 0 | 100 | 100 | 100 | 100 | 0 | 0 | 100 |
| Field Corn | 30 | 100 | 0 | 0 | 0 | 100 | 100 | 100 | 100 | 0 | 30 | 100 |
| Rice | — | — | — | — | — | 100 | 100 | 90 | — | — | — | 100 |
| Wheat | 0 | 0 | 0 | 0 | 0 | 100 | 100 | 100 | 100 | 0 | 0 | 100 |
| Field Bindweed | 0 | 0 | 0 | 0 | 0 | 100 | 100 | 70 | 100 | 0 | 0 | 100 |
| Morningglory | 0 | — | — | — | — | 100 | 100 | 70 | 100 | 0 | 0 | 100 |
| Velvetleaf | 100 | 100 | 50 | 40 | 90 | 100 | 100 | 100 | 100 | 0 | 0 | — |
| Barnyardgrass | 20 | 100 | 0 | 0 | 0 | 100 | 100 | 100 | 100 | 0 | 0 | 100 |
| Green Foxtail | 100 | 95 | 80 | 10 | 0 | 100 | 100 | — | 100 | 0 | 0 | 100 |
| Johnsongrass | 40 | — | 0 | — | — | 100 | 100 | 100 | 100 | 0 | 0 | 100 |
| Yellow Nutsedge | — | — | — | — | — | 100 | 100 | 20 | — | — | — | 100 |

TABLE 3 (Continued)

| Compound No. | 122 | 123 | 124 | 125 | 126 | 127 | 128 | 129 | 130 | 131 | 132 | 133 |
|---|---|---|---|---|---|---|---|---|---|---|---|---|
| Rate (kg/ha) | 8.0 | 8.0 | 4.0 | 1.0 | 4.0 | 2.0 | 2.0 | 2.0 | 4.0 | 8.0 | 2.0 | 2.0 |
| Species | % C | % C | % C | % C | % C | % C | % C | % C | % C | % C | % C | % C |
| Cotton | 0 | 0 | 40 | 80 | 60 | 10 | 100 | 30 | 100 | 100 | 50 | 100 |
| Soybean | 0 | 0 | 10 | 100 | 0 | 10 | 10 | 20 | 10 | 70 | 20 | 100 |
| Field Corn | 0 | 0 | 0 | 90 | 30 | 10 | 30 | 100 | 50 | 30 | 40 | 100 |
| Rice | 0 | — | 40 | 80 | 40 | 20 | 90 | 70 | 80 | — | 70 | 100 |
| Wheat | 0 | 0 | 20 | 80 | 40 | '0 | 90 | 70 | 80 | 40 | 60 | 100 |
| Field Bindweed | 0 | 0 | 60 | 70 | 80 | 10 | 100 | 40 | 100 | 100 | 100 | 100 |
| Morningglory | 0 | 0 | 100 | 100 | 100 | 30 | 100 | 20 | 100 | 100 | 90 | 100 |
| Velvetleaf | 0 | 0 | 100 | 100 | 100 | 10 | 100 | 100 | 100 | 60 | 100 | 100 |
| Barnyardgrass | 30 | 0 | 90 | 100 | 80 | 20 | 100 | 100 | 100 | 0 | 90 | 100 |
| Green Foxtail | 40 | 0 | 100 | 100 | 100 | 70 | 100 | 100 | 100 | 100 | 100 | 100 |
| Johnsongrass | 30 | 0 | 70 | 100 | 100 | 10 | 100 | 90 | 100 | 80 | 80 | 100 |
| Yellow Nutsedge | 0 | — | 20 | 80 | 50 | 10 | 90 | 20 | 90 | — | 40 | 100 |

TABLE 3 (continued)

| Compound No. | 134 | 135 | 136 | 137 | 138 | 139 | 140 | 141 | 142 | 143 | 144 | 145 |
|---|---|---|---|---|---|---|---|---|---|---|---|---|
| Rate (kg/ha) | 2.0 | 2.0 | 2.0 | 2.0 | 2.0 | 2.0 | 2.0 | 0.125 | 2.0 | 8.0 | 4.0 | 8.0 |
| Species | % C | % C | % C | % C | % C | % C | % C | % C | % C | % C | % C | % C |
| Cotton | 80 | 40 | 20 | 80 | 100 | 100 | 100 | 30 | 100 | 0 | 60 | 10 |
| Soybean | 20 | 0 | 30 | 20 | 10 | 100 | 10 | 30 | 20 | 0 | 60 | 0 |
| Field Corn | 20 | 0 | 80 | 80 | 70 | 100 | 70 | 80 | 50 | 0 | 90 | 40 |
| Rice | 70 | 40 | 90 | 100 | 100 | 100 | 100 | 80 | 100 | — | 70 | — |
| Wheat | 60 | 40 | 80 | 80 | 100 | 90 | 100 | 20 | 90 | 0 | 100 | 0 |
| Field Bindweed | 100 | 100 | 90 | 100 | 100 | 100 | 100 | 40 | 100 | 0 | 90 | 0 |
| Morningglory | 80 | 80 | 100 | 90 | 100 | 100 | 100 | 50 | 100 | 0 | 50 | 70 |
| Velvetleaf | 80 | 90 | 100 | 100 | 100 | 100 | 100 | 100 | 100 | 0 | 100 | 90 |
| Barnyardgrass | 80 | 50 | 100 | 100 | 100 | 100 | 100 | 100 | 100 | 0 | 90 | 90 |
| Green Foxtail | 100 | 100 | 100 | 100 | 100 | 100 | 100 | 90 | 100 | 0 | 100 | 100 |
| Johnsongrass | 30 | 90 | 100 | 100 | 100 | 100 | 100 | 60 | 100 | 0 | 80 | 90 |
| Yellow Nutsedge | 50 | 10 | 30 | 100 | 100 | 100 | 100 | 50 | 100 | — | 30 | — |

TABLE 3 (Continued)

| Compound No. | 146 | 147 | 148 | 149 | 150 | 151 | 152 | 153 | 154 | 157 | 158 | 159 |
|---|---|---|---|---|---|---|---|---|---|---|---|---|
| Rate (kg/ha) | 8.0 | 8.0 | 8.0 | 8.0 | 8.0 | 8.0 | 1.0 | 1.0 | 2.0 | 8.0 | 8.0 | 8.0 |
| Species | % C | % C | % C | % C | % C | % C | % C | % C | % C | % C | % C | % C |
| Cotton | 50 | 20 | 0 | 40 | 0 | 0 | 100 | 20 | 100 | 20 | 100 | 90 |
| Soybean | 100 | 50 | 0 | 70 | 0 | 0 | 10 | 10 | 20 | 0 | 80 | 100 |
| Field Corn | 90 | 20 | 30 | 90 | 0 | 0 | 10 | 0 | 30 | 0 | 90 | 100 |
| Rice | — | — | — | — | — | — | 90 | 0 | 90 | — | — | — |
| Wheat | 100 | 0 | 0 | 40 | 0 | 0 | 70 | 0 | 90 | 0 | 90 | 100 |
| Field Bindweed | 100 | 0 | 0 | 80 | 0 | 0 | 100 | 90 | 100 | 10 | 100 | 100 |
| Morningglory | 100 | 50 | 0 | 90 | 0 | 0 | 90 | 20 | 100 | 60 | 100 | 100 |
| Velvetleaf | 100 | 90 | 0 | 100 | 0 | 0 | 100 | 90 | 100 | 60 | 100 | 100 |
| Barnyardgrass | 100 | 40 | 0 | 100 | 0 | 0 | 100 | 90 | 100 | 50 | 100 | 100 |
| Green Foxtail | 100 | 50 | 0 | 100 | 0 | 0 | 100 | 100 | 100 | 80 | 100 | 100 |
| Johnsongrass | 100 | 0 | 0 | 100 | 0 | 0 | 90 | 20 | 100 | 20 | 100 | 100 |
| Yellow Nutsedge | — | — | — | — | — | — | 90 | 0 | 90 | — | — | — |

TABLE 3 (Continued)

| Compound No. | 160 | 161 | 162 | 163 | 164 | 165 | 166 | 167 | 168 | 169 | 170 | 171 |
|---|---|---|---|---|---|---|---|---|---|---|---|---|
| Rate (kg/ha) | 1.0 | 8.0 | 8.0 | 8.0 | 0.5 | 1.0 | 8.0 | 8.0 | 1.0 | 0.125 | 1.0 | 1.0 |
| Species | % C | % C | % C | % C | % C | % C | % C | % C | % C | % C | % C | % C |
| Cotton | 90 | 100 | 20 | 30 | 80 | 80 | 100 | 100 | 20 | 40 | 10 | 100 |
| Soybean | 70 | 100 | 10 | 50 | 10 | 10 | 90 | 90 | 50 | 40 | 10 | 60 |
| Field Corn | 90 | 100 | 0 | 90 | 0 | 80 | 90 | 90 | 80 | 90 | 70 | 10 |
| Rice | 90 | — | — | — | 100 | 80 | — | — | 70 | 80 | 30 | 100 |
| Wheat | 90 | 100 | 0 | 50 | 80 | 80 | 100 | 90 | 40 | 80 | 40 | 100 |
| Field Bindweed | 100 | 100 | 20 | 100 | 100 | 60 | 100 | 100 | 10 | 100 | 0 | 100 |
| Morningglory | 100 | 100 | 30 | 70 | 80 | 60 | 100 | 100 | 30 | 80 | 10 | 90 |
| Velvetleaf | 100 | 100 | 30 | 100 | 100 | 100 | 100 | 100 | 100 | 100 | 90 | 100 |
| Barnyardgrass | 100 | 100 | 20 | 100 | 100 | 100 | 100 | 100 | 70 | 100 | 90 | 100 |
| Green Foxtail | 100 | 100 | 80 | 100 | 100 | 100 | 100 | 100 | 100 | 100 | 100 | 100 |
| Johnsongrass | 100 | 100 | 0 | 90 | 90 | 60 | 100 | 100 | 70 | 100 | 90 | 90 |
| Yellow Nutsedge | 90 | — | — | — | 60 | 80 | — | — | 40 | 50 | 10 | 90 |

TABLE 3 (Continued)

| Compound No. | 172 | 173 | 174 | 175 | 176 | 177 | 178 | 179 | 180 | 181 | 182 | 183 |
|---|---|---|---|---|---|---|---|---|---|---|---|---|
| Rate (kg/ha) | 0.5 | 0.125 | 0.5 | 1.0 | 8.0 | 8.0 | 8.0 | 4.0 | 8.0 | 8.0 | 2.0 | 1.0 |
| Species | % C | % C | % C | % C | % C | % C | % C | % C | % C | % C | % C | % C |
| Cotton | 20 | 10 | 20 | 20 | 0 | 70 | 20 | 60 | 0 | 20 | 100 | 100 |
| Soybean | 20 | 40 | 90 | 50 | 0 | 90 | 50 | 20 | 0 | 40 | 20 | 20 |
| Field Corn | 40 | 70 | 60 | 0 | 0 | 90 | 90 | 20 | 0 | 60 | 70 | 20 |
| Rice | 60 | 80 | 70 | 70 | — | — | — | 80 | — | 0 | 100 | 90 |
| Wheat | 20 | 40 | 40 | 90 | 0 | 70 | 70 | 50 | 0 | 30 | 90 | 80 |
| Field Bindweed | 30 | 30 | 100 | 100 | 0 | 40 | 20 | 100 | 0 | 40 | 100 | 100 |
| Morningglory | 10 | 0 | 100 | 60 | 0 | 70 | 80 | 90 | 20 | 10 | 90 | 90 |
| Velvetleaf | 100 | 100 | 100 | 100 | 0 | 100 | 100 | 100 | 10 | 30 | 100 | 100 |
| Barnyardgrass | 90 | 100 | 100 | 80 | 0 | 90 | 90 | 100 | 80 | 80 | 100 | 100 |
| Green Foxtail | 100 | 100 | 100 | 100 | 0 | 90 | 100 | 100 | 90 | 90 | 100 | 100 |
| Johnsongrass | 80 | 90 | 90 | 100 | 0 | 70 | 50 | 90 | 80 | 40 | 100 | 100 |
| Yellow Nutsedge | 40 | 30 | 30 | 70 | — | — | — | 60 | — | — | 100 | 90 |

TABLE 3 (Continued)

| Compound No. | 184 | 185 | 186 | 187 | 188 | 189 | 190 | 191 | 192 | 193 | 194 | 195 |
|---|---|---|---|---|---|---|---|---|---|---|---|---|
| Rate (kg/ha) | 1.0 | 1.0 | 8.0 | 0.5 | 1.0 | 1.0 | 0.25 | 1.0 | 1.0 | 1.0 | 1.0 | 1.0 |
| Species | % C | % C | % C | % C | % C | % C | % C | % C | % C | % C | % C | % C |
| Cotton | 100 | 20 | 0 | 0 | 100 | 10 | 80 | 40 | 10 | 90 | 0 | 100 |
| Soybean | 20 | 20 | 0 | 30 | 0 | 0 | 70 | 30 | 0 | 100 | 60 | 50 |
| Field Corn | 40 | 80 | 0 | 90 | 10 | 0 | 100 | 100 | 10 | 100 | 10 | 100 |
| Rice | 100 | 80 | — | 70 | 90 | 10 | — | 60 | 0 | 100 | 0 | — |
| Wheat | 90 | 70 | 0 | 70 | 70 | 10 | 100 | 90 | 10 | 100 | 10 | 90 |
| Field Bindweed | 100 | 30 | 0 | 30 | 100 | 0 | — | 50 | 0 | 90 | 0 | — |
| Morningglory | 100 | 60 | 0 | 50 | 100 | 10 | 50 | 90 | 0 | 90 | 60 | 100 |
| Velvetleaf | 100 | 100 | 0 | 100 | 100 | 70 | 100 | 100 | 80 | 100 | 60 | 100 |
| Barnyardgrass | 100 | 100 | 0 | 100 | 90 | 40 | 100 | 60 | 0 | 100 | 70 | 100 |
| Green Foxtail | 100 | 100 | 0 | 100 | 100 | 30 | 100 | 90 | 0 | 100 | 0 | 100 |
| Johnsongrass | 100 | 90 | 0 | 90 | 100 | 10 | 100 | 90 | 50 | 100 | 0 | 80 |
| Yellow Nutsedge | 90 | 20 | — | 40 | 90 | 0 | 90 | 60 | 0 | 90 | 0 | 90 |

TABLE 3 (Continued)

| Compound No. | 196 | 197 | 198 | 199 | 200 | 201 | 202 | 203 | 204 | 205 | 206 | 207 |
|---|---|---|---|---|---|---|---|---|---|---|---|---|
| Rate (kg/ha) | 8.0 | 8.0 | 8.0 | 0.5 | 2.0 | 2.0 | 2.0 | 0.5 | 0.5 | 0.5 | 0.25 | 4.0 |
| Species | % C | % C | % C | % C | % C | % C | % C | % C | % C | % C | % C | % C |
| Cotton | 50 | 10 | 10 | 0 | 30 | 90 | 20 | 80 | 40 | 60 | 90 | 50 |
| Soybean | 40 | 0 | 0 | 10 | 50 | 90 | 60 | 70 | 70 | 40 | 90 | 90 |
| Field Corn | 40 | 70 | 50 | 60 | 70 | 100 | 0 | 90 | 80 | 90 | 90 | 20 |
| Rice | — | — | — | 10 | 20 | 90 | 20 | 90 | 90 | 90 | 70 | 20 |
| Wheat | 80 | 70 | 70 | 40 | 20 | 100 | 10 | 90 | 70 | 90 | 60 | 30 |
| Field Bindweed | 60 | 90 | 60 | 50 | 20 | 60 | 10 | 100 | 100 | 100 | 100 | 30 |
| Morningglory | 40 | 60 | 80 | 10 | 10 | 20 | 10 | 100 | 90 | 100 | 90 | 30 |
| Velvetleaf | 100 | 100 | 90 | 90 | 10 | 100 | 900 | 100 | 100 | 100 | 100 | 100 |
| Barnyardgrass | 90 | 100 | 90 | 30 | 10 | 100 | 0 | 100 | 100 | 100 | 100 | 40 |
| Green Foxtail | 90 | 100 | 100 | 70 | 50 | 100 | 0 | 100 | 100 | 100 | 100 | 10 |
| Johnsongrass | 70 | 90 | 90 | 60 | 10 | 70 | 0 | 90 | 100 | 100 | 100 | 40 |
| Yellow Nutsedge | — | — | — | 20 | 10 | 50 | 0 | 70 | 40 | 60 | 60 | 20 |

TABLE 3 (Continued)

| Compound No. | 208 | 209 | 210 | 211 | 212 | 213 | 214 | 215 | 216 | 217 | 218 | 219 |
|---|---|---|---|---|---|---|---|---|---|---|---|---|
| Rate (kg/ha) | 0.5 | 1.0 | 8.0 | 4.0 | 8.0 | 8.0 | 8.0 | 8.0 | 2.0 | 8.0 | 0.5 | 0.5 |
| Species | % C | % C | % C | % C | % C | % C | % C | % C | % C | % C | % C | % C |
| Cotton | 50 | 50 | 0 | 0 | 0 | 0 | 0 | 100 | 100 | 30 | 90 | 80 |
| Soybean | 40 | 20 | 0 | 0 | 0 | 0 | 0 | 100 | 90 | 20 | 80 | 60 |
| Field Corn | 50 | 40 | 100 | 0 | 0 | 0 | 0 | 100 | 100 | 40 | 100 | 100 |
| Rice | 40 | 90 | — | 0 | — | — | — | 80 | 100 | — | 90 | 80 |
| Wheat | 40 | 50 | 0 | 0 | 0 | 0 | 0 | 100 | 100 | 40 | 100 | 90 |
| Field Bindweed | 100 | 100 | 0 | 0 | 0 | 0 | 0 | 100 | 100 | 0 | 100 | 80 |
| Morningglory | 50 | 90 | 0 | 0 | 0 | 0 | 0 | 100 | 100 | 0 | 100 | 100 |
| Velvetleaf | 100 | 100 | 0 | 0 | 0 | 0 | 0 | 100 | 100 | 90 | 100 | 100 |
| Barnyardgrass | 100 | 100 | 0 | 0 | 0 | 0 | 40 | 100 | 100 | 40 | 100 | 100 |
| Green Foxtail | 100 | 100 | 0 | 0 | 0 | 90 | 0 | 100 | 100 | 0 | 100 | 100 |
| Johnsongrass | 90 | 90 | 0 | 0 | 0 | 0 | 60 | 100 | 100 | 70 | 100 | 100 |
| Yellow Nutsedge | 40 | 70 | — | 0 | — | — | — | 100 | 90 | — | 90 | 90 |

TABLE 3 (Continued)

| Compound No. | 220 | 221 | 222 | 223 | 224 | 225 |
|---|---|---|---|---|---|---|
| Rate (kg/ha) | 2.0 | 1.0 | 1.0 | 1.0 | 0.5 | 0.5 |
| Species | % C | % C | % C | % C | % C | % C |
| Cotton | 70 | 90 | 60 | 10 | 80 | 90 |
| Soybean | 40 | 80 | 60 | 0 | 70 | 100 |
| Field Corn | 80 | 100 | 100 | 20 | 100 | 100 |
| Rice | 50 | 95 | 50 | 10 | 95 | 90 |
| Wheat | 70 | 95 | 80 | 0 | 100 | 95 |
| Field Bindweed | 20 | 100 | 90 | 0 | 100 | 100 |
| Morningglory | 50 | 95 | 80 | 20 | 100 | 100 |
| Velvetleaf | 100 | 100 | 100 | 40 | 100 | 100 |
| Barnyardgrass | 90 | 100 | 95 | 10 | 100 | 100 |
| Green Foxtail | 90 | 100 | 95 | 0 | 100 | 100 |
| Johnsongrass | 90 | 100 | 70 | 0 | 100 | 95 |
| Yellow Nutsedge | 40 | 70 | 40 | 0 | 95 | 70 |

TABLE 4

Postemergence Herbicidal Activity

| Compound No. | 4 | 5 | 8 | 9 | 11 | 12 | 14 | 15 | 18 | 19 | 20 | 21 |
|---|---|---|---|---|---|---|---|---|---|---|---|---|
| Rate (kg/ha) | 4.0 | 4.0 | 4.0 | 4.0 | 4.0 | 4.0 | 4.0 | 4.0 | 4.0 | 4.0 | 4.0 | 4.0 |
| Species | % K | % K | % K | % K | % K | % K | % K | % K | % K | % K | % K | % K |
| Cotton | 90 | 30 | 50 | 50 | 100 | 30 | 60 | 20 | 60 | 100 | 0 | 80 |
| Soybean | 0 | 0 | 0 | 0 | 100 | 0 | 0 | 0 | 0 | 0 | 0 | 0 |
| Field Corn | 0 | 0 | 0 | 0 | 100 | 0 | 0 | 0 | 0 | 0 | 0 | 0 |
| Rice | 0 | 0 | 0 | 0 | 100 | 0 | 0 | 0 | 0 | 20 | 0 | 0 |
| Wheat | 0 | 0 | 0 | 0 | 100 | 0 | 0 | 0 | 0 | 0 | 0 | 0 |
| Field Bindweed | 0 | 0 | 0 | 80 | 100 | 0 | 0 | 0 | 20 | 70 | 40 | 0 |
| Morningglory | 0 | 0 | 0 | 0 | 100 | 0 | 0 | 0 | 0 | 30 | 0 | 0 |
| Velvetleaf | 100 | 100 | 100 | 100 | 100 | 100 | 100 | 100 | 100 | 100 | 100 | 100 |
| Barnyardgrass | 0 | 0 | 0 | 0 | 100 | 0 | 0 | 0 | 0 | 10 | 0 | 0 |
| Green Foxtail | 20 | 80 | 0 | 40 | 100 | 80 | 0 | 0 | 0 | 100 | 80 | 60 |
| Johnsongrass | 0 | 0 | 0 | 0 | 100 | 0 | 0 | 0 | 70 | 90 | 0 | 0 |
| Yellow Nutsedge | 0 | 0 | 0 | 0 | 60 | 0 | 0 | 0 | 0 | 0 | 0 | 0 |

TABLE 4

Postemergence Herbicidal Activity

| Compound No. | 24 | 27 | 28 | 31 | 32 | 34 | 35 | 36 | 37 | 38 | 40 | 41 |
|---|---|---|---|---|---|---|---|---|---|---|---|---|
| Rate (kg/ha) | 4.0 | 4.0 | 4.0 | 4.0 | 4.0 | 4.0 | 4.0 | 4.0 | 4.0 | 4.0 | 4.0 | 4.0 |
| Species | % K | % K | % K | % K | % K | % K | % K | % K | % K | % K | % K | % K |
| Cotton | 100 | 0 | 50 | 90 | 70 | 60 | 100 | 100 | 20 | 100 | 70 | 0 |
| Soybean | 0 | 0 | 0 | 10 | 0 | 0 | 0 | 0 | 0 | 0 | 0 | 0 |
| Field Corn | 0 | 0 | 0 | 0 | 0 | 0 | 0 | 0 | 0 | 0 | 0 | 0 |
| Rice | 0 | 0 | 0 | 30 | 0 | 0 | 30 | 0 | 0 | 0 | 0 | 0 |
| Wheat | 0 | 0 | 0 | 0 | 0 | 0 | 0 | 0 | 0 | 0 | 0 | 0 |
| Field Bindweed | 0 | 0 | 0 | 20 | 0 | 95 | 95 | 50 | 0 | 50 | 90 | 0 |
| Morningglory | 0 | 0 | 0 | 80 | 0 | 20 | 50 | 0 | 0 | 20 | 30 | 0 |
| Velvetleaf | 90 | 95 | 0 | 100 | 100 | 100 | 100 | 100 | 100 | 100 | 100 | 60 |
| Barnyardgrass | 0 | 0 | 0 | 90 | 0 | 0 | 40 | 85 | 20 | 0 | 0 | 0 |
| Green Foxtail | 0 | 0 | 0 | 95 | 95 | 95 | 90 | 40 | 70 | 90 | 100 | 0 |
| Johnsongrass | 0 | 0 | 0 | 100 | 50 | 50 | 60 | 65 | 0 | 0 | 10 | 0 |
| Yellow Nutsedge | 0 | 0 | 0 | 0 | 0 | 0 | 40 | 0 | 0 | 0 | 0 | 0 |

TABLE 4

Postemergence Herbicidal Activity

| Compound No. | 42 | 43 | 44 | 45 | 47 | 48 | 49 | 51 | 52 | 53 | 54 | 55 |
|---|---|---|---|---|---|---|---|---|---|---|---|---|
| Rate (kg/ha) | 4.0 | 4.0 | 4.0 | 2.0 | 4.0 | 4.0 | 4.0 | 4.0 | 4.0 | 4.0 | 2.0 | 4.0 |
| Species | % K | % K | % K | % K | % K | % K | % K | % K | % K | % K | % K | % K |
| Cotton | 65 | 100 | 100 | 80 | 100 | 100 | 100 | 100 | 100 | 100 | 50 | 40 |
| Soybean | 0 | 30 | 10 | 0 | 10 | 100 | 0 | 50 | 0 | 10 | 0 | 0 |
| Field Corn | 0 | 40 | 0 | 0 | 0 | 100 | 0 | 30 | 0 | 0 | 0 | 0 |
| Rice | 40 | 95 | 0 | 50 | 30 | 100 | 0 | 30 | 0 | 0 | 0 | 0 |
| Wheat | 0 | — | 0 | 40 | — | 100 | 0 | — | 80 | 0 | 0 | 0 |
| Field Bindweed | 65 | 100 | 80 | 90 | 0 | 100 | 0 | 100 | 60 | 90 | 85 | 100 |
| Morningglory | 90 | 100 | 90 | 80 | 100 | 100 | 60 | 90 | 20 | 100 | 30 | 80 |
| Velvetleaf | 100 | 100 | 100 | 100 | 100 | 100 | 100 | 100 | 100 | 100 | 100 | 100 |
| Barnyardgrass | 100 | 100 | 100 | 70 | 80 | 100 | 20 | 95 | 0 | 70 | 10 | 50 |
| Green Foxtail | 90 | 90 | 100 | 100 | 100 | 100 | 0 | 100 | 90 | 100 | 70 | 90 |
| Johnsongrass | 100 | 100 | 80 | 100 | 100 | 100 | 80 | 100 | 10 | 100 | 100 | 95 |
| Yellow Nutsedge | 0 | — | 0 | 0 | 0 | 90 | 0 | 0 | 0 | 0 | 0 | 0 |

TABLE 4

Postemergence Herbicidal Activity

| Compound No. | 56 | 57 | 58 | 59 | 60 | 61 | 65 | 66 | 68 | 69 | 70 | 71 |
|---|---|---|---|---|---|---|---|---|---|---|---|---|
| Rate (kg/ha) | 4.0 | 4.0 | 4.0 | 4.0 | 4.0 | 4.0 | 4.0 | 4.0 | 4.0 | 4.0 | 4.0 | 4.0 |
| Species | % K | % K | % K | % K | % K | % K | % K | % K | % K | % K | % K | % K |
| Cotton | 100 | 100 | 90 | 100 | 100 | 100 | 100 | 10 | 100 | 100 | 90 | 60 |
| Soybean | 80 | 0 | 0 | 0 | 0 | 0 | 0 | 0 | 0 | 100 | 0 | 10 |
| Field Corn | 100 | 0 | 0 | 0 | 0 | 0 | 0 | 0 | 0 | 100 | 0 | 0 |
| Rice | 40 | 0 | 0 | 0 | 0 | 0 | 0 | 10 | 0 | 100 | 0 | 0 |
| Wheat | 100 | 0 | 0 | 0 | 0 | 0 | 0 | 0 | 0 | 100 | 0 | 0 |
| Field Bindweed | 100 | 100 | 0 | 0 | 0 | 100 | 20 | 30 | 50 | 100 | 30 | 30 |
| Morningglory | 100 | 100 | 0 | 0 | 10 | 0 | 50 | 50 | 40 | 100 | 30 | 80 |
| Velvetleaf | 100 | 100 | 100 | 100 | 90 | 100 | 100 | 100 | 100 | 100 | 100 | 100 |
| Barnyardgrass | 100 | 0 | 0 | 0 | 0 | 100 | 80 | 70 | 0 | 100 | 90 | 30 |
| Green Foxtail | 100 | 30 | 90 | 95 | 0 | 100 | 20 | 65 | 100 | 100 | 20 | 30 |
| Johnsongrass | 100 | 0 | 0 | 50 | 70 | 100 | 80 | 10 | 0 | 100 | 30 | 0 |
| Yellow Nutsedge | 40 | 0 | 0 | 0 | 0 | 50 | 0 | 0 | 0 | 100 | 0 | 0 |

TABLE 4

Postemergence Herbicidal Activity

| Compound No. | 72 | 73 | 74 | 76 | 78 | 79 | 80 | 84 | 87 | 88 | 94 | 95 |
|---|---|---|---|---|---|---|---|---|---|---|---|---|
| Rate (kg/ha) | 4.0 | 4.0 | 4.0 | 4.0 | 4.0 | 4.0 | 4.0 | 4.0 | 4.0 | 4.0 | 4.0 | 4.0 |
| Species | % K | % K | % K | % K | % K | % K | % K | % K | % K | % K | % K | % K |
| Cotton | 10 | 0 | 10 | 50 | 60 | 90 | 95 | 60 | 30 | 60 | 0 | 85 |
| Soybean | 0 | 0 | 0 | 0 | 0 | 0 | 0 | 0 | 0 | 0 | 0 | 0 |
| Field Corn | 0 | 0 | 0 | 0 | 0 | 0 | 0 | 0 | 0 | 0 | 0 | 0 |
| Rice | 0 | 0 | 0 | 0 | 0 | 0 | 0 | 0 | 0 | 0 | 0 | 10 |
| Wheat | 0 | 0 | 0 | 0 | 0 | 0 | 0 | 0 | 0 | 0 | 0 | 0 |
| Field Bindweed | 100 | 0 | 0 | 40 | 0 | 50 | 10 | 70 | 0 | 30 | 90 | 35 |
| Morningglory | 10 | 0 | 10 | 50 | 0 | 0 | 10 | 0 | 20 | 0 | 0 | 20 |
| Velvetleaf | 60 | 0 | 100 | 100 | 0 | 100 | 100 | 20 | 100 | 100 | 90 | 100 |
| Barnyardgrass | 0 | 0 | 0 | 0 | 0 | 0 | 20 | 0 | 25 | 50 | 0 | 0 |
| Green Foxtail | 0 | 0 | 0 | 0 | 0 | 0 | 20 | 0 | 10 | 65 | 0 | 10 |
| Johnsongrass | 0 | 0 | 0 | 0 | 0 | 0 | 20 | 0 | 0 | 40 | 40 | 0 |
| Yellow Nutsedge | 0 | 0 | 0 | 0 | 0 | 0 | 0 | 0 | 0 | 0 | 0 | 0 |

TABLE 4

Postemergence Herbicidal Activity

| Compound No. | 96 | 97 | 115 | 116 | 117 | 118 | 119 | 120 | 121 | 122 | 123 | 124 |
|---|---|---|---|---|---|---|---|---|---|---|---|---|
| Rate (kg/ha) | 4.0 | 4.0 | 4.0 | 2.0 | 4.0 | 8.0 | 8.0 | 8.0 | 2.0 | 8.0 | 8.0 | 4.0 |
| Species | % K | % K | % K | % K | % K | % K | % K | % K | % C | % C | % C | % C |
| Cotton | 85 | 10 | 100 | 100 | 80 | 100 | 0 | 100 | 100 | 30 | 20 | 100 |
| Soybean | 0 | 0 | 40 | 100 | 40 | 100 | 0 | 10 | 100 | 10 | 20 | 30 |
| Field Corn | 0 | 0 | 100 | 100 | 100 | 100 | 0 | 30 | 100 | 0 | 30 | 40 |
| Rice | 0 | 0 | 100 | 100 | 30 | — | — | — | 100 | 0 | — | 90 |
| Wheat | 0 | 0 | 100 | 100 | 60 | 100 | 0 | 10 | 100 | 0 | 10 | 20 |
| Field Bindweed | 50 | 0 | 100 | 100 | 90 | 100 | 0 | 90 | 100 | 70 | 10 | 100 |
| Morningglory | 0 | 0 | 100 | 100 | 70 | 100 | 0 | 90 | 100 | 0 | 20 | 100 |
| Velvetleaf | 100 | 100 | 100 | 100 | 100 | 100 | 0 | 100 | — | 50 | 20 | 100 |
| Barnyardgrass | 0 | 90 | 100 | 100 | 90 | 100 | 0 | 100 | 100 | 30 | 20 | 100 |
| Green Foxtail | 0 | 60 | 100 | 100 | — | 100 | 0 | 100 | — | 0 | 70 | 100 |
| Johnsongrass | 0 | 90 | 100 | 100 | 100 | 100 | 0 | 100 | 100 | 0 | 20 | 30 |
| Yellow Nutsedge | 0 | 0 | 100 | 100 | 0 | — | — | — | 100 | 10 | — | 0 |

TABLE 4

Postemergence Herbicidal Activity

| Compound No. | 125 | 126 | 127 | 128 | 129 | 130 | 131 | 132 | 133 | 134 | 135 | 136 |
|---|---|---|---|---|---|---|---|---|---|---|---|---|
| Rate (kg/ha) | 1.0 | 4.0 | 2.0 | 2.0 | 2.0 | 4.0 | 8.0 | 2.0 | 2.0 | 2.0 | 2.0 | 2.0 |
| Species | % C | % C | % C | % C | % C | % C | % C | % C | % C | % C | % C | % C |
| Cotton | 100 | 100 | 100 | 100 | 100 | 100 | 0 | 100 | 100 | 100 | 100 | 70 |
| Soybean | 100 | 40 | 0 | 60 | 30 | 50 | 20 | 60 | 100 | 40 | 40 | 50 |
| Field Corn | 100 | 40 | 0 | 100 | 30 | 20 | 0 | 10 | 100 | 40 | 0 | 90 |
| Rice | 100 | 100 | 0 | 100 | 60 | 20 | — | 60 | 100 | 10 | 20 | 30 |
| Wheat | 90 | 70 | 0 | 100 | 10 | 30 | 0 | 20 | 100 | 30 | 40 | 90 |
| Field Bindweed | 100 | 100 | 10 | 100 | 100 | 100 | 0 | 100 | 100 | 100 | 100 | 100 |
| Morningglory | 100 | 100 | 10 | 100 | 100 | 100 | 0 | 80 | 100 | 80 | 90 | 80 |
| Velvetleaf | 100 | 100 | 100 | 100 | 100 | 100 | 0 | 100 | 100 | 100 | 100 | 100 |
| Barnyardgrass | 100 | 90 | 10 | 100 | 20 | 90 | 0 | 80 | 100 | 90 | 90 | 80 |
| Green Foxtail | 100 | 100 | 90 | 100 | 100 | 100 | 0 | 100 | 100 | 100 | 100 | 100 |
| Johnsongrass | 100 | 80 | 0 | 100 | 40 | 80 | 0 | 10 | 100 | 50 | 90 | 80 |
| Yellow Nutsedge | 70 | 30 | 0 | 80 | 0 | 30 | — | 10 | 100 | 0 | 10 | 50 |

TABLE 4

Postemergence Herbicidal Activity

| Compound No. | 137 | 138 | 139 | 140 | 141 | 142 | 143 | 144 | 145 | 146 | 147 | 148 |
|---|---|---|---|---|---|---|---|---|---|---|---|---|
| Rate (kg/ha) | 2.0 | 2.0 | 2.0 | 2.0 | 0.125 | 2.0 | 8.0 | 4.0 | 8.0 | 8.0 | 8.0 | 8.0 |
| Species | % C | % C | % C | % C | % C | % C | % C | % C | % C | % C | % C | % C |
| Cotton | 100 | 100 | 100 | 100 | 100 | 100 | 30 | 100 | 90 | 90 | 20 | 10 |
| Soybean | 20 | 50 | 100 | 20 | 50 | 50 | 10 | 40 | 30 | 70 | 20 | 30 |
| Field Corn | 60 | 40 | 100 | 50 | 30 | 90 | 20 | 60 | 60 | 90 | 20 | 20 |
| Rice | 100 | 100 | 100 | 90 | 40 | 100 | — | 70 | — | — | — | — |
| Wheat | 90 | 100 | 100 | 100 | 10 | 100 | 20 | 80 | 20 | 80 | 10 | 10 |
| Field Bindweed | 100 | 100 | 100 | 100 | 80 | 100 | 10 | 90 | 20 | 30 | 0 | 0 |
| Morningglory | 100 | 90 | 100 | 100 | 80 | 100 | 20 | 90 | 50 | 100 | 20 | 20 |
| Velvetleaf | 100 | 100 | 100 | 100 | 100 | 100 | 20 | 100 | 100 | 100 | 30 | 20 |
| Barnyardgrass | 100 | 100 | 100 | 100 | 70 | 100 | 0 | 100 | 20 | 90 | 0 | 10 |
| Green Foxtail | 100 | 100 | 100 | 100 | 90 | 100 | 50 | 90 | 70 | 70 | 0 | 0 |
| Johnsongrass | 100 | 100 | 100 | 100 | 10 | 90 | 0 | 70 | 20 | 80 | 0 | 0 |
| Yellow Nutsedge | 80 | 70 | 100 | 60 | 20 | 80 | — | 10 | — | — | — | — |

TABLE 4

Postemergence Herbicidal Activity

| Compound No. | 149 | 150 | 151 | 152 | 153 | 154 | 155 | 156 | 157 | 158 | 159 | 160 |
|---|---|---|---|---|---|---|---|---|---|---|---|---|
| Rate (kg/ha) | 8.0 | 8.0 | 8.0 | 1.0 | 1.0 | 2.0 | 8.0 | 8.0 | 8.0 | 8.0 | 8.0 | 1.0 |
| Species | % C | % C | % C | % C | % C | % C | % C | % C | % C | % C | % C | % C |
| Cotton | 80 | 40 | 20 | 100 | 100 | 100 | 100 | 80 | 40 | 100 | 100 | 100 |
| Soybean | 40 | 20 | 30 | 30 | 30 | 50 | 70 | 60 | 30 | 100 | 100 | 70 |
| Field Corn | 40 | 20 | 20 | 30 | 20 | 50 | 70 | 30 | 20 | 100 | 100 | 30 |
| Rice | — | — | — | 100 | 20 | 100 | — | — | — | — | — | 90 |
| Wheat | 30 | 10 | 10 | 100 | 20 | 100 | 20 | 10 | 0 | 100 | 100 | 40 |
| Field Bindweed | 80 | 0 | 10 | 100 | 90 | 100 | 100 | 20 | 0 | 100 | 100 | 100 |
| Morningglory | 60 | 50 | 20 | 100 | 90 | 100 | 100 | 80 | 20 | 100 | 100 | 90 |
| Velvetleaf | 100 | 50 | 20 | 100 | 100 | 100 | 100 | 90 | 90 | 100 | 100 | 100 |
| Barnyardgrass | 20 | 0 | 10 | 100 | 70 | 100 | 100 | — | 0 | 100 | 100 | 100 |
| Green Foxtail | 100 | 10 | 10 | 100 | 100 | 100 | 100 | 40 | 20 | 100 | 100 | 100 |
| Johnsongrass | 80 | 0 | 10 | 40 | 40 | 100 | 90 | 40 | 20 | 100 | 100 | 100 |
| Yellow Nutsedge | — | — | — | 30 | 20 | 90 | — | — | — | — | — | 40 |

TABLE 4

Postemergence Herbicidal Activity

| Compound No. | 161 | 162 | 163 | 164 | 165 | 166 | 167 | 168 | 169 | 170 | 171 | 172 |
|---|---|---|---|---|---|---|---|---|---|---|---|---|
| Rate (kg/ha) | 8.0 | 8.0 | 8.0 | 0.5 | 1.0 | 8.0 | 8.0 | 1.0 | 0.125 | 1.0 | 1.0 | 0.5 |
| Species | % C | % C | % C | % C | % C | % C | % C | % C | % C | % C | % C | % C |
| Cotton | 100 | 60 | 80 | 100 | 100 | 100 | 100 | 80 | 100 | 100 | 100 | 100 |
| Soybean | 100 | 30 | 40 | 50 | 40 | 80 | 70 | 20 | 80 | 50 | 50 | 60 |
| Field Corn | 100 | 0 | 30 | 60 | 40 | 90 | 100 | 30 | 30 | 50 | 10 | 90 |
| Rice | — | — | — | 80 | 60 | — | — | 80 | 50 | 40 | 100 | 40 |
| Wheat | 100 | 20 | 40 | 80 | 70 | 100 | 100 | 30 | 70 | 30 | 90 | 60 |
| Field Bindweed | 100 | 20 | 90 | 90 | 100 | 100 | 100 | 20 | 90 | 100 | 100 | 90 |
| Morningglory | 100 | 20 | 50 | 90 | 100 | 100 | 100 | 100 | 100 | 90 | 90 | 50 |
| Velvetleaf | 100 | 90 | 100 | 100 | 100 | 100 | 100 | 100 | 100 | 100 | 100 | 100 |
| Barnyardgrass | 100 | 0 | 60 | 60 | 100 | — | — | 80 | 50 | 40 | 100 | 90 |
| Green Foxtail | 100 | — | — | 100 | 100 | 100 | 100 | 40 | 100 | 100 | 100 | 90 |
| Johnsongrass | 100 | 20 | 40 | 30 | 40 | 100 | 100 | 30 | 50 | 40 | 50 | 80 |
| Yellow Nutsedge | — | — | — | 50 | 10 | — | — | 10 | 30 | 20 | 60 | 30 |

TABLE 4

Postemergence Herbicidal Activity

| Compound No. | 173 | 174 | 175 | 176 | 177 | 178 | 179 | 180 | 181 | 182 | 183 | 184 |
|---|---|---|---|---|---|---|---|---|---|---|---|---|
| Rate (kg/ha) | 0.125 | 0.5 | 1.0 | 8.0 | 8.0 | 8.0 | 4.0 | 8.0 | 8.0 | 2.0 | 1.0 | 1.0 |
| Species | % C | % C | % C | % C | % C | % C | % C | % C | % C | % C | % C | % C |
| Cotton | 90 | 100 | 100 | 30 | 80 | 70 | 100 | 40 | 30 | 80 | 90 | 100 |
| Soybean | 40 | 90 | 80 | 20 | 50 | 40 | 40 | 30 | 30 | 30 | 30 | 50 |
| Field Corn | 60 | 100 | 60 | 10 | 60 | 20 | 30 | 20 | 20 | 20 | 20 | 30 |
| Rice | 60 | 50 | 90 | — | — | — | 40 | — | — | 60 | 90 | 80 |
| Wheat | 70 | 80 | 100 | 0 | 40 | 20 | 20 | 0 | 10 | 50 | 50 | 50 |
| Field Bindweed | 70 | 100 | 100 | 40 | 70 | 40 | 100 | 50 | 0 | 90 | 100 | 100 |
| Morningglory | 90 | 100 | 90 | 0 | 80 | 50 | 100 | 40 | 30 | 80 | 90 | 100 |
| Velvetleaf | 100 | 100 | 100 | 20 | 100 | 90 | 100 | 50 | 60 | 100 | 100 | 100 |
| Barnyardgrass | 60 | 100 | 100 | 30 | 70 | 40 | 100 | 30 | 90 | 100 | 80 | 90 |
| Green Foxtail | 50 | 100 | 100 | 20 | 70 | 40 | 100 | 30 | 30 | 90 | 90 | 90 |
| Johnsongrass | 60 | 90 | 100 | 20 | 80 | 50 | 50 | 30 | 0 | 50 | 50 | 40 |
| Yellow Nutsedge | 20 | 40 | 70 | — | 100 | — | 20 | — | — | 60 | 30 | 50 |

TABLE 4

Postemergence Herbicidal Activity

| Compound No. | 185 | 186 | 187 | 188 | 189 | 190 | 191 | 192 | 193 | 194 | 195 | 196 |
|---|---|---|---|---|---|---|---|---|---|---|---|---|
| Rate (kg/ha) | 1.0 | 8.0 | 0.5 | 1.0 | 1.0 | 0.125 | 1.0 | 1.0 | 1.0 | 1.0 | 1.0 | 8.0 |
| Species | % C | % C | % C | % C | % C | % C | % C | % C | % C | % C | % C | % C |
| Cotton | 50 | 0 | 90 | 100 | 10 | 100 | 80 | 40 | 90 | 60 | 100 | 60 |
| Soybean | 30 | 10 | 20 | 70 | 10 | 60 | 70 | 30 | 90 | 80 | 60 | 40 |
| Field Corn | 20 | 10 | 0 | 10 | 0 | 70 | 70 | 10 | 100 | 20 | 50 | 10 |
| Rice | 40 | — | 0 | 90 | 0 | 100 | 70 | 0 | 100 | 0 | 60 | — |
| Wheat | 20 | 10 | 0 | 100 | 0 | 80 | 80 | 0 | 30 | 10 | 10 | 30 |
| Field Bindweed | 70 | 0 | 50 | 100 | 0 | 100 | 90 | 80 | 100 | 100 | 100 | 100 |
| Morningglory | 60 | 0 | 20 | 90 | 0 | 30 | 90 | 10 | 100 | 30 | 100 | 30 |
| Velvetleaf | 100 | 0 | 100 | 100 | 0 | 100 | 100 | 90 | 100 | 100 | 100 | 100 |
| Barnyardgrass | 50 | 0 | 0 | 100 | 10 | 90 | 100 | 20 | 100 | 60 | 100 | 30 |
| Green Foxtail | 60 | 0 | 10 | 100 | 0 | 100 | 100 | 100 | 100 | 80 | 100 | 30 |
| Johnsongrass | 60 | 0 | 60 | 80 | 0 | 70 | 90 | 70 | 100 | 20 | 70 | 20 |
| Yellow Nutsedge | 20 | — | 0 | 90 | 0 | 40 | 10 | 0 | 70 | 0 | 20 | — |

TABLE 4

Postemergence Herbicidal Activity

| Compound No. | 197 | 198 | 199 | 200 | 201 | 202 | 203 | 204 | 205 | 206 | 207 | 208 |
|---|---|---|---|---|---|---|---|---|---|---|---|---|
| Rate (kg/ha) | 8.0 | 8.0 | 0.5 | 2.0 | 2.0 | 2.0 | 0.5 | 0.5 | 0.5 | 0.25 | 4.0 | 0.5 |
| Species | % C | % C | % C | % C | % C | % C | % C | % C | % C | % C | % C | % C |
| Cotton | 80 | 90 | 90 | 60 | 90 | 90 | 90 | 90 | 100 | 100 | 90 | 100 |
| Soybean | 30 | 20 | 40 | 60 | 90 | 40 | 80 | 80 | 50 | 70 | 90 | 60 |
| Field Corn | 30 | 10 | 20 | 40 | 20 | 30 | 70 | 80 | 40 | 40 | 30 | 30 |
| Rice | — | — | 10 | 10 | 20 | 10 | 30 | 40 | 40 | 70 | 20 | 50 |
| Wheat | 50 | 40 | 50 | 0 | 40 | 10 | 50 | 100 | 90 | 60 | 30 | 70 |
| Field Bindweed | 100 | 100 | 90 | 20 | 60 | 10 | 100 | 90 | 90 | 100 | 40 | 90 |
| Morningglory | 80 | 60 | 90 | 20 | 10 | 60 | 90 | 100 | 90 | 100 | 30 | 100 |
| Velvetleaf | 100 | 100 | 100 | 10 | 100 | 100 | 100 | 100 | 100 | 100 | 100 | 100 |
| Barnyardgrass | 80 | 40 | 0 | 10 | 10 | 0 | — | — | — | — | — | 60 |
| Green Foxtail | 100 | 50 | 100 | 70 | 90 | 50 | 80 | 100 | 100 | 100 | 40 | 90 |
| Johnsongrass | 60 | 30 | 10 | 0 | 30 | 0 | 40 | 70 | 50 | 90 | 20 | 70 |
| Yellow Nutsedge | — | — | 0 | 0 | 10 | 0 | 40 | 50 | 60 | 40 | 30 | 30 |

TABLE 4

Postemergence Herbicidal Activity

| Compound No. | 209 | 210 | 212 | 213 | 214 | 215 | 216 | 217 | 218 | 219 | 220 | 221 |
|---|---|---|---|---|---|---|---|---|---|---|---|---|
| Rate (kg/ha) | 1.0 | 8.0 | 8.0 | 8.0 | 8.0 | 8.0 | 2.0 | 8.0 | 0.5 | 0.5 | 2.0 | 1.0 |
| Species | % C | % C | % C | % C | % C | % C | % C | % C | % C | % C | % C | % C |
| Cotton | 100 | 0 | 0 | 10 | 0 | 100 | 100 | 80 | 100 | 90 | 100 | 100 |
| Soybean | 40 | 0 | 0 | 10 | 0 | 100 | 90 | 40 | 60 | 60 | 90 | 90 |
| Field Corn | 20 | 0 | 0 | 10 | 0 | 100 | 90 | 10 | 70 | 60 | 50 | 90 |
| Rice | 100 | — | — | — | — | 100 | 90 | — | 90 | 50 | 100 | 95 |
| Wheat | 90 | 0 | 0 | 0 | 0 | 100 | 100 | 20 | 60 | 70 | 60 | 90 |
| Field Bindweed | 100 | 0 | 0 | 0 | 0 | 100 | 100 | 80 | 100 | 100 | 100 | 100 |
| Morningglory | 100 | 0 | 0 | 80 | 0 | 100 | 100 | 70 | 100 | 100 | 100 | 100 |
| Velvetleaf | 100 | 0 | 0 | 0 | 0 | 100 | 100 | 100 | 100 | 100 | 100 | 100 |
| Barnyardgrass | 100 | 0 | 0 | 0 | 0 | 100 | 100 | 40 | 90 | 100 | 60 | 100 |
| Green Foxtail | 100 | 0 | 0 | 0 | 0 | 100 | 100 | 20 | 100 | 60 | 50 | 95 |
| Johnsongrass | 70 | 0 | 0 | 0 | 0 | 100 | 100 | 10 | 90 | 90 | 60 | 90 |
| Yellow Nutsedge | 70 | — | — | — | — | 80 | 80 | — | 90 | 40 | 40 | 40 |

TABLE 4

Postemergence Herbicidal Activity

| Compound No. | 222 | 223 | 224 | 225 |
|---|---|---|---|---|
| Rate (kg/ha) | 1.0 | 1.0 | 0.5 | 0.5 |
| Species | % C | % C | % C | % C |
| Cotton | 100 | 95 | 100 | 100 |
| Soybean | 90 | 50 | 95 | 100 |
| Field Corn | 80 | 50 | 100 | 100 |
| Rice | 60 | 40 | 95 | 90 |
| Wheat | 70 | 30 | 100 | 95 |
| Field Bindweed | 90 | 70 | 100 | 100 |
| Morningglory | 90 | 95 | 100 | 100 |
| Velvetleaf | 100 | 80 | 100 | 100 |
| Barnyardgrass | 100 | 30 | 100 | 100 |
| Green Foxtail | 100 | 10 | 100 | 100 |
| Johnsongrass | 70 | 20 | 100 | 100 |
| Yellow Nutsedge | 30 | 10 | 95 | 40 |

## Claims

1. A herbicidal compound of the formula

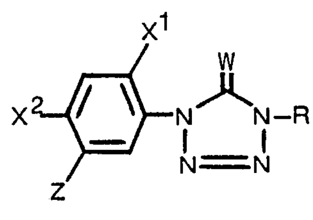

in which

W is oxygen or sulfur;

R is alkyl, haloalkyl, alkoxyalkyl, alkylthioalkyl, cyanoalkyl, haloalkoxyalkyl, trifluoromethylthio, alkenyl, or haloalkenyl;

one of $X^1$ and $X^2$ is fluorine, chlorine, or bromine and the other is fluorine, chlorine, bromine, alkyl, or haloalkyl; or $X^2$ is nitro and $X^1$ is fluorine, chlorine, or bromine; and

Z is

(a) hydrogen, fluorine, chlorine, bromine, cyano, nitro, alkyl, alkynyl, or alkyl substituted with fluorine, chlorine, bromine or $C_{1-4}$ alkoxy;

(b) a group $-QR^1$, $-OSO_2R^2$, $-QR^7CO-Q^1R^8$, $-QR^7CO_2N=C(R^9)(R^{10})$, $-QR^7C(CH_2)=R^{11}$, or $-QR^7CON(R^{12})(R^{13})$ in which

Q and $Q^1$ are independently oxygen or sulfur,

$R^1$ is alkyl, haloalkyl, hydroxyalkyl, cyanoalkyl, alkoxyalkyl, alkylthioalkyl, alkylsulfinylalkyl, alkylsulfonylalkyl, alkanoyl, alkenyl, haloalkenyl, alkenyloxyalkyl, alkynyl, haloalkynyl, alkoxycarbonyl, a three- to eight-membered ring heterocyclic group having one or two similar or different ring heteroatoms that are oxygen or sulfur, or an alkyl radical substituted with such a heterocyclic group,

$R^2$ is alkyl, haloalkyl, cyanoalkyl, phenylalkyl, cyclic alkyl, alkenyl, haloalkenyl, phenylalkenyl, alkynyl, haloalkynyl, phenylalkynyl, phenyl, or a group of the formula $-(CH_2)_mNR^3R^4$ or $(CH_2)_n-Y-R^5$ where

m is 0 or an integer from 1 to 5,

n is an integer from 1 to 5,

$R^3$ is hydrogen or alkyl,

$R^4$ is alkyl or a group $-CH_2-Y-R^5$,

$R^5$ is alkyl, alkenyl, alkynyl, or a radical $-CH(R^{18})CO_2R^{19}$,

$R^{18}$ and $R^{19}$ are independently hydrogen or alkyl, and

Y is oxygen or $S(O)_r$ where R is 0, 1 or 2,

$R^7$ is an alkylene or fluoroalkylene radical,

$R^8$ is hydrogen, alkyl, substituted alkyl, alkenyl, alkynyl, phenyl, 3-tetrahydrofuranyl, 2-oxo-3-tetrahydrofuranyl, 3-tetrahydrothienyl or the oxide or dioxide thereof, 3-pyridyl, cyclopropyl, cyclopentyl, cyclohexyl, cyclopropylmethyl, tetrahydrofurfuryl, furfuryl, thenyl, or benzyl,

one of $R^9$ and $R^{10}$ is alkyl and the other is alkyl or alkylthio,

$R^{11}$ is oxygen or $N-OR^{20}$ in which $R^{20}$ is hydrogen or alkyl, and

one of $R^{12}$ and $R^{13}$ is hydrogen, alkyl, or alkenyl and the other is hydrogen, alkyl, cyanoalkyl, alkoxyalkyl, alkenyl, alkynyl, alkylsulfonyl, arylsulfonyl (in which aryl is unsubstituted phenyl or phenyl substituted with halogen or alkyl), haloalkylsulfonyl, alkylaminosulfonyl, dialkylaminosulfonyl, or CH(H or $CH_3)CO_2$alkyl, or $N(R^{12})(R^{13})$ is a pyrrolidino, piperidino, or morpholino ring; or

(c) a group $-N(R^{14})(R^{15})$, $-SO_2R^{16}$, or $-CO-R^{17}$ in which

$R^{14}$ and $R^{15}$ are independently hydrogen, alkyl, alkenyl, alkynyl, cyanoalkyl, acetyl, alkoxycarbonyl, alkoxyalkyl, aminocarbonyl, alkylaminocarbonyl, dialkylaminocarbonyl, alkylsulfonyl, haloalkylsulfonyl, arylsulfonyl (in which aryl is unsubstituted phenyl or phenyl substituted with halogen or alkyl) or CH(H or $CH_3)CO_2$-alkyl, or $N(R^{14})(R^{15})$ is a group $N=C(R^9)(R^{10})$ in which one of $R^9$ and $R^{10}$ is alkyl and the other is hydrogen, alkyl or alkylthio, or $N(R^{14})(R^{15})$ is a tetrahydrophthalimido or 2-oxopyrrolidino group,

$R^{16}$ is hydroxy, amino, alkylamino, dialkylamino, or arylamino in which aryl is unsubstituted phenyl or phenyl substituted with halogen or alkyl, and

$R^{17}$ is hydroxy, alkoxy, alkylthio, amino, alkylamino, dialkylamino, or arylamino in which aryl is unsubstituted phenyl or phenyl substituted with halogen or alkyl;

or a compound that is a salt of such a compound having a free acidic hydrogen atom.

2. A compound as claimed in Claim 1 in which W is oxygen and Z is nitro.

3. A compound as claimed in Claim 1 in which W is oxygen and Z is $-QR^1$.

4. A compound as claimed in Claim 3 in which Q is oxygen and $R^1$ is alkoxyalkyl.

5. A compound as claimed in Claim 4 in which $R^1$ is methoxymethyl.

6. A compound as claimed in Claim 1 in which W is oxygen and Z is $-N(R^{14})(R^{15})$, where $R^{14}$ and $R^{15}$ are as defined in Claim 1, or a salt thereof.

7. A compound as claimed in Claim 6 in which Z is $-NH_2$.

8. A compound as claimed in Claim 6 in which at least one of $R^{14}$ and $R^{15}$ is alkylsulfonyl or haloalkylsulfonyl.

9. A compound as claimed in Claim 6 in which one of $R^{14}$ and $R^{15}$ is alkylsulfonyl and the other is hydrogen or alkylsulfonyl.

10. A compound as claimed in Claim 9 in which one of $R^{14}$ and $R^{15}$ is alkylsulfonyl and the other is hydrogen.

11. A salt as claimed in Claim 10.

12. A compound as claimed in Claim 6 in which one of $R^{14}$ and $R^{15}$ is $-CH(CH_3)CO_2$-alkyl or $-CH_2CO_2$alkyl.

13. A compound as claimed in Claim 12 in which one of $R^{14}$ and $R^{15}$ is hydrogen and the other is $CH(CH_3)CO_2$-alkyl.

14. A compound as claimed in any preceding claim in which R is n-propyl or 3-fluoropropyl, $X^1$ is fluorine or chlorine, and $X^2$ is chlorine or bromine.

15. A compound as claimed in Claim 14 in which R is 3-fluoropropyl, $X^1$ is fluorine, and $X^2$ is chlorine.

16. A herbicidal composition containing, as a herbicidal ingredient, a compound as claimed in any one of Claims 1 to 15.

17. A method for controlling undesired plant growth comprising applying to the locus where such control is desired a herbicidally effective amount of a composition as claimed in Claim 30.

18. A compound of the formula

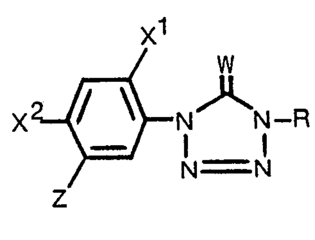

in which W, $X^1$ and $X^2$ are as defined in Claim 1, and

(a) R is alkyl, haloalkyl, alkoxyalkyl, alkylthioalkyl, cyanoalkyl, haloalkoxyalkyl, trifluoromethylthio, alkenyl, haloalkenyl, 2-oxopropyl, or 3-oxopropyl; and Z is hydroxy, mercapto or benzyloxy; or

(b) R is hydrogen and Z is fluorine, chlorine, bromine, cyano, nitro, alkyl, haloalkyl, alkynyl, or $QR^1$ in which Q is sulfur or oxygen and $R^1$ is alkyl, benzyl, haloalkyl, cyanoalkyl, alkoxyalkyl, alkylthioalkyl, alkylsulfinylalkyl, alkylsulfonylalkyl, alkenyl, haloalkenyl, alkenyloxyalkyl, alkynyl, or haloalkynyl of 2 to 5 carbon atoms.

19. A compound as claimed in Claim 18 in which W is oxygen, $X^1$ is fluorine or chlorine, $X^2$ is chlorine, and

(a) R is n-propyl or 3-fluoropropyl and Z is hydroxy, mercapto, or benzyloxy, or

(b) R is hydrogen and Z is benzyloxy or $C_{1-4}$ alkoxy.

## Patentansprüche

1. Herbizide Verbindung der Formel

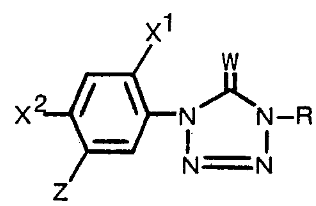

in der

W Sauerstoff oder Schwefel ist;

R Alkyl, Halogenalkyl, Alkoxyalkyl, Alkylthioalkyl, Cyanoalkyl, Halogenalkoxyalkyl, Trifluormethylthio, Alkenyl oder Halogenalkenyl bedeutet;

einer der Reste $X^1$ und $X^2$ Fluor, Chlor oder Brom ist und der andere Fluor, Chlor, Brom, Alkyl oder Halogenalkyl bedeutet; oder $X^2$ Nitro ist und $X^1$ Fluor, Chlor oder Brom bedeutet; und

Z

(a) Wasserstoff, Fluor, Chlor, Brom, Cyano, Nitro, Alkyl, Alkynyl oder durch Fluor, Chlor, Brom oder Alkoxy substituiertes Alkyl;

(b) einen der Reste $—QR^1$, $—OSO_2R^2$, $—QR^7CO—Q^1R^8$, $—QR^7CO_2N=C(R^9)(R^{10})$, $—QR^7C(CH_3)=R^{11}$ oder $—QR^7CON(R^{12})(R^{13})$ darstellt, wobei

Q und $Q^1$ unabhängig voneinander Sauerstoff oder Schwefel sind,

$R^1$ Alkyl, Halogenalkyl, Hydroxyalkyl, Cyanoalkyl, Alkoxyalkyl, Alkylthioalkyl, Alkylsulfinylalkyl, Alkylsulfonylalkyl, Alkanoyl, Alkenyl, Halogenalkenyl, Alkenyloxyalkyl, Alkinyl, Halogenalkinyl, Alkoxycarbonyl, einen drei- bis achtgliedrigen heterocyclischen Ring mit einem oder zwei gleichen oder verschiedenen Ring-Heteroatomen, die Sauerstoff oder Schwefel sind, oder einen durch eine derartige heterocyclische Gruppe substituierten Alkylrest darstellt,

$R^2$ Alkyl, Halogenalkyl, Cyanoalkyl, Phenylalkyl, Cycloalkyl, Alkenyl, Halogenalkenyl, Phenylalkenyl, Alkinyl, Halogenalkinyl, Phenylalkinyl, Phenyl oder einen Rest der Formel

$$—(CH_2)_mNR^3R^4 \text{ oder } (CH_2)_n—Y—R^5$$

bedeutet, wobei

m 0 oder eine ganze Zahl von 1 bis 5 ist,

n eine ganze Zahl von 1 bis 5 ist,

$R^3$ Wasserstoff oder Alkyl bedeutet,

$R^4$ Alkyl oder einen Rest $—CH_2—Y—R^5$ darstellt,

$R^5$ Alkyl, Alkenyl, Alkinyl oder einen Rest $—CH(R^{18})CO_2R^{19}$ bedeutet,

$R^{18}$ und $R^{19}$ unabhängig voneinander Wasserstoff oder Alkyl darstellen, und

Y Sauerstoff oder $S(O)_r$ bedeutet, wobei r 0, 1 oder 2 ist,

$R^7$ einen Alkylen- oder Fluoralkylenrest darstellt,

$R^8$ Wasserstoff, Alkyl, substituiertes Alkyl, Alkenyl, Alkinyl, Phenyl, 3-Tetrahydrofuranyl, 2-Oxo-3-tetrahydrofuranyl, 3-Tetrahydrothienyl oder das Oxid oder Dioxid davon, 3-Pyridyl, Cyclopropyl, Cyclopentyl, Cyclohexyl, Cyclopropylmethyl, Tetrahydrofurfuryl, Furfuryl, Thenyl oder Benzyl bedeutet,

einer der Reste $R^9$ und $R^{10}$ Alkyl und der andere Alkyl oder Alkylthio ist,

$R^{11}$ Sauerstoff oder $N—OR^{20}$ bedeutet, wobei $R^{20}$ Wasserstoff oder Alkyl ist, und

einer der Reste $R^{12}$ und $R^{13}$ Wasserstoff, Alkyl oder Alkenyl und der andere Wasserstoff, Alkyl, Cyanoalkyl, Alkoxyalkyl, Alkenyl, Alkinyl, Alkylsulfonyl, Arylsulfonyl (in dem der Arylrest unsubstituiertes Phenyl oder mit Halogen oder Alkyl substituiertes Phenyl ist), Halogenalkylsulfonyl, Alkylaminosulfonyl, Dialkylaminosulfonyl oder CH(H oder $CH_3$)$CO_2$alkyl ist oder $N(R^{12})(R^{13})$ ein Pyrrolidino-, Piperidino- oder Morpholinoring ist; oder

(c) einen Rest —$N(R^{14})(R^{15})$, —$SO_2R^{16}$ oder —CO—$R^{17}$ bedeutet, wobei

$R^{14}$ und $R^{15}$ unabhängig voneinander Wasserstoff, Alkyl, Alkenyl, Alkinyl, Cyanoalkyl, Acetyl, Alkoxycarbonyl, Alkoxyalkyl, Aminocarbonyl, Alkylaminocarbonyl, Dialkylaminocarbonyl, Alkylsulfonyl, Halogenalkylsulfonyl, Arylsulfonyl (in dem das Aryl unsubstituiertes Phenyl oder mit Halogen oder Alkyl substituiertes Phenyl ist) oder CH(H oder $CH_3)CO_2$-Alkyl bedeuten, oder $N(R^{14})(R^{15})$ einen Rest $N{=}C(R^9)(R^{10})$ darstellt, indem einer der Reste $R^9$ und $R^{10}$ Alkyl und der andere Wasserstoff, Alkyl oder Alkylthio ist, oder $N(R^{14})(R^{15})$ eine Tetrahydrophthalimido- oder 2-Oxopyrrolidinogruppe ist,

$R^{16}$ Hydroxy, Amino, Alkylamino, Dialkylamino oder Arylamino, wobei das Aryl unsubstituiertes Phenyl oder mit Halogen oder Alkyl substituiertes Phenyl bedeutet, ist, und

$R^{17}$ Hydroxy, Alkoxy, Alkylthio, Amino, Alkylamino, Dialkylamino oder Arylamino darstellt, in dem das Aryl unsubstituiertes Phenyl oder mit Halogen oder Alkyl substituiertes Phenyl bedeutet; oder

eine Verbindung, die ein Salz einer solchen Verbindung mit einem freien sauren Wasserstoffatom ist.

2. Verbindung nach Anspruch 1, in der W Sauerstoff ist und Z Nitro bedeutet.

3. Verbindung nach Anspruch 1, in der W Sauerstoff ist und Z —$QR^1$ bedeutet.

4. Verbindung nach Anspruch 3, in der Q Sauerstoff ist und $R^1$ Alkoxyalkyl bedeutet.

5. Verbindung nach Anspruch 4, in der $R^1$ Methoxymethyl bedeutet.

6. Verbindung nach Anspruch 1, in der W Sauerstoff ist und Z —$N(R^{14})(R^{15})$ bedeutet, wobei $R^{14}$ und $R^{15}$ wie in Anspruch 1 definiert sind, oder ein Salz davon.

7. Verbindung nach Anspruch 6, in der Z —$NH_2$ bedeutet.

8. Verbindung nach Anspruch 6, in der mindestens einer der Reste $R^{14}$ und $R^{15}$ Alkylsulfonyl oder Halogenalkylsulfonyl bedeutet.

9. Verbindung nach Anspruch 6, in der einer der Reste $R^{14}$ und $R^{15}$ Alkylsulfonyl ist und der andere Wasserstoff oder Alkylsulfonyl bedeutet.

10. Verbindung nach Anspruch 9, in der einer der Reste $R^{14}$ und $R^{15}$ Alkylsulfonyl ist und der andere Wasserstoff ist.

11. Ein Salz gemäß Anspruch 10.

12. Verbindung nach Anspruch 10, in der einer der Reste $R^{14}$ und $R^{15}$ —$CH(CH_3)CO_2$-alkyl oder $CH_2CO_2$-alkyl bedeutet.

13. Verbindung nach Anspruch 12, in der einer der Reste $R^{14}$ und $R^{15}$ Wasserstoff ist und der andere $CH(CH_3)CO_2$-alkyl bedeutet.

14. Verbindung nach einem der vorangehenden Ansprüche, in der R n-Propyl oder 3-Fluorpropyl ist, $X^1$ Fluor oder Chlor ist und $X^2$ Chlor oder Brom ist.

15. Verbindung nach Anspruch 14, in der R 3-Fluorpropyl ist, $X^1$ Fluor ist und $X^2$ Chlor ist.

16. Herbizides Mittel, enthaltend als herbiziden Bestandteil eine Verbindung nach einem der Ansprüche 1 bis 15.

17. Verfahren zur Unterdrückung von unerwünschtem Pflanzenwachstum, umfassend das Aufbringen einer Herbizid wirksamen Menge eines Mittels nach Anspruch 16 auf die Stelle, an der eine solche Unterdrückung gewünscht wird.

18. Verbindung der Formel

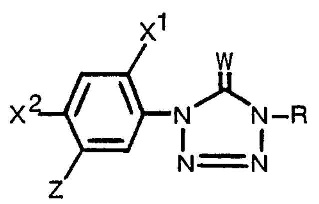

in der W, $X^1$ und $X^2$ wie in Anspruch 1 definiert sind und

(a) R Alkyl, Halogenalkyl, Alkoxyalkyl, Alkylthioalkyl, Cyanoalkyl, Halogenalkoxyalkyl, Trifluormethylthio, Alkenyl, Halogenalkenyl, 2-Oxopropyl oder 3-Oxopropyl bedeutet und Z Hydroxy, Mercapto oder Benzyloxy ist; oder

(b) R Wasserstoff ist und Z Fluor, Chlor, Brom, Cyano, Nitro, Alkyl, Halogenalkyl, Alkenyl oder $QR^1$ bedeutet, wobei Q Schwefel oder Sauerstoff ist und $R^1$ Alkyl, Benzyl, Halogenalkyl, Cyanoalkyl, Alkoxyalkyl, Alkylthioalkyl, Alkylsulfinylalkyl, Alkylsulfonylalkyl, Alkenyl, Halogenalkenyl, Alkenyloxyalkyl, Alkinyl oder Halogenalkinyl mit 2 bis 5 Kohlenstoffatomen bedeutet.

19. Verbindung nach Anspruch 18, in der W Sauerstoff ist, $X^1$ Fluor oder Chlor ist, $X^2$ Chlor ist und

(a) R n-Propyl oder 3-Fluorpropyl bedeutet und Z Hydroxy, Mercapto oder Benzyloxy darstellt, oder

(b) R Wasserstoff ist und Z Benzyloxy oder $C_{1-4}$-Alkoxy bedeutet.

**Revendications**

1. Composé herbicide, de formule:

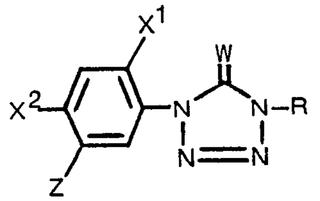

dans laquelle

W représente un atome d'oxygène ou de soufre;

R représente un groupe alkyle, haloalkyle, alkoxyalkyle, alkylthioalkyle, cyanoalkyle, haloalkoxyalkyle, trifluorométhylthio, alkényle ou haloalkényle;

l'un des groupes $X^1$ et $X^2$, représente un atome de fluor, de chlore ou de brome, et l'autre représente un atome de fluor, de chlore, de brome, un groupe alkyle ou haloalkyle; ou $X^2$ représente un groupe nitro et $X^1$ représente un atome de fluor, de chlore ou de brome; et

Z représente:

(a) un atome d'hydrogène, de fluor, de chlore, de brome, un groupe cyano, nitro, alkyle, alkynyle ou alkyle substitué avec un atome de fluor, de chlore, de brome ou un groupe alkoxy en $C_1$—$C_4$;

(b) un groupe $—QR^1$, $—OSO_2R^2$, $—QR^7CO—Q^1R^8$, $—QR^7CO_2N{=}C(R^9)(R^{10})$, $—QR^7C(CH_3){=}R^{11}$ ou $—QR^7CON(R^{12})(R^{13})$ dans lesquels:

Q et $Q^1$ représentent indépendamment des atomes d'oxygène ou de soufre,

$R^1$ représente un groupe alkyle, haloalkyle, hydroxyalkyle, cyanoalkyle, alkoxyalkyle, alkylthioalkyle, alkylsulfinylalkyle, alkylsulfonylalkyle, alkanoyle, alkényle, haloalkényle, alkényloxyalkyle, alkynyle, haloalkynyle, alkoxycarbonyle, un groupe hétérocyclique à cycle comportant de trois à huit chaînons et comprenant un ou deux hétéroatomes de cycle indentiques ou différents, qui sont des atomes d'oxygène ou de soufre, ou un groupe alkyle substitué avec un tel groupe hétérocyclique,

$R^2$ représente un groupe alkyle, haloalkyle, cyanoalkyle, phénylalkyle, alkyle cyclique, alkényle, haloalkényle, phénylalkényle, alkynyle, halo-alkynyle, phénylalkynyle, phényle, ou un groupe de formule $—(CH_2)_mNR^3R^4$ ou $(CH_2)_n—Y—R^5$ dans lequel:

m est égal à 0 ou représente un entier de 1 à 5,

n est un entier de 1 à 5,

$R^3$ représente un atome d'hydrogène ou un groupe alkyle,

$R^4$ représente un groupe alkyle ou un groupe $—CH_2—Y—R^5$,

$R^5$ représente un groupe alkyle, alkényle, alkynyle ou un groupe $—CH(R^{18})CO_2R^{19}$,

$R^{18}$ et $R^{19}$, représentent indépendamment un atome d'hydrogène ou un groupe alkyle, et

Y représente un atome d'oxygène ou un groupe $S(O)_r$ dans lequel r est égal à 0, 1 ou 2,

$R^7$ représente un groupe alkylène ou fluoroalkylène,

$R^8$ représente un atome d'hydrogène, un groupe alkyle, alkyle substitué, alkényle, alkynyle, phényle, 3-tétrahydrofurannyle, 2-oxo-3-tétrahydrofurannyle, 3-tétrahydrothiényle, l'oxyde ou le bioxyde de ce dernier, 3-pyridyle, cyclopropyle, cyclopentyle, cyclohexyle, cyclopropylméthyl, tétrahydrofurfuryle, furfuryle, thényle ou benzyle,

l'un des groupes $R^9$ et $R^{10}$, représente un groupe alkyle et l'autre représente un groupe alkyle ou alkylthio,

$R^{11}$ représente un atome d'hydrogène ou un groupe $N—OR^{20}$ dans lequel $R^{20}$ représente un atome d'hydrogène ou un groupe alkyle, et

l'un des groupes $R^{12}$ et $R^{13}$, représente un atome d'hydrogène, un groupe alkyle ou alkényle et l'autre représente un atome d'hydrogène, un groupe alkyle, cyanoalkyle, alkoxyalkyle, alkényle, alkynyle, alkylsulfonyle, arylsulfonyle (dans lequel le groupe aryle est un groupe phényle non substitué ou un groupe phényle à substituant halogène ou alkyle), haloalkylsulfonyle, alkylaminosulfonyle, dialkylamino-sulfonyle, ou CH(H ou $CH_3$)$CO_2$alkyle, ou $N(R^{12})(R^{13})$ représente un cycle pyrrolidino, pipéridino ou morpholino; ou

(c) un groupe $—N(R^{14})(R^{15})$, $—SO_2R^{16}$ ou $—CO—R^{17}$, dans lequel:

les groupes $R^{14}$ et $R^{15}$, représentent indépendamment un atome d'hydrogène, un groupe alkyle, alkényle, alkynyle, cyanoalkyle, acétyle, alkoxycarbonyle, alkoxyalkyle, aminocarbonyle, alkylamino-carbonyle, dialkylaminocarbonyle, alkylsulfonyle, haloalkylsulfonyle, arylsulfonyle (dans lequel le groupe aryle est un groupe phényle non substitué ou un groupe phényle à substituant halogène ou alkyle) ou un groupe CH(H ou $CH_3$)$CO_2$-alkyle, ou $N(R^{14})(R^{15})$ représente un groupe $N—C(R^9)(R^{10})$ dans lequel l'un des groupes $R^9$ et $R^{10}$, représente un groupe alkyle et l'autre un atome d'hydrogène, ou groupe alkyle ou alkylthio, ou $N(R^{14})(R^{15})$ représente un groupe tétrahydrophtalimido ou 2-oxopyrrolidino,

$R^{16}$ représente un groupe hydroxy, amino, alkylamino, dialkylamino, ou arylamino dans lequel le groupe aryle est un groupe phényle non substitué ou un groupe phényle à substituant halogène ou alkyle, et

$R^{17}$ représente un groupe hydroxy, alkoxy, alkylthio, amino, alkylamino, dialkylamino, ou arylamino dans lequel le groupe aryle est un groupe phényle non substitué ou un groupe phényle à substituant halogène ou alkyle;

ou un sel d'un tel composé comportant un atome d'hydrogène acide libre.

2. Composé selon la revendication 1, dans lequel W représente un atome d'oxygène, et Z un groupe nitro.

3. Composé selon la revendication 1, dans lequel W représente un atome d'oxygène, et Z un groupe $-QR^1$.

4. Composé selon la revendication 3, dans lequel Q représente un atome d'oxygène, et $R^1$ un groupe alkoxyalkyle.

5. Composé selon la revendication 4, dans lequel $R^1$ représente un groupe méthoxyméthyle.

6. Composé selon la revendication 1, dans lequel W représente un atome d'oxygène, et Z un groupe $-N(R^{14})(R^{15})$ dans lequel $R^{14}$ et $R^{15}$ sont tels que définis dans la revendication 1, ou un sel de celui-ci.

7. Composé selon la revendication 6, dans lequel Z représente un groupe $-NH_2$.

8. Composé selon la revendication 6, dans lequel au moins un des groupes $R^{14}$ et $R^{15}$, est un groupe alkylsulfonyle ou haloalkylsulfonyle.

9. Composé selon la revendication 6, dans lequel l'un des groupes $R^{14}$ et $R^{15}$, est un groupe alkyl sulfonyle, et l'autre est un atome d'hydrogène ou un groupe alkyl sulfonyle.

10. Composé selon la revendication 9, dans lequel l'un des groupes $R^{14}$ et $R^{15}$, est un groupe alkylsulfonyle, et l'autre est un atome d'hydrogène.

11. Sel selon la revendication 10.

12. Composé selon la revendication 6, dans lequel l'un des groupes $R^{14}$ et $R^{15}$, est un groupe $-CH(CH_3)CO_2$-alkyle ou $-CH_2CO_2$ alkyle.

13. Composé selon la revendication 12, dans lequel l'un des groupes $R^{14}$ et $R^{15}$, est un atome d'hydrogène, et l'autre un groupe $CH(CH_3)CO_2$-alkyle.

14. Composé selon l'une quelconque des revendications précédentes, dans lequel R représente un groupe n-propyle ou 3-fluoropropyle, $X^1$ représente un atome de fluor ou de chlore, et $X^2$ représente un atome de chlore ou de brome.

15. Composé selon la revendication 14, dans lequel R représente un groupe 3-fluoropropyle, $X^1$ représente un atome de fluor, et $X^2$ représente un atome de chlore.

16. Composition herbicide, contenant, comme ingrédient herbicide, un composé selon l'une quelconque des revendications 1 à 15.

17. Procédé de lutte contre la croissance de plantes non souhaitables, dans lequel on applique, à l'emplacement où l'on souhaite mettre en oeuvre cette lutte, une quantité à effet herbicide, d'une composition selon la revendication 16.

18. Composé de formule:

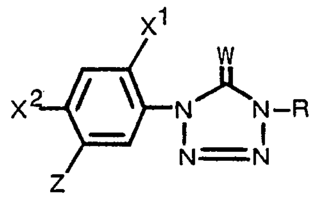

dans laquelle les groupes W, $X^1$ et $X^2$ sont tels que définis dans la revendication 1, et

(a) R représente un groupe alkyle, haloalkyle, alkoxyalkyle, alkylthioalkyle, cyanoalkyle, haloalkoxyalkyle, trifluorométhylthio, alkényle, haloalkényle, 2-oxopropyle ou 3-oxopropyle; et Z représente un groupe hydroxy, mercapto ou benzyloxy; ou

(b) R représente un atome d'hydrogène, et Z représente un atome de fluor, de chlore, de brome, un groupe cyano, nitro, alkyle, haloalkyle, alkynyle, ou $QR^1$ dans lequel Q représente un atome de soufre ou d'oxygène, et $R^1$ représente un groupe alkyle, benzyle, haloalkyle, cyanoalkyle, alkoxyalkyle, alkylthioalkyle, alkylsulfinylalkyle, alkylsulfonylalkyle, alkényle, haloalkényle, alkényloxyalkyle, alkynyle ou haloalkynyle comportant de 2 à 5 atomes de carbone.

19. Composé selon la revendication 18, dans lequel W représente un atome d'oxygène, $X^1$ représente un atome de fluor ou de chlore, $X^2$ représente un atome de chlore, et

(a) R représente un groupe n-propyle ou 3-fluoropropyle, et Z représente un groupe hydroxy, mercapto ou benzyloxy, ou

(b) R représente un atome d'hydrogène, et Z représente un groupe benzyloxy ou alkoxy en $C_1-C_4$.